(19) Europäisches Patentamt
European Patent Office
Office européen des brevets

(11) EP 4 424 323 A2

(12) **EUROPEAN PATENT APPLICATION**

(43) Date of publication:
**04.09.2024 Bulletin 2024/36**

(21) Application number: **24170072.3**

(22) Date of filing: **08.10.2014**

(51) International Patent Classification (IPC):
***A61K 39/00*** *(2006.01)*

(52) Cooperative Patent Classification (CPC):
**C07K 16/28; A61K 47/68033; A61K 47/6849; A61K 47/6851; A61P 35/00;** A61K 2039/505; A61K 2039/545; C07K 2317/24; C07K 2317/73

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.10.2013 US 201361888337 P**
**08.10.2013 US 201361888365 P**
**05.03.2014 US 201461948363 P**
**29.05.2014 US 201462004815 P**

(62) Document number(s) of the earlier application(s) in accordance with Art. 76 EPC:
**19199163.7 / 3 653 228**
**14852834.2 / 3 055 332**

(27) Previously filed application:
**08.10.2014 EP 19199163**

(71) Applicant: **ImmunoGen, Inc.**
**Waltham, MA 02451 (US)**

(72) Inventors:
- **LUTZ, Robert J.**
  **Wayland, MA, 01778 (US)**
- **PONTE, Jose**
  **Weymouth, MA, 02188 (US)**

(74) Representative: **Simmons & Simmons LLP (Munich)**
**Lehel Carré**
**Thierschplatz 6**
**80538 Munich (DE)**

Remarks:
•The complete document including Reference Table(s) and the Sequence Listing(s) can be downloaded from the EPO website
•This application was filed on 12.04.2024 as a divisional application to the application mentioned under INID code 62.
•Claims filed after the date of filing of the application/after the date of receipt of the divisional application (Rule 68(4) EPC).

(54) **ANTI-FOLR1 IMMUNOCONJUGATE DOSING REGIMENS**

(57) Methods of administering immunoconjugates that bind to FOLR1 are provided. The methods comprise administering an anti-FOLR1 immunoconjugate to a person in need thereof, for example, a cancer patient, at a therapeutically effective dosing regimen that results in minimal adverse effects.

EP 4 424 323 A2

## Description

### Field of the Invention

The field of the invention generally relates to methods of administering anti-FOLR1 immunoconjugates for the treatment of diseases, such as cancer. The methods provide dosing regimens that minimize unwanted side-effects.

### Background of the Invention

Cancer is one of the leading causes of death in the developed world, with over one million people diagnosed with cancer and 500,000 deaths per year in the United States alone. Overall it is estimated that more than 1 in 3 people will develop some form of cancer during their lifetime. There are more than 200 different types of cancer, four of which-breast, lung, colorectal, and prostate-account for over half of all new cases (Jemal et al., 2003, Cancer J. Clin. 53:5-26).

Folate Receptor 1 (FOLR1), also known as Folate Receptor-alpha, or Folate Binding Protein, is an N-glycosylated protein expressed on plasma membrane of cells. FOLR1 has a high affinity for folic acid and for several reduced folic acid derivatives. FOLR1 mediates delivery of the physiological folate, 5-mcthyltctrahydrofolatc, to the interior of cells.

FOLR1 is overexpressed in vast majority of ovarian cancers, as well as in many uterine, endometrial, pancreatic, renal, lung, and breast cancers, while the expression of FOLR1 on normal tissues is restricted to the apical membrane of epithelial cells in the kidney proximal tubules, alveolar pncumocytcs of the lung, bladder, testes, choroid plexus, and thyroid (Weitman SD, et al., Cancer Res 52: 3396-3401 (1992); Antony AC, Annu Rev Nutr 16: 501-521 (1996); Kalli KR, et al. Gynecol Oncol 108: 619-626 (2008)). This expression pattern of FOLR1 makes it a desirable target for FOLR1-directed cancer therapy.

Because ovarian cancer is typically asymptomatic until advanced stage, it is often diagnosed at a late stage and has poor prognosis when treated with currently available procedures, typically chemotherapeutic drugs after surgical de-bulking (von Gruenigen V et al., Cancer 112: 2221-2227 (2008); Ayhan A et al., Am J Obstet Gynecol 196: 81 e81-86 (2007); Harry VN et al., Obstet Gynecol Surv 64: 548-560 (2009)). Thus there is a clear unmet medical need for more effective therapeutics for ovarian cancers.

Antibodies are emerging as a promising method to treat such cancers. In addition, immunoconjugates, which comprise an antibody conjugated to another compound, for example, a cytotoxin, are also being investigated as potential therapeutics. In particular, immunoconjugates comprising maytansinoids, which are plant derived anti-fungal and anti-tumor agents, have been shown to have some beneficial activities. The isolation of three ansa macrolides from ethanolic extracts of *Maytenus ovatus* and *Maytenus buchananii* was first reported by S. M. Kupchan *et al.* and is the subject of U.S. Pat. No. 3,896.111 along with demonstration of their anti-leukemic effects in murine models at the microgram/kg dose range. Maytansinoids, however, have unacceptable toxicity, causing both central and peripheral neuropathies, and side effects: particularly nausea, vomiting, diarrhea, elevations of hepatic function tests and, less commonly, weakness and lethargy. This overall toxicity is reduced to some extent by the conjugation of maytansinoids to antibodies because an antibody conjugate has a toxicity which is several orders of magnitude lower on antigen-negative cells compared to antigen-positive cells. However, there is still a need to identify particular dosage regimens of anti-FOLR1 immunoconjugates that are therapeutically effective in humans but avoid adverse effects.

### BRIEF SUMMARY OF THE INVENTION

Methods of administering an anti-FOLR1 immunoconjugate at a therapeutically effective dosing regimen that minimizes unwanted side-effects are provided herein. As described in more detail below, administration of the same dose of an anti-FOLR1 immunoconjugate to different patients results in substantial variations in the pharmacokinetics (e.g., Cmax and AUC) of the immunoconjugate. The present inventors have discovered, and the experiments provided herein demonstrate, that ocular toxicity correlates with a high Cmax and high initial AUC value. However, the high Cmax and initial AUC values are not required for efficacy. Accordingly, described herein are methods for treating a patient having cancer comprising administering to the patient an effective dose of an immunoconjugate which binds to FOLR1, wherein the immunoconjugate is administered at a dose of about 3.0 mg/kg to about 7.0 mg/kg, wherein the kilograms of body weight are adjusted to ideal body weight (IBW), lean body weight (LBW), or adjusted ideal body weight (ADJ or AIBW). The abbreviations "ADJ" and "AIBW" can be used interchangeably to refer to adjusted ideal body weight. In some embodiments, the kilograms arc adjusted to AIBW (ADJ). Also, described herein are methods for treating a patient having cancer comprising administering to the patient an effective dose of an immunoconjugate which binds to FOLR1, wherein the immunoconjugate is administered once a week for three weeks on a four-week schedule (e.g., on days 1, 8, and 15 of a four-week schedule). Also described herein are methods for treating a patient having cancer comprising administering to the patient an effective dose of an immunoconjugate which binds to FOLR1, wherein the immunoconjugate is administered at a dose about 1 to about 7 mg/kg, wherein the kilograms are adjusted to IBW, LBW, or AIBW

(ADJ), and wherein the immunoconjugate is administered once a week for three weeks on a four-week schedule (e.g., on days 1, 8, and 15 of a four-week schedule). The methods described herein can result in a decrease in toxicity, e.g., ocular toxicity.

In addition, described herein are methods for treating a patient having cancer comprising administering to the patient an effective dose of an immunoconjugate which binds to FOLR1, wherein the Cmax and initial AUC values that result in toxicity are not exceeded. For example, in some embodiments, the administration produces a Cmax of about 90-160 μg/mL, and in some embodiments, the administration produces a Cmax of about 110-160 μg/mL. In some embodiments, the administration produces an $AUC_{0-24}$ of no more than 2785 hr•μg/mL. In some embodiments, the administration produces an $AUC_{0-24}$ of no more than 2741 hr•μg/mL. In some embodiments, the administration produces an $AUC_{0-24}$ of no more than 2700 hr•μg/mL. In some embodiments, the administration produces a Cmax of no more than 160 gg/mL. In some embodiments, the administration produces a Cmax of no more than 150 μg/mL.

In some embodiments, the administration produces an $AUC_{0-24}$ of about 1000-3500 hr•μg/mL. In some embodiments, the administration produces an $AUC_{0-24}$ of about 1000-3000 hr•μg/mL. In some embodiments, the administration produces an $AUC_{0-24}$ of about 1000-2785 hr•μg/mL. In some embodiments, the administration produces an $AUC_{0-24}$ of about 1000-2741 hr•μg/mL. In some embodiments, the administration produces an $AUC_{0-24}$ of about 1000-2700 hr•μg/mL. In some embodiments, the administration produces an $AUC_{0-24}$ of about 1000-2500 hr•μg/mL. In some embodiments, the administration produces an $AUC_{0-24}$ of no more than 1500-3500 hr•μg/mL. In some embodiments, the administration produces an $AUC_{0-24}$ of no more than 1500-3000 hr•μg/mL. In some embodiments, the administration produces an $AUC_{0-24}$ of no more than 1500-2785 hr•μg/mL. In some embodiments, the administration produces an $AUC_{0-24}$ of no more than 1500-2741 hr•μg/mL. In some embodiments, the administration produces an $AUC_{0-24}$ of no more than 1500-2700 hr•μg/mL. In some embodiments, the administration produces an $AUC_{0-24}$ of about 1500-2500 hr•μg/mL.

In some embodiments, the administration produces a Cmax of 110-160 μg/mL. In some embodiments, the administration produces a Cmax of 110-150 μg/mL. In some embodiments, the administration produces a Cmax of 110-140 μg/mL. In some embodiments, the administration produces a Cmax of 120-160 μg/mL. In some embodiments, the administration produces a Cmax of about 120-150 μg/mL. In some embodiments, the administration produces a Cmax of about 120-140 μg/mL. In some embodiments, the administration produces a Cmax of 90-160 μg/mL. In some embodiments, the administration produces a Cmax of about 90-150 μg/mL. In some embodiments, the administration produces a Cmax of about 90-140 μg/mL. In some embodiments, the administration produces a Cmax of 100-160 μg/mL. In some embodiments, the administration produces a Cmax of about 100-150 μg/mL. In some embodiments, the administration produces a Cmax of about 100-140 μg/mL.

The anti-FOLR1 immunoconjugate can comprise a charged linker. In some embodiments, the anti-FOLR1 immunoconjugate comprises the antibody huMov19, the linker sulfo-SPDB, and the maytansinoid DM4.

In some embodiments, the immunoconjugate comprises an antibody or antigen-binding fragment thereof that competitively inhibits the binding of an antibody with the variable region sequences of SEQ ID NO:3 and SEQ ID NO:5 to FOLR1. In some embodiments, the antibody or fragment thereof comprises the CDRs of huMOV19 (i.e., SEQ ID NOs: 6-10 and 12 or SEQ ID NOs: 6-9, 11, and 12). In some embodiments, the antibody or antigen-binding fragment thereof comprises the variable region sequences of huMOV19 (i.e., SEQ ID NOs: 3 and 5). In some embodiments, the antibodies or fragments do not comprise the six CDRs of murine Mov19 (i.e., SEQ ID NOs:6-9, 16, and 12). In some embodiments, the antibody is huMov19. In some embodiments, the immunoconjugate comprises a maytansinoid. In some embodiments, the maytansinoid is DM4. In some embodiments, the immunoconjugate comprises a linker that is sulfo-SPDB. In some embodiments, the immunoconjugate is IMGN853 (huMov19-sulfo-SPDB-DM4).

In some embodiments, the anti-FOLR1 binding agent (e.g., huMov19-sulfo-SPDB-DM4) is administered at a dose of about 1 to 7 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 1.0 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 1.1 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 1.5 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 1.8 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 2.0 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 2.5 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 2.8 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose

of about 3.0 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 3.3 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 3.75 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 4.0 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 4.1 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 4.2 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 5 mg/kg, wherein the kilograms of body weight are adjusted to 1BW, LBW, BSA, or AIBW (ADJ). In some embodiments, the anti-FOLR1 binding agent (e.g., huMov19-sulfo-SPDB-DM4) is administered at a dose of about 5.5 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 5.6 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 6 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 6.5 mg/kg, wherein the kilograms of body weight are adjusted to IBW, LBW, BSA, or AIBW (ADJ). In some embodiments, the anti-FOLR1 binding agent (e.g., huMov19-sulfo-SPDB-DM4) is administered at a dose of about 7 mg/kg, wherein the kilograms of body weight are adjusted to IBW, LBW, BSA, or AIBW (ADJ). In some embodiments, the kilograms of body weight are adjusted to AIBW (ADJ).

According to the methods described herein, the anti-FOLR1 binding agent (e.g., huMov19-sulfo-SPDB-DM4) can be administered about once every 4 weeks. In some embodiments, the anti-FOLR1 binding agent (e.g., huMov19-sulfo-SPDB-DM4) is administered about once every 3 weeks. In some embodiments, the anti-FOLR1 binding agent (e.g., huMov19-sulfo-SPDB-DM4) is administered about once every 2 weeks. In some embodiments, the anti-FOLR1 binding agent (e.g., huMov19-sulfo-SPDB-DM4) is administered about once every 1 week.

In some embodiments, the immunoconjugate is administered at a dose of about I to 7 mg/kg once a week for three weeks on a four-week schedule. In some embodiments, the immunoconjugate is administered at a dose of about 1.5 mg/kg to about 6 mg/kg once a week for three weeks on a four-week schedule. In some embodiments, the immunoconjugate is administered at a dose of about 1.5, 2.0, 2.5, 3, 3.75, 4.0, 4.1, 4.2, 5.0, 5.5, or 6.0 mg/kg once a week for three weeks on a four-week schedule. In some embodiments, the immunoconjugate is administered at a dose of about 1.1, 1.8, 2.5, 3.3, or 4.2 mg/kg once a week for three weeks on a four-week schedule. In some embodiments, the anti-FULR1 immunoconjugate is administered at a dose of about 1.0 mg/kg once a week for three weeks on a four-week schedule. In some embodiments, the anti-FOLR1 immunoconjugate is administered at a dose of about 1.1 mg/kg once a week for three weeks on a four-week schedule. In some embodiments, the anti-FOLR1 immunoconjugate is administered at a dose of about 1.5 mg/kg once a week for three weeks on a four-week schedule. In some embodiments, the anti-FOLR1 immunoconjugate is administered at a dose of about 1.8 mg/kg once a week for three weeks on a four-week schedule. In some embodiments, the anti-FOLR1 immunoconjugate is administered at a dose of about 2 mg/kg once a week for three weeks on a four-week schedule. In some embodiments, the anti-FOLR1 immunoconjugate is administered at a dose of about 2.5 mg/kg once a week for three weeks on a four-week schedule. In some embodiments, the anti-FOLR1 immunoconjugate is administered at a dose of about 2.8 mg/kg once a week for three weeks on a four-week schedule. In some embodiments, the anti-FOLR1 binding agent is administered at a dose of about 3.0 mg/kg once a week for three weeks on a four-week schedule. In some embodiments, the anti-FOLR1 immunoconjugate is administered at a dose of about 3.3 mg/kg once a week for three weeks on a four-week schedule. In some embodiments, the anti-FOLR1 immunoconjugate is administered at a dose of about 3.75 mg/kg once a week for three weeks on a four-week schedule. In some embodiments, the anti-FOLR1 binding agent is administered at a dose of about 4.0 mg/kg once a week for three weeks on a four-week schedule. In some embodiments, the anti-FOLR1 immunoconjugate is administered at a dose of about 4.1 mg/kg once a week for three weeks on a four-week schedule. In some embodiments, the anti-FOLR1 immunoconjugate is administered at a dose of about 4.2 mg/kg once a week for three weeks on a four-week schedule. In some embodiments, the anti-FOLR1 immunoconjugate is administered at a dose of about 5 mg/kg once a week for three weeks on a four-week schedule. In some embodiments, the anti-FOLR1 binding agent is administered at a dose of about 5.5 mg/kg once a week for three weeks on a four-week schedule. In some embodiments, the anti-FOLR1 immunoconjugate is administered at a dose of about 5.6 mg/kg once a week for three weeks on a four-week schedule. In some embodiments, the anti-FOLR1 binding agent is administered at a dose of about 6 mg/kg once a week for three weeks on a four-week schedule. In some embodiments, the anti-FOLR1 immunoconjugate is administered at a dose of about 6.5 mg/kg once a week for three weeks on a four-week schedule. In some embodiments, the anti-FOLR1

immunoconjugate is administered at a dose of about 7 mg/kg once a week for three weeks on a four-week schedule.

In some embodiments, the immunoconjugate (e.g., huMov19-sulfo-SPDB-DM4) is administered at a dose of about 1 to 7 mg/kg, wherein the kilograms are adjusted to IBW, LBW, BSA, or AIBW (ADJ), and wherein the immunoconjugate is administered once a week for three weeks on a four-week schedule. In some embodiments, the immunoconjugate (e.g., huMov19-sulfo-SPDB-DM4) is administered at a dose of about 1.5 mg/kg to about 6 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 1 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 1.1 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 1.5 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 1.8 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 2 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 2.5 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 2.8 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMovl9-sulfo-SPDB-DM4) is administered at a dose of about 3 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 3.3 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR 1 immunoconjugate (e.g., huMov19-sulfo-SPDB-DM4) is administered at a dose of about 3.75 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 4 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 4.1 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 4.2 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 5 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 5.5 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 5.6 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 6 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 6.5 mg/kg, wherein the kilograms are adjusted to IBW, LBW, BSA, or AIBW (ADJ), and wherein the immunoconjugate is administered once a week for three weeks on a four-week schedule. In some embodiments, the anti-FOLR1 immunoconjugate (e.g., huMov19-sulfo-SPDB-DM4) is administered at a dose of about 7 mg/kg, wherein the kilograms are adjusted to IBW, LBW, BSA, or AIBW (ADJ), and wherein the immunoconjugate is administered once a week for three weeks on a four-week schedule. In some embodiments, the kilograms are adjusted to AIBW (ADJ).

In some embodiments, the FOLR1-binding agents are administered to obtain the AUC obtained in Examples 1-6 and shown in Figures 1-2 and 7-12.

In some embodiments, the FOLR1 -binding agents are administered to obtain the Cmax obtained in Examples 1-6 and shown in Figures 1-6 and 9-12.

In some embodiments, the anti-FOLR1 binding agent (e.g., huMov19-sulfo-SPDB-DM4) is administered intravenously.

The methods described herein can be used to treat cancer. In some embodiments, the cancer is selected from the group consisting of ovarian, brain, breast, uterine, endometrial, pancreatic, renal (e.g., renal cell carcinoma), and lung cancer (e.g., non small cell lung cancer, adenocarcinoma of the lung, or bronchioloalveolar carcinoma (BAC)). In some embodiments, the cancer is ovarian cancer or lung cancer. In some embodiments, the cancer is epithelial ovarian cancer.

In some embodiments, the cancer expresses FOLR1 polypeptide or nucleic acid. In some embodiments, the cancer has an increased expression level of FOLR1 polypeptide as measured by immunohistochemistry (IHC). For example, in some embodiments, the cancer is a cancer that expresses FOLR1 polypeptide at a level of 1 hetero or higher by IHC. In some embodiments, the cancer is a cancer that expresses FOLR1 polypeptide at a level of 1 homo or higher by IHC. In some embodiments, the cancer is a cancer that expresses FOLR1 polypeptide at a level of 2 hetero or higher by IHC. In some embodiments, the cancer is a cancer that expresses FOLR1 polypeptide at a level of 2 homo or higher by 1HC. In some embodiments, the cancer is a cancer that expresses FOLR1 polypeptide at a level of 3 hetero or higher by 1HC. In some embodiments, the cancer is a cancer that expresses FOLR1 polypeptide at a level of 3 homo or higher by IHC. In some embodiments, the cancer is a lung cancer that expresses FOLR1 polypeptide at a level of 2 hetero or higher by IHC. In some embodiments, the cancer is a lung cancer that expresses FOLR1 polypeptide at a level of 3 hetero or higher by IHC. In some embodiments, the cancer is an epithelial ovarian cancer (e.g., platinum resistant or relapsed or refractory) that expresses FOLR1 polypeptide at a level of 2 hetero or higher. In some embodiments, the cancer is an epithelial ovarian cancer (e.g., platinum resistant or relapsed or refractory) that expresses FOLR1 polypeptide at a level of 3 hetero or higher. In some embodiments, the cancer is an endometriod cancer that expresses FOLR1 polypeptide at a level of 1 hetero or higher.In some embodiments, the cancer is an endometriod cancer that expresses FOLR1 polypeptide at a level of 2 hetero or higher.

In some embodiments, the methods further comprise administering a steroid to the patient. The steroid can be administered as a pre-treatment, i.c., prior to the administration of the anti-FOLR1 binding agent. The steroid can be dexamethasone.

The methods described herein can result in a decrease in tumor size. The methods described herein can result in a decrease in CA125 levels in ovarian cancer patients. In one example, CA125 levels are measured in a sample from an ovarian cancer patient prior to treatment and then one or more times after treatment, and a decrease in the CA125 level over time is indicative of therapeutic efficacy. The methods described herein can result in an increased time between cancer treatments. The methods described herein can result in increased progression free survival (PFS). The methods described herein can result in increased disease-free survival (DFS). The methods described herein can result in increased overall survival (OS). The methods described herein can result in increased complete response (CR). The methods described herein can result in increased partial response (PR). The methods described herein can result in increased stable disease (SD). The methods described herein can result in increased decrease in progressive disease (PD). The methods described herein can result in a reduced time to progression (TTP).

In particular, the dosing regiments provided herein achieve an optimal balance between efficacy (e.g., PR) and reduced toxicity as demonstrated, for instance, in Examples 1-4 and Figures 1-7.

## BRIEF DESCRIPTION OF THE DRAWINGS/FIGURES

Figures 1A and B provide pharmacokinetic data resulting from the administration of IMGN853 (0.15 mg/kg to 7.0 mg/kg) as described in Example 1. Figure 1B provides a later summary of the pharmacokinetic data that includes the data from Figure 1A and additional data obtained from additional patients.

Figures 2A-C show the responses and the occurrence of ocular toxicity in patients with a range of Cmax and $AUC_{0-24}$ and $AUC_{0-168}$ values.

Figure 3 shows the range of Cmax values measured at various doses.

Figure 4 shows the dependence of Cmax on patient body weight.

Figure 5 shows the variance in Cmax and $AUC_{0-24}$ associated with alternate dosing approaches.

Figure 6 shows the projected dependence of Cmax on body weight using alternate dosing approaches.

Figure 7 shows a plot of the $AUC_{0-24}$ values observed in 24 patients receiving 3.3, 5, or 7 mg/kg IMGN853 based on total body weight (actual). These values are compared to projected values if all the patients had been treated

with 5 mg/kg based on total body weight, (TBW 5 mg/kg) and the projected values if all of the patients were dosed at 5, 5.4, or 6 mg/kg based on adjusted ideal body weight (ADJ 5, 5.4 or 6). The actual data of 7 patients treated at 5 mg/kg by adjusted ideal body weight (5 ADJ Actual) is also shown. The percentage of patients that have or are projected to have AUC values above the ocular toxicity threshold is shown in the table below the plot.

Figure 8 shows $AUC_{0-24}$ values for all patients in the 3.3 - 7.0 mg/kg. TBW cohorts were used to calculate predicted $AUC_{0-24}$ values at the indicated dose levels. Actual patient $AUC_{0-24}$ data for the indicated doses are also plotted, including patients dosed in the 5.0 and 6.0 adjusted idea body weight (ATBW) cohorts to date.

Figure 9 shows the anti-tumor activity, predicted plasma concentration, and other pharmacokinetic parameters of IMGN853 in mice treated with single doses of 2.8 mg/kg, 5.6 mg/kg, or 8.5 mg/kg of the immunoconjugate.

Figure 10 shows the anti-tumor activity, predicted plasma concentration, and other pharmacokinetic parameters of IMGN853 in mice treated with a single dose of 8.5 mg/kg, three daily doses of 2.8 mg/kg, or three doses of 2.8 mg/kg every three days.

Figure 11 shows the anti-tumor activity, predicted plasma concentration, and other pharmacokinetic parameters of IMGN853 in mice treated with a single dose of 5.6 mg/kg or 1.4 mg/kg daily for three days.

Figure 12 shows the anti-tumor activity, predicted plasma concentration, and other pharmacokinetic parameters of IMGN853 in mice treated with a single dose of 8.5 mg/kg or 2.8 mg/kg weekly for three weeks.

DETAILED DESCRIPTION OF THE INVENTION

The present invention provides new dosing regimens for FOLR1 binding immunoconjugates.

**I. Definitions**

To facilitate an understanding of the present invention, a number of terms and phrases are defined below.

The terms "human folate receptor 1," "FOLR1," or "folate receptor alpha (FR-$\alpha$)", as used herein, refers to any native human FOLR1, unless otherwise indicated. Thus, all of these terms can refer to either a protein or nucleic acid sequence as indicated herein. The term "FOLR1" encompasses "full-length," unprocessed FOLR1 as well as any form of FOLR1 that results from processing within the cell. The term also encompasses naturally occurring variants of FOLR1, e.g., splice variants, allelic variants and isoforms. The FOLR1 polypeptides described herein can be isolated from a variety of sources, such as from human tissue types or from another source, or prepared by recombinant or synthetic methods. Examples of FOLR1 sequences include, but are not limited to NCBI reference numbers P15328, NP 001092242.1, AAX29268.1, AAX37119.1, NP_057937.1, and NP_057936.1.

The term "antibody" means an immunoglobulin molecule that recognizes and specifically binds to a target, such as a protein, polypeptide, peptide, carbohydrate, polynucleotide, lipid, or combinations of the foregoing through at least one antigen recognition site within the variable region of the immunoglobulin molecule. As used herein, the term "antibody" encompasses intact polyclonal antibodies, intact monoclonal antibodies, antibody fragments (such as Fab, Fab', F(ab')2, and Fv fragments), single chain Fv (scFv) mutants, multispecific antibodies such as bispecific antibodies generated from at least two intact antibodies, chimeric antibodies, humanized antibodies, human antibodies, fusion proteins comprising an antigen determination portion of an antibody, and any other modified immunoglobulin molecule comprising an antigen recognition site so long as the antibodies exhibit the desired biological activity. An antibody can be of any the five major classes of immunoglobulins: IgA, IgD, IgE, IgG, and IgM, or subclasses (isotypes) thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as toxins, radioisotopes, etc.

A "blocking" antibody or an "antagonist" antibody is one which inhibits or reduces biological activity of the antigen it binds, such as FOLR1. In some embodiments, blocking antibodies or antagonist antibodies substantially or completely inhibit the biological activity of the antigen. The biological activity can be reduced by 10%, 20%, 30%, 50%, 70%, 80%, 90%, 95%, or even 100%.

The term "anti-FOLR1 antibody" or "an antibody that binds to FOLR1" refers to an antibody that is capable of binding FOLR1 with sufficient affinity such that the antibody is useful as a diagnostic and/or therapeutic agent in targeting FOLR1. The extent of binding of an anti-FOLR1 antibody to an unrelated, non-FOLR1 protein can be less than about 10% of the binding of the antibody to FOLR1 as measured, e.g., by a radioimmunoassay (RIA). In certain embodiments, an antibody that binds to FOLR1 has a dissociation constant (Kd) of $\leq 1\ \mu M$, $\leq 100$ nM, $\leq 10$ nM, $\leq 1$ nM, or $\leq 0.1$ nM.

The term "antibody fragment" refers to a portion of an intact antibody and refers to the antigenic determining variable regions of an intact antibody. Examples of antibody fragments include, but are not limited to Fab, Fab', F(ab')2, and Fv fragments, linear antibodies, single chain antibodies, and multispecific antibodies formed from antibody fragments.

A "monoclonal antibody" refers to a homogeneous antibody population involved in the highly specific recognition

and binding of a single antigenic determinant, or epitope. This is in contrast to polyclonal antibodies that typically include different antibodies directed against different antigenic determinants. The term "monoclonal antibody" encompasses both intact and full-length monoclonal antibodies as well as antibody fragments (such as Fab, Fab', F(ab')2, Fv), single chain (scFv) mutants, fusion proteins comprising an antibody portion, and any other modified immunoglobulin molecule comprising an antigen recognition site. Furthermore, "monoclonal antibody" refers to such antibodies made in any number of manners including but not limited to by hybridoma, phage selection, recombinant expression, and transgenic animals.

The term "humanized antibody" refers to forms of non-human (e.g. murine) antibodies that are specific immunoglobulin chains, chimeric immunoglobulins, or fragments thereof that contain minimal non-human (e.g., murine) sequences. Typically, humanized antibodies are human immunoglobulins in which residues from the complementary determining region (CDR) are replaced by residues from the CDR of a non-human species (e.g. mouse, rat, rabbit, hamster) that have the desired specificity, affinity, and capability (Jones ct al., 1986, Nature, 321:522-525; Ricchmann et al., 1988, Nature, 332:323-327; Verhoeyen et al., 1988, Science, 239:1534-1536). In some instances, the Fv framework region (FR) residues of a human immunoglobulin are replaced with the corresponding residues in an antibody from a non-human species that has the desired specificity, affinity, and capability. The humanized antibody can be further modified by the substitution of additional residues either in the Fv framework region and/or within the replaced non-human residues to refine and optimize antibody specificity, affinity, and/or capability. In general, the humanized antibody will comprise substantially all of at least one, and typically two or three, variable domains containing all or substantially all of the CDR regions that correspond to the non-human immunoglobulin whereas all or substantially all of the FR regions are those of a human immunoglobulin consensus sequence. The humanized antibody can also comprise at least a portion of an immunoglobulin constant region or domain (Fc), typically that of a human immunoglobulin. Examples of methods used to generate humanized antibodies are described in U.S. Pat. 5,225,539. In some embodiments, a "humanized antibody" is a resurfaced antibody.

A "variable region" of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies. There are at least two techniques for determining CDRs: (1) an approach based on cross-species sequence variability (i.e., Kabat et al. Sequences of Proteins of Immunological Interest, (5th ed., 1991, National Institutes of Health, Bethesda Md.)); and (2) an approach based on crystallographic studies of antigen-antibody complexes (Al-lazikani et al (1997) J. Molec. Biol. 273:927-948)). In addition, combinations of these two approaches are sometimes used in the art to determine CDRs.

The Kabat numbering system is generally used when referring to a residue in the variable domain (approximately residues 1-107 of the light chain and residues 1-113 of the heavy chain) (e.g., Kabat et al., Sequences of Immunological Interest. 5th Ed. Public Health Service, National Institutes of Health, Bethesda, Md. (1991)).

The amino acid position numbering as in Kabat, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, Md. (1991). Using this numbering system, the actual linear amino acid sequence can contain fewer or additional amino acids corresponding to a shortening of, or insertion into, a FR or CDR of the variable domain. For example, a heavy chain variable domain can include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues can be determined for a given antibody by alignment at regions of homology of the sequence of the antibody with a "standard" Kabat numbered sequence. Chothia refers instead to the location of the structural loops (Chothia and Lesk J. Mol. Biol. 196:901-917 (1987)). The end of the Chothia CDR-H1 loop when numbered using the Kabat numbering convention varies between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). The AbM hypervariable regions represent a compromise between the Kabat CDRs and Chothia structural loops, and are used by Oxford Molecular's AbM antibody modeling software,

| Loop | Kabat | AbM | Chothia |
|---|---|---|---|
| L1 | L24-L34 | L24-L34 | L24-L34 |
| L2 | L50-L56 | L50-L56 | L50-L56 |
| L3 | L89-L97 | L89-L97 | L89-L97 |
| H1 | H31-H35B | H26-H35B | H26-H32..34 |

(continued)

| Loop | Kabat | AbM | Chothia |
|---|---|---|---|
| | | (Kabat Numbering) | |
| H1 | H31-H35 | H26-H35 | H26-H32 |
| | | (Chothia Numbering) | |
| H2 | H50-H65 | H50-H58 | H52-H56 |
| H3 | H95-H102 | H95-H102 | H95-H102 |

The term "human antibody" means an antibody produced by a human or an antibody having an amino acid sequence corresponding to an antibody produced by a human made using any technique known in the art. This definition of a human antibody includes intact or full-length antibodies, fragments thereof, and/or antibodies comprising at least one human heavy and/or light chain polypeptide such as, for example, an antibody comprising murine light chain and human heavy chain polypeptides.

The term "chimeric antibodies" refers to antibodies wherein the amino acid sequence of the immunoglobulin molecule is derived from two or more species. Typically, the variable region of both light and heavy chains corresponds to the variable region of antibodies derived from one species of mammals (e.g. mouse, rat, rabbit, etc.) with the desired specificity, affinity, and capability while the constant regions are homologous to the sequences in antibodies derived from another (usually human) to avoid eliciting an immune response in that species.

The term "epitope" or "antigenic determinant" are used interchangeably herein and refer to that portion of an antigen capable of being recognized and specifically bound by a particular antibody. When the antigen is a polypeptide, epitopes can be formed both from contiguous amino acids and noncontiguous amino acids juxtaposed by tertiary folding of a protein. Epitopes formed from contiguous amino acids are typically retained upon protein denaturing, whereas epitopes formed by tertiary folding are typically lost upon protein denaturing. An epitope typically includes at least 3, and more usually, at least 5 or 8-10 amino acids in a unique spatial conformation.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative embodiments arc described in the following.

"Or better" when used herein to refer to binding affinity refers to a stronger binding between a molecule and its binding partner. "Or better" when used herein refers to a stronger binding, represented by a smaller numerical Kd value. For example, an antibody which has an affinity for an antigen of "0.6 nM or better", the antibody's affinity for the antigen is <0.6 nM, i.e. 0.59 nM, 0.58 nM, 0.57 nM etc. or any value less than 0.6 nM.

By "specifically binds," it is generally meant that an antibody binds to an epitope via its antigen binding domain, and that the binding entails some complementarity between the antigen binding domain and the epitope. According to this definition, an antibody is said to "specifically bind" to an epitope when it binds to that epitope, via its antigen binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D."

By "preferentially binds," it is meant that the antibody specifically binds to an epitope more readily than it would bind to a related, similar, homologous, or analogous epitope. Thus, an antibody which "preferentially binds" to a given epitope would more likely bind to that epitope than to a related epitope, even though such an antibody may cross-react with the related epitope.

An antibody is said to "competitively inhibit" binding of a reference antibody to a given epitope if it preferentially binds to that epitope to the extent that it blocks, to some degree, binding of the reference antibody to the epitope. Competitive inhibition may be determined by any method known in the art, for example, competition ELISA assays. An antibody may be said to competitively inhibit binding of the reference antibody to a given epitope by at least 90%, at least 80%, at least 70%, at least 60%, or at least 50%.

The phrase "substantially similar," or "substantially the same", as used herein, denotes a sufficiently high degree of similarity between two numeric values (generally one associated with an antibody of the invention and the other

associated with a reference/comparator antibody) such that one of skill in the art would consider the difference between the two values to be of little or no biological and/or statistical significance within the context of the biological characteristic measured by said values (e.g., Kd values). The difference between said two values can be less than about 50%, less than about 40%, less than about 30%, less than about 20%, or less than about 10% as a function of the value for the reference/comparator antibody.

A polypeptide, antibody, polynucleotide, vector, cell, or composition which is "isolated" is a polypeptide, antibody, polynucleotide, vector, cell, or composition which is in a form not found in nature. Isolated polypeptides, antibodies, polynucleotides, vectors, cell or compositions include those which have been purified to a degree that they are no longer in a form in which they are found in nature. In some embodiments, an antibody, polynucleotide, vector, cell, or composition which is isolated is substantially pure.

As used herein, "substantially pure" refers to material which is at least 50% pure (i.e., free from contaminants), at least 90% pure, at least 95% pure, at least 98% pure, or at least 99% pure.

The term "immunoconjugate" or "conjugate" as used herein refers to a compound or a derivative thereof that is linked to a cell binding agent (i.e., an anti-FOLR1 antibody or fragment thereof) and is defined by a generic formula: C-L-A, wherein C = cytotoxin, L = linker, and A = anti-FOLR1 antibody or antibody fragment. Immunoconjugates can also be defined by the generic formula in reverse order: A-L-C.

The term "IMGN853" refers to the immunoconjugate described herein containing the huMov19 antibody, the sulfoSPDB linker, and the DM4 maytansinoid. The huMov19 antibody contains a variable heavy chain with the amino acid sequence of SEQ ID NO:3 and a variable light chain with the amino acid sequence of SEQ ID NO: 5.

A "linker" is any chemical moiety that is capable of linking a compound, usually a drug, such as a maytansinoid, to a cell-binding agent such as an anti FOLR1 antibody or a fragment thereof in a stable, covalent manner. Linkers can be susceptible to or be substantially resistant to acid-induced cleavage, light-induced cleavage, peptidase-induced cleavage, esterase-induced cleavage, and disulfide bond cleavage, at conditions under which the compound or the antibody remains active. Suitable linkers are well known in the art and include, for example, disulfide groups, thioether groups, acid labile groups, photolabile groups, peptidase labile groups and esterase labile groups. Linkers also include charged linkers, and hydrophilic forms thereof as described herein and know in the art.

The terms "cancer" and "cancerous" refer to or describe the physiological condition in mammals in which a population of cells are characterized by unregulated cell growth. Examples of cancer include, but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancers. The cancer can be a cancer that expresses FOLR1.

"Tumor" and "neoplasm" refer to any mass of tissue that result from excessive cell growth or proliferation, either benign (noncancerous) or malignant (cancerous) including prc-canccrous lesions.

The terms "cancer cell," "tumor cell," and grammatical equivalents refer to the total population of cells derived from a tumor or a pre-cancerous lesion, including both non-tumorigenic cells, which comprise the bulk of the tumor cell population, and tumorigenic stem cells (cancer stem cells). As used herein, the term "tumor cell" will be modified by the term "non-tumorigenic" when referring solely to those tumor cells lacking the capacity to renew and differentiate to distinguish those tumor cells from cancer stem cells.

The term "subject" refers to any animal (e.g., a mammal), including, but not limited to humans, non-human primates, rodents, and the like, which is to be the recipient of a particular treatment. Typically, the terms "subject" and "patient" are used interchangeably herein in reference to a human subject.

The term "ideal body weight" (IBW) refers to a size descriptor that is unrelated to total body weight. IBW is an estimate of weight corrected for sex and height, and optionally frame size. IBW can be calculated, for example, using the formulas IBW = 0.9H-88 (for males) and IBW = 0.9H-92 (for females), wherein H=height in cm.

The term "lean body weight" (LBW) refers to a size descriptor that can account for fractional fat mass ($FM_{frac}$). LBW is equal to total body weight minus the product of $FM_{frac}$ and weight. LBW can be calculated, for example, using the formulas LBW = 1.10 $\times$ weight in kg - 128([weight in kg]$^2$ / [100 $\times$ height in meters]$^2$) (for males) and LBW = 1.07 $\times$ weight in kg - 148([weight in kg]$^2$/[100 $\times$ height in meters]$^2$) (for females).

The term "adjusted ideal body weight" (AIBW) or "adjusted body weight" (ADJ) refers to a size descriptor that accounts for sex, total body weight, and height. AIBW and ADJ are used interchangeably throughout the specification. AIBW (ADJ) can be calculated, for example, using the formula ADJ = IBW + 0.4(weight in kg - LBW).

IBW, LBW, and AIBW (ADJ) are discussed in more detail in Green and Duffull, British Journal of Clinical Pharmacology 58: 119-133 (2004), which is herein incorporated by reference in its entirety.

Administration "in combination with" one or more further therapeutic agents includes simultaneous (concurrent)

and consecutive administration in any order.

The term "pharmaceutical formulation" refers to a preparation which is in such form as to permit the biological activity of the active ingredient to be effective, and which contains no additional components which are unacceptably toxic to a subject to which the formulation would be administered. The formulation can be sterile.

An "effective amount" of an antibody or immunoconjugate as disclosed herein is an amount sufficient to carry out a specifically stated purpose. An "effective amount" can be determined empirically and in a routine manner, in relation to the stated purpose.

The term "therapeutically effective amount" refers to an amount of an antibody or other drug effective to "treat" a disease or disorder in a subject or mammal. In the case of cancer, the therapeutically effective amount of the drug can reduce the number of cancer cells; reduce the tumor size; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP), a decrease in CA125 in the case of ovarian cancer, or any combination thereof.

See the definition herein of "treating." To the extent the drug can prevent growth and/or kill existing cancer cells, it can be cytostatic and/or cytotoxic. In certain embodiments, identification of increased FOLR1 levels allows for administration of decreased amounts of the FOLR1-targeting therapeutic to achieve the same therapeutic effect as seen with higher dosages. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "respond favorably" generally refers to causing a beneficial state in a subject. With respect to cancer treatment, the term refers to providing a therapeutic effect on the subject. Positive therapeutic effects in cancer can be measured in a number of ways (*See,* W.A. Weber, J. Nucl. Med. 50:1S-10S (2009)). For example, tumor growth inhibition, molecular marker expression, serum marker expression, and molecular imaging techniques can all be used to assess therapeutic efficacy of an anti-cancer therapeutic. With respect to tumor growth inhibition, according to NCI standards, a T/C $\leq$ 42% is the minimum level of anti-tumor activity. A T/C <10% is considered a high anti-tumor activity level, with T/C (%) = Median tumor volume of the treated / Median tumor volume of the control x 100. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP), a decrease in CA125 in the case of ovarian cancer or any combination thereof.

PFS, DFS, and OS can be measured by standards set by the National Cancer Institute and the U.S. Food and Drug Administration for the approval of new drugs. See Johnson et al, (2003) J. Clin. Oncol. 21(7): 1404-1411.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

"Time to Tumor Progression" (TTP) is defined as the time from enrollment to disease progression. TTP is generally measured using the RECIST 1.1 criteria.

A "complete response" or "complete remission" or "CR" indicates the disappearance of all signs of tumor or cancer in response to treatment This does not always mean the cancer has been cured.

A "partial response" or "PR" refers to a decrease in the size or volume of one or more tumors or lesions, or in the extent of cancer in the body, in response to treatment.

"Stable disease" refers to disease without progression or relapse. In stable disease there is neither sufficient tumor shrinkage to qualify for partial response nor sufficient tumor increase to qualify as progressive disease.

"Progressive disease" refers to the appearance of one more new lesions or tumors and/or the unequivocal progression of existing non-target lesions. Progressive disease can also revert to a tumor growth of more than 20 percent since treatment began, either due to an increases in mass or in spread of the tumor.

"Disease free survival" (DFS) refers to the length of time during and after treatment that the patient remains free of disease.

"Overall Survival" (OS) refers to the time from patient enrollment to death or censored at the date last known alive. OS includes a prolongation in life expectancy as compared to naive or untreated individuals or patients. Overall survival refers to the situation wherein a patient remains alive for a defined period of time, such as one year, five years, etc., c.g., from the time of diagnosis or treatment.

A "decrease in CA125 levels" can be assessed according to the Gynecologic Cancer Intergroup (GCIG) guidelines. For example, CA125 levels can be measured prior to treatment to establish a baseline CA125 level. CA125 levels can be measured one or more times during or after treatment, and a reduction in the CA125 levels over time as compared to the baseline level is considered a decrease in CA125 levels.

Terms such as "treating" or "treatment" or "to treat" or "alleviating" or "to alleviate" refer to therapeutic measures that cure, slow down, lessen symptoms of, and/or halt progression of a diagnosed pathologic condition or disorder. Thus, those in need of treatment include those already diagnosed with or suspected of having the disorder. In certain embodiments, a subject is successfully "treated" for cancer according to the methods of the present invention if the patient shows one or more of the following: a reduction in the number of or complete absence of cancer cells; a reduction in the tumor size; inhibition of or an absence of cancer cell infiltration into peripheral organs including, for example, the spread of cancer into soft tissue and bone; inhibition of or an absence of tumor metastasis; inhibition or an absence of tumor growth; relief of one or more symptoms associated with the specific cancer; reduced morbidity and mortality; improvement in quality of life; reduction in tumorigenicity, tumorigenic frequency, or tumorigenic capacity, of a tumor; reduction in the number or frequency of cancer stem cells in a tumor; differentiation of tumorigenic cells to a non-tumorigenic state; increased progression-free survival (PFS), disease-free survival (DFS), or overall survival (OS), complete response (CR), partial response (PR), stable disease (SD), a decrease in progressive disease (PD), a reduced time to progression (TTP), a decrease in CA125 in the case of ovarian cancer, or any combination thereof.

Prophylactic or preventative measures refer to therapeutic measures that prevent and/or slow the development of a targeted pathologic condition or disorder. Thus, those in need of prophylactic or preventative measures include those prone to have the disorder and those in whom the disorder is to be prevented.

The terms "pre-treat" and "pre-treatment" refer to therapeutic measures that occur prior to the administration of an anti-FOLR1 therapeutic. For example, as described in more detail herein, a prophylactic such as a steroid can administered within about a week, about five days, about three days, about two days, or about one day or 24 hours prior to the administration of the anti-FOLR1 therapeutic. The prophylactic can also be administered prior to the anti-FOLR1 therapeutic on the same day as the anti-FOLR1 therapeutic.

The term "maximum concentration" (Cmax) refers to the highest concentration of drug in the blood that is measured after a dose of the drug.

The term "area-under-the-curve" (AUC) refers to the overall amount of drug in the bloodstream after a dose of the drug. The AUC can be defined over a particular time period. Thus, for example, the $AUC_{0-\infty}$ refers to the overall amount of drug in the bloodstream for an infinite time after a dose of the drug. In another example, the $AUC_{0-24}$ refers to the overall amount of drug in the bloodstream for a period of 24 hours after a dose of the drug. In another example, the $AUC_{0-168}$ refers to the overall amount of drug in the bloodstream for a period of 1 68 hours (or 1 week) after a dose of the drug.

The "apparent volume of distribution at steady state" ($V_{ss}$) refers to the ratio of the total amount of drug in the body to the concentration of the drug in the plasma, or the "apparent" volume necessary to contain the entire amount of a drug, if the drug in the entire body were in the same concentration as in the plasma,

A "chemotherapeutic agent" is a chemical compound useful in the treatment of cancer, regardless of mechanism of action. Chemotherapeutic agents include, for example, antagonists of CD20 such as Rituximab and cyclophosphamide, doxorubicin, vincristine, predinisone, fludarabine, etoposide, methotrexate, lenalidomide, chlorambucil, bentamustine and/or modified versions of such chemotherapeutics.

The terms "polypeptide," "peptide," and "protein" arc used interchangeably herein to refer to polymers of amino acids of any length. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition arc, for example, polypeptides containing one or more analogs of an amino acid (including, for example, unnatural amino acids, etc.), as well as other modifications known in the art. It is understood that, because the polypeptides of this invention are based upon antibodies, in certain embodiments, the polypeptides can occur as single chains or associated chains.

The terms "identical" or percent "identity" in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm described in Karlin et al, 1990, Proc. Natl. Acad. Sci., 87:2264-2268, as modified in Karlin et al., 1993, Proc. Natl. Acad. Sci., 90:5873-5877, and incorporated into the NBLAST and XBLAST programs (Altschul et al., 1991, Nucleic Acids Res., 25:3389-3402). In certain embodiments, Gapped BLAST can be used as described in Altschul et al., 1997, Nucleic Acids Res. 25:3389-3402. BLAST-2, WU-BLAST-2 (Altschul et al., 1996, Methods in Enzymology, 266:460-480), ALIGN, ALIGN-2 (Genentech, South San Francisco,

California) or Megalign (DNASTAR) are additional publicly available software programs that can be used to align sequences. In certain embodiments, the percent identity between two nucleotide sequences is determined using the GAP program in GCG software (e.g., using a NWSgapcina.CMP matrix and a gap weight of 40, 50, 60, 70, or 90 and a length weight of 1, 2, 3, 4, 5, or 6). In certain alternative embodiments, the GAP program in the GCG software package, which incorporates the algorithm of Needleman and Wunsch (J. Mol. Biol. (48):444-453 (1970)) can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, in certain embodiments, the percent identity between nucleotide or amino acid sequences is determined using the algorithm of Myers and Miller (CABIOS, 4:11-17 (1989)). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. In certain embodiments, the default parameters of the alignment software are used. In certain embodiments, the percentage identity "X" of a first amino acid sequence to a second sequence amino acid is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the length of a first sequence is longer than the second sequence, the percent identity of the first sequence to the second sequence will be longer than the percent identity of the second sequence to the first sequence.

As a non-limiting example, whether any particular polynucleotide has a certain percentage sequence identity (e.g., is at least 80% identical, at least 85% identical, at least 90% identical, and in some embodiments, at least 95%, 96%, 97%, 98%, or 99% identical) to a reference sequence can, in certain embodiments, be determined using the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2: 482 489 (1981), to find the best segment of homology between two sequences. When using Bestfit or any other sequence alignment program to determine whether a particular sequence is, for instance, 95% identical to a reference sequence according to the present invention, the parameters are set such that the percentage of identity is calculated over the full length of the reference nucleotide sequence and that gaps in homology of up to 5% of the total number of nucleotides in the reference sequence are allowed.

In some embodiments, two nucleic acids or polypeptides of the invention are substantially identical, meaning they have at least 70%, at least 75%, at least 80%, at least 85%, at least 90%, and in some embodiments at least 95%, 96%, 97%, 98%, 99% nucleotide or amino acid residue identity, when compared and aligned for maximum correspondence, as measured using a sequence comparison algorithm or by visual inspection. Identity can exist over a region of the sequences that is at least about 10, about 20, about 40-60 residues in length or any integral value there between, and can be over a longer region than 60-80 residues, for example, at least about 90-100 residues, and in some embodiments, the sequences are substantially identical over the full length of the sequences being compared, such as the coding region of a nucleotide sequence for example.

A "conservative amino acid substitution" is one in which one amino acid residue is replaced with another amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). For example, substitution of a phenylalanine for a tyrosine is a conservative substitution. In some embodiments, conservative substitutions in the sequences of the polypeptides and antibodies of the invention do not abrogate the binding of the polypeptide or antibody containing the amino acid sequence, to the antigen(s), i.e., the FOLR1 to which the polypeptide or antibody binds. Methods of identifying nucleotide and amino acid conservative substitutions which do not eliminate antigen binding are well-known in the art (see, e.g., Brummell ct al., Biochem. 32: 1180-1 187 (1993); Kobayashi et al. Protein Eng. 12(10):879-884 (1999); and Burks et al. Proc. Natl. Acad. Sci. USA 94:.412-417 (1997)).

As used in the present disclosure and claims, the singular forms "a," "an," and "the" include plural forms unless the context clearly dictates otherwise.

It is understood that wherever embodiments are described herein with the language "comprising," otherwise analogous embodiments described in terms of "consisting of" and/or "consisting essentially of" are also provided.

The term "and/or" as used in a phrase such as "A and/or B" herein is intended to include both "A and B," "A or B," "A," and "B." Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

## II. FOLR1 binding agents

The methods described herein provide methods of administering agents (e.g., antibodies or antigen-binding fragments thereof or polypeptides) that specifically bind FOLR1 ("FOLR1 binding agents"). In certain embodiments, the FOLR1 binding agents are antibodies, immunoconjugates or polypeptides. The amino acid and nucleotide sequences for human FOLR1 are known in the art and are also provided herein as represented by SEQ ID NO:1 and SEQ ID NO:2. Thus, in some embodiments, the FOLR1 binding agents can bind to an epitope found within SEQ ID NO:1.

Examples of therapeutically effective anti-FOLR1 antibodies can be found in US Appl. Pub. No. US 2012/0009181 which is herein incorporated by reference. An example of a therapeutically effective anti-FOLR1 antibody is huMov19 (M9346A). The polypeptides of SEQ ID NOs: 3-5 comprise the variable domain of the heavy chain of huMov19 (M9346A), and the variable domain light chain version 1.00, the variable domain light chain version 1.60 of huMov19, respectively. In certain embodiments, the huMov19 anti-FOLR1 antibody is comprised of a variable domain heavy chain represented by SEQ ID NO:3 and a variable domain light chain represented by SEQ ID NO:5 (version 1.60 ofhuMovl9). In certain embodiments, the huMov19 (M9346A) antibody is encoded by the plasmids deposited with the American Type Culture Collection (ATCC), located at 10801 University Boulevard, Manassas, VA 20110 on April 7, 2010 under the terms of the Budapest Treaty and having ATCC deposit nos. PTA-10772 and PTA-10773 or 10774. Examples of FOLR1 immunoconjugates useful in the therapeutic methods of the invention are provided below.

In some embodiments, the FOLR1 binding agents arc humanized antibodies or antigen-binding fragments thereof. In some embodiments, the humanized antibody or fragment is a resurfaced antibody or antigen-binding fragment thereof. In other embodiments, the FOLR1 binding agent is a fully human antibody or antigen-binding fragment thereof.

In certain embodiments, the FOLR1-binding agents have one or more of the following effects: induce stable disease, inhibit proliferation of tumor cells, reduce the tumorigenicity of a tumor by reducing the frequency of cancer stem cells in the tumor, inhibit tumor growth, increase survival, trigger cell death of tumor cells, differentiate tumorigenic cells to a non-tumorigenic state, or prevent metastasis of tumor cells.

In certain embodiments, a FOLR1-binding agent that is an antibody that has antibody-dependent cellular cytotoxicity (ADCC) activity.

In some embodiments, the FOLR1-binding agents are capable of reducing tumor volume. The ability of a FOLR1-binding agent to reduce tumor volume can be assessed, for example, by measuring a %T/C value, which is the median tumor volume of treated subjects divided by the median tumor volume of the control subjects. In certain embodiments, immunoconjugates or other agents that specifically bind human FOLR1 trigger cell death via a cytotoxic agent. For example, in certain embodiments, an antibody to a human FOLR1 antibody is conjugated to a maytansinoid that is activated in tumor cells expressing the FOLR1 by protein internalization. In certain embodiments, the FOLR1-binding agents are capable of inhibiting tumor growth. In certain embodiments, the FOLR1-binding agents are capable of inhibiting tumor growth *in vivo* (e.g., in a xenograft mouse model and/or in a human having cancer).

The FOLR1 binding molecules can be antibodies or antigen binding fragments that specifically bind to FOLR1 that comprise the CDRs of huMov19 (M9346A) with up to four (i.e. 0, 1, 2, 3, or 4) conservative amino acid substitutions per CDR, e.g., wherein the antibodies or fragments do not comprise the six CDRs of murine Mov19 (i.e., SEQ ID NOs:6-9, 16, and 12). Polypeptides can comprise one of the individual variable light chains or variable heavy chains described herein. Antibodies and polypeptides can also comprise both a variable light chain and a variable heavy chain.

In some embodiments, the FOLR1 binding molecule is an antibody or antigen-binding fragment comprising the sequences of SEQ ID NOs:6-10 and the sequence of SEQ ID NO:12. In some embodiments, the FOLR1 binding molecule is an antibody or antigen-binding fragment comprising the sequences of SEQ ID NOs:6-9 and the sequences of SEQ ID NOs:11 and 12

Also provided are polypeptides that comprise a polypeptide having at least about 90% sequence identity to SEQ ID NO:3, SEQ ID NO:4 or SEQ ID NO:5. In certain embodiments, the polypeptide comprises a polypeptide having at least about 95%, at least about 96%, at least about 97%, at least about 98%, or at least about 99% sequence identity to SEQ ID NO:3, SEQ ID NO:4 or SEQ ID NO:5 Thus, in certain embodiments, the polypeptide comprises (a) a polypeptide having at least about 95% sequence identity to SEQ ID NO:3 and/or (b) a polypeptide having at least about 95% sequence identity to SEQ ID NO:4 or SEQ ID NO:5. In certain embodiments, the polypeptide comprises (a) a polypeptide having the amino acid sequence of SEQ ID NO:3; and/or (b) a polypeptide having the amino acid sequence of SEQ ID NO:4 or SEQ ID NO:5. In certain embodiments, the polypeptide is an antibody and/or the polypeptide specifically binds FOLR1. In certain embodiments, the polypeptide is a murine, chimeric, or humanized antibody that specifically binds FOLR1. In certain embodiments, the polypeptide having a certain percentage of sequence identity to SEQ ID NO:3, SEQ ID NO:4 or SEQ ID NO:5 differs from SEQ ID NO:3, SEQ ID NO:4 or SEQ ID NO:5 by conservative amino acid substitutions only.

Polypeptides can comprise one of the individual light chains or heavy chains described herein. Antibodies and polypeptides can also comprise both a light chain and a heavy chain.

Monoclonal antibodies can be prepared using hybridoma methods, such as those described by Kohler and Milstein (1975) Nature 256:495. Using the hybridoma method, a mouse, hamster, or other appropriate host animal, is

immunized as described above to elicit the production by lymphocytes of antibodies that will specifically bind to an immunizing antigen. Lymphocytes can also be immunized *in vitro.* Following immunization, the lymphocytes are isolated and fused with a suitable myeloma cell line using, for example, polyethylene glycol, to form hybridoma cells that can then be selected away from unfused lymphocytes and myeloma cells. Hybridomas that produce monoclonal antibodies directed specifically against a chosen antigen as determined by immunoprecipitation, immunoblotting, or by an *in vitro* binding assay (e.g. radioimmunoassay (RIA); enzyme-linked immunosorbent assay (ELISA)) can then be propagated either *in vitro* culture using standard methods (Goding, Monoclonal Antibodies: Principles and Practice, Academic Press, 1986) or *in vivo* as ascites tumors in an animal. The monoclonal antibodies can then be purified from the culture medium or ascites fluid as described for polyclonal antibodies above.

Alternatively monoclonal antibodies can also be made using recombinant DNA methods as described in U.S. Patent 4,816,567. The polynucleotides encoding a monoclonal antibody are isolated from mature B-cells or hybridoma cell, such as by RT-PCR using oligonucleotide primers that specifically amplify the genes encoding the heavy and light chains of the antibody, and their sequence is determined using conventional procedures. The isolated polynucleotides encoding the heavy and light chains are then cloned into suitable expression vectors, which when transfected into host cells such as E. coli cells, simian COS cells, Chinese hamster ovary (CHO) cells, or myeloma cells that do not otherwise produce immunoglobulin protein, monoclonal antibodies are generated by the host cells. Also, recombinant monoclonal antibodies or fragments thereof of the desired species can be isolated from phage display libraries expressing CDRs of the desired species as described (McCafferty et al., 1990, Nature, 348:552-554; Clackson et al., 1991, Nature, 352:624-628; and Marks et al., 1991, J. Mol. Biol., 222:581-597).

The polynuclcotidc(s) encoding a monoclonal antibody can further be modified in a number of different manners using recombinant DNA technology to generate alternative antibodies. In some embodiments, the constant domains of the light and heavy chains of, for example, a mouse monoclonal antibody can be substituted 1) for those regions of, for example, a human antibody to generate a chimeric antibody or 2) for a non-immunoglobulin polypeptide to generate a fusion antibody. In some embodiments, the constant regions are truncated or removed to generate the desired antibody fragment of a monoclonal antibody. Site-directed or high-density mutagenesis of the variable region can be used to optimize specificity, affinity, etc. of a monoclonal antibody.

In some embodiments, the monoclonal antibody against the human FOLR1 is a humanized antibody. In some embodiments, the humanized antibody is a resurfaced antibody. In certain embodiments, such antibodies are used therapeutically to reduce antigenicity and HAMA (human antimouse antibody) responses when administered to a human subject. Humanized antibodies can be produced using various techniques known in the art. In certain alternative embodiments, the antibody to FOLR1 is a human antibody.

Human antibodies can be directly prepared using various techniques known in the art. Immortalized human B lymphocytes immunized *in vitro* or isolated from an immunized individual that produce an antibody directed against a target antigen can be generated (See, e.g., Cole et al., Monoclonal Antibodies and Cancer Therapy, Alan R. Liss, p. 77 (1985); Bocmer et al., 1991, J. Immunol., 147 (1):86-95; and U.S. Patent 5,750,373). Also, the human antibody can be selected from a phage library, where that phage library expresses human antibodies, as described, for example, in Vaughan et al., 1996, Nat. Biotech., 14:309-314, Sheets et al., 1998, Proc. Nat'l. Acad. Sci., 95:6157-6162, Hoogenboom and Winter, 1991, J. Mol. Biol., 227:381, and Marks et al., 1991, J. Mol. Biol., 222:581). Techniques for the generation and use of antibody phage libraries are also described in U.S. Patent Nos. 5,969,108, 6,172,197, 5,885,793, 6,521,404; 6,544,731; 6,555,313; 6,582,915; 6,593,081; 6,300,064; 6,653,068; 6,706,484; and 7,264,963; and Rothe et al., 2007, J. Mol. Bio., doi:10.1016/j.jmb.2007.12.018 (each of which is incorporated by reference in its entirety). Affinity maturation strategies and chain shuffling strategics (Marks et al., 1992, BioITcchnology 10:779-783, incorporated by reference in its entirety) are known in the art and can be employed to generate high affinity human antibodies.

Humanized antibodies can also be made in transgenic mice containing human immunoglobulin loci that are capable upon immunization of producing the full repertoire of human antibodies in the absence of endogenous immunoglobulin production. This approach is described in U.S. Patents 5,545,807; 5,545,806; 5,569,825; 5,625,126; 5,633,425; and 5,661,016.

This invention also encompasses bispecific antibodies that specifically recognize a FOLR1. Bispecific antibodies are antibodies that are capable of specifically recognizing and binding at least two different epitopes. The different epitopes can either be within the same molecule (e.g. the same FOLR1) or on different molecules such that both, for example, the antibodies can specifically recognize and bind a FOLR1 as well as, for example, 1) an effector molecule on a leukocyte such as a T-cell receptor (e.g. CD3) or Fc receptor (e.g. CD64, CD32, or CD16) or 2) a cytotoxic agent as described in detail below.

The polypeptides of the present invention can be recombinant polypeptides, natural polypeptides, or synthetic polypeptides comprising an antibody, or fragment thereof, against a human FOLR1.

The polypeptides and analogs can be further modified to contain additional chemical moieties not normally part of the protein. Those derivatized moieties can improve the solubility, the biological half life or absorption of the protein. The moieties can also reduce or eliminate any desirable side effects of the proteins and the like. An overview for those

moieties can be found in REMINGTON'S PHARMACEUTICAL SCIENCES, 20th ed., Mack Publishing Co., Easton, PA (2000).

Methods known in the art for purifying antibodies and other proteins also include, for example, those described in U.S. Patent Publication No. 2008/0312425, 2008/0177048, and 2009/0187005, each of which is hereby incorporated by reference herein in its entirety.

### III. Immunoconjugates

Methods for administering conjugates comprising the anti-FOLR1 antibodies, antibody fragments, and their functional equivalents as disclosed herein, linked or conjugated to a drug or prodrug (also referred to herein as immunoconjugates) are also described herein. Suitable drugs or prodrugs are known in the art. The drugs or prodrugs can be cytotoxic agents. The cytotoxic agent used in the cytotoxic conjugate of the present invention can be any compound that results in the death of a cell, or induces cell death, or in some manner decreases cell viability, and includes, for example, maytansinoids and maytansinoid analogs. Other suitable cytotoxic agents are for example benzodiazepines, taxoids, CC-1065 and CC-1065 analogs, duocarmycins and duocarmycin analogs, enediynes, such as calicheamicins, dolastatin and dolastatin analogs including auristatins, tomaymycin derivaties, leptomycin derivaties, methotrexate, cisplatin, carboplatin, daunorubicin, doxorubicin, vincristine, vinblastine, melphalan, mitomycin C, chlorambucil and morpholino doxorubicin.

Such conjugates can be prepared by using a linking group in order to link a drug or prodrug to the antibody or functional equivalent. Suitable linking groups are well known in the art and include, for example, disulfide groups, thioether groups, acid labile groups, photolabile groups, peptidase labile groups and esterase labile groups.

The drug or prodrug can, for example, be linked to the anti-FOLR1 antibody or fragment thereof through a disulfide bond. The linker molecule or crosslinking agent comprises a reactive chemical group that can react with the anti-FOLR1 antibody or fragment thereof. The reactive chemical groups for reaction with the cell-binding agent can be N-succinimidyl esters and *N*-sulfosuccinimidyl esters. Additionally the linker molecule comprises a reactive chemical group, which can be a dithiopyridyl group that can react with the drug to form a disulfide bond. Linker molecules include, for example, *N*-succinimidyl 3-(2-pyridyldithio) propionate (SPDP) (see, e.g., Carlsson et al., Biochem. J., 173: 723-737 (1978)), *N*-succinimidyl 4-(2-pyridyldithio)butanoate (SPDB) (see, e.g., U.S. Patent No. 4,563,304), *N*-succinimidyl 4-(2-pyridyldithio)2-sulfobutanoate (sulfo-SPDB) (see US Publication No. 20090274713) , *N*-succinimidyl 4-(2-pyridyldithio) pentanoate (SPP) (sec, c.g., CAS Registry number 341498-08-6), 2-iminothiolane, or acetylsuccinic anhydride. For example, the antibody or cell binding agent can be modified with crosslinking reagents and the antibody or cell binding agent containing free or protected thiol groups thus derived is then reacted with a disulfide- or thiol-containing maytansinoid to produce conjugates. The conjugates can be purified by chromatography, including but not limited to HPLC, size-exclusion, adsorption, ion exchange and affinity capture, dialysis or tangential flow filtration.

In another aspect of the present invention, the anti-FOLR1 antibody is linked to cytotoxic drugs via disulfide bonds and a polyethylene glycol spacer in enhancing the potency, solubility or the efficacy of the immunoconjugate. Such cleavable hydrophilic linkers are described in WO2009/0134976. The additional benefit of this linker design is the desired high monomer ratio and the minimal aggregation of the antibody-drug conjugate. Specifically contemplated in this aspect are conjugates of cell-binding agents and drugs linked via disulfide group (-S-S-) bearing polyethylene glycol spacers ($(CH_2CH_2O)_{n=1-14}$) with a narrow range of drug load of 2-8 are described that show relatively high potent biological activity toward cancer cells and have the desired biochemical properties of high conjugation yield and high monomer ratio with minimal protein aggregation.

Antibody-maytansinoid conjugates with non-cleavable linkers can also be prepared. Such crosslinkers are described in the art (see US Publication No. 20050169933) and include but are not limited to, *N*-succinimidyl 4-(maleimidomethyl) cyclohexanecarboxylate (SMCC). In some embodiments, the antibody is modified with crosslinking reagents such as succinimidyl 4-(N-maleimidomethyl)-cyclohexane-1-carboxylate (SMCC), sulfo-SMCC, maleimidobenzoyl-N-hydroxysuccinimide ester (MBS), sulfo-MBS or succinimidyl-iodoacetate, as described in the literature, to introduce 1-10 reactive groups (Yoshitakc et al, Eur. J. Biochem., 101:395-399 (1979); Hashida et al, J. Applied Biochem., 56-63 (1984); and Liu et al, Biochem., 18:690-697 (1979)). The modified antibody is then reacted with the thiol-containing maytansinoid derivative to produce a conjugate. The conjugate can be purified by gel filtration through a Sephadex G25 column or by dialysis or tangential flow filtration. The modified antibodies are treated with the thiol-containing maytansinoid (1 to 2 molar equivalent/malcimido group) and antibody-maytansinoid conjugates are purified by gel filtration through a Sephadex G-25 column, chromatography on a ceramic hydroxyapatite column, dialysis or tangential flow filtration or a combination of methods thereof. Typically, an average of 1-10 maytansinoids per antibody arc linked. One method is to modify antibodies with succinimidyl 4-(N-maleimidomethyl)-cyclohexane-1-carboxylate (SMCC) to introduce malcimido groups followed by reaction of the modified antibody with a thiol-containing maytansinoid to give a thioether-linkcd conjugate. Again conjugates with 1 to 10 drug molecules per antibody molecule result. Maytansinoid conjugates of antibodies, antibody fragments, and other proteins are made in the same way.

In another aspect of the invention, the FOLR1 antibody is linked to the drug via a non-cleavable bond through the intermediacy of a PEG spacer. Suitable crosslinking reagents comprising hydrophilic PEG chains that form linkers between a drug and the anti-FOLR1 antibody or fragment are also well known in the art, or are commercially available (for example from Quanta Biodesign, Powell, Ohio). Suitable PEG-containing crosslinkers can also be synthesized from commercially available PEGs themselves using standard synthetic chemistry techniques known to one skilled in the art. The drugs can be reacted with bifunctional PEG-containing cross linkers to give compounds of the following formula, Z $-X_1-(-CH_2-CH_2-O-)_n-Y_P-D$, by methods described in detail in US Patent Publication 20090274713 and in WO2009/0134976, which can then react with the cell binding agent to provide a conjugate. Alternatively, the cell binding can be modified with the bifunctional PEG crosslinker to introduce a thiol-reactive group (such as a maleimide or haloacetamide) which can then be treated with a thiol-containing maytansinoid to provide a conjugate. In another method, the cell binding can be modified with the bifunctional PEG crosslinker to introduce a thiol moiety which can then be treated with a thiol-reactive maytansinoid (such as a maytansinoid bearing a maleimide or haloacetamide), to provide a conjugate.

Examples of suitable PEG-containing linkers include linkers having an *N*-succinimidyl ester or *N*-sulfosuccinimidyl ester moiety for reaction with the anti-FOLR1 antibody or fragment thereof, as well as a maleimido- or haloacetyl-based moiety for reaction with the compound. A PEG spacer can be incorporated into any crosslinker known in the art by the methods described herein.

In some embodiments, the linker is a linker containing at least one charged group as described, for example, in U.S. Patent Publication No. 2012/0282282, the contents of which are entirely incorporated herein by reference. In some embodiments, the charged or pro-charged cross-linkers are those containing sulfonate, phosphate, carboxyl or quaternary amine substituents that significantly increase the solubility of the modified cell-binding agent and the cell-binding agent-drug conjugates, especially for monoclonal antibody-drug conjugates with 2 to 20 drugs/antibody linked. Conjugates prepared from linkers containing a pro-charged moiety would produce one or more charged moieties after the conjugate is metabolized in a cell. In some embodiments, the linker is selected from the group consisting of: N-succinimidyl 4-(2-pyridyldithio)-2-sulfopcntanoatc (sulfo-SPP) and N-succinimidyl 4-(2-pyridyldithio)-2-sulfobutanoatc (sulfo-SPDB).

Many of the linkers disclosed herein are described in detail in U.S. Patent Publication Nos. 2005/0169933, 2009/0274713, and 2012/0282282, and in WO2009/0134976; the contents of which are entirely incorporated herein by reference.

The present invention includes aspects wherein about 2 to about 8 drug molecules ("drug load"), for example, maytansinoid, are linked to an anti-FOLR1 antibody or fragment thereof. "Drug load", as used herein, refers to the number of drug molecules (e.g., a maytansinoid) that can be attached to a cell binding agent (e.g., an anti-FOLR1 antibody or fragment thereof). In one aspect, the number of drug molecules that can be attached to a cell binding agent can average from about 2 to about 8 (e.g., 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1). N2'-deacetyl-N2'-(3-mercapto-1-oxopropyl)-maytansine (DM1) and N2'-deacetyl-N2'-(4-mercapto-4-methyl-1-oxopentyl) maytansine (DM4) can be used.

Thus, in one aspect, an immunoconjugate comprises 1 maytansinoid per antibody. In another aspect, an immunoconjugate comprises 2 maytansinoids per antibody. In another aspect, an immunoconjugate comprises 3 maytansinoids per antibody. In another aspect, an immunoconjugate comprises 4 maytansinoids per antibody. In another aspect, an immunoconjugate comprises 5 maytansinoids per antibody. In another aspect, an immunoconjugate comprises 6 maytansinoids per antibody. In another aspect, an immunoconjugate comprises 7 maytansinoids per antibody. In another aspect, an immunoconjugate comprises 8 maytansinoids per antibody.

In one aspect, an immunoconjugate comprises about 1 to about 8 maytansinoids per antibody. In another aspect, an immunoconjugate comprises about 2 to about 7 maytansinoids per antibody. In another aspect, an immunoconjugate comprises about 2 to about 6 maytansinoids per antibody. In another aspect, an immunoconjugate comprises about 2 to about 5 maytansinoids per antibody. In another aspect, an immunoconjugate comprises about 3 to about 5 maytansinoids per antibody. In another aspect, an immunoconjugate comprises about 3 to about 4 maytansinoids per antibody.

In one aspect, a composition comprising immunoconjugates has an average of about 2 to about 8 (e.g., 1.9, 2.0, 2.1, 2.2, 2.3, 2.4, 2.5, 2.6, 2.7, 2.8, 2.9, 3.0, 3.1, 3.2, 3.3, 3.4, 3.5, 3.6. 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1) drug molecules (e.g., maytansinoids) attached per antibody. In one aspect, a composition comprising immunoconjugates has an average of about 1 to about 8 drug molecules (e.g., maytansinoids) per antibody. In one aspect, a composition comprising immunoconjugates has an average of about 2 to about 7 drug molecules (e.g., maytansinoids) per antibody. In one aspect, a composition comprising immunoconjugates has an average of about 2 to about 6 drug molecules (e.g., maytansinoids) per antibody. In one aspect, a composition comprising immunoconjugates has an average of about 2 to about 5 drug molecules (e.g., maytansinoids) per antibody. In one

aspect, a composition comprising immunoconjugates has an average of about 3 to about 5 drug molecules (e.g., maytansinoids) per antibody. In one aspect, a composition comprising immunoconjugates has an average of about 3 to about 4 drug molecules (e.g., maytansinoids) per antibody.

In one aspect, a composition comprising immunoconjugates has an average of about 2 $\pm$ 0.5, about 3 $\pm$ 0.5, about 4 $\pm$ 0.5, about 5 $\pm$ 0.5, about 6 $\pm$ 0.5, about 7 $\pm$ 0.5, or about 8 $\pm$ 0.5 drug molecules (e.g., maytansinoids) attached per antibody. In one aspect, a composition comprising immunoconjugates has an average of about 3.5 $\pm$ 0.5 drug molecules (e.g., maytansinoids) per antibody.

The anti-FOLR1 antibody or fragment thereof can be modified by reacting a bifunctional crosslinking reagent with the anti-FOLR1 antibody or fragment thereof, thereby resulting in the covalent attachment of a linker molecule to the anti-FOLR1 antibody or fragment thereof. As used herein, a "bifunctional crosslinking reagent" is any chemical moiety that covalently links a cell-binding agent to a drug, such as the drugs described herein. In another method, a portion of the linking moiety is provided by the drug. In this respect, the drug comprises a linking moiety that is part of a larger linker molecule that is used to join the cell-binding agent to the drug. For example, to form the maytansinoid DM1, the side chain at the C-3 hydroxyl group of maytansinc is modified to have a free sulfhydryl group (SH). This thiolated form of maytansine can react with a modified cell-binding agent to form a conjugate. Therefore, the final linker is assembled from two components, one of which is provided by the crosslinking reagent, while the other is provided by the side chain from DM1.

The drug molecules can also be linked to the antibody molecules through an intermediary carrier molecule such as serum albumin.

As used herein, the expression "linked to a cell-binding agent" or "linked to an anti-FOLR1 antibody or fragment" refers to the conjugate molecule comprising at least one drug derivative bound to a cell-binding agent anti-FOLR1 antibody or fragment via a suitable linking group, or a precursor thereof. Exemplary linking groups are SPDB or sulfo-SPDB.

In certain embodiments, cytotoxic agents useful in the present invention are maytansinoids and maytansinoid analogs. Examples of suitable maytansinoids include esters of maytansinol and maytansinol analogs. Included are any drugs that inhibit microtubule formation and that are highly toxic to mammalian cells, as are maytansinol and maytansinol analogs.

Examples of suitable maytansinol esters include those having a modified aromatic ring and those having modifications at other positions. Such suitable maytansinoids are disclosed in U.S. Patent Nos. 4,424,219: 4,256,746; 4,294,757; 4,307,016; 4,313,946; 4,315,929; 4,331,598; 4,361,650; 4,362,663; 4,364,866; 4,450,254; 4,322,348; 4,371,533; 5,208,020; 5,416,064; 5,475,092; 5,585,499; 5,846,545; 6,333,410; 7,276,497 and 7,473,796.

In a certain embodiment, the immunoconjugates of the invention utilize the thiol-containing maytansinoid (DM1), formally termed *N*$^{2'}$-deacetyl-*N*$^{2'}$-(3-mercapto-1- oxopropyl)-maytansine, as the cytotoxic agent. DM1 is represented by the following structural formula (1):

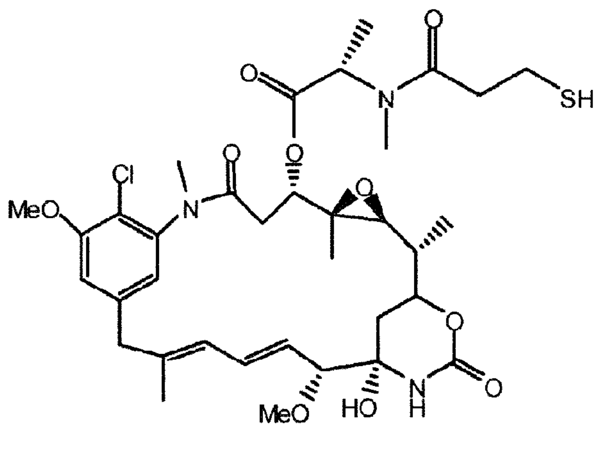

(I)

In another embodiment, the conjugates of the present invention utilize the thiol-containing maytansinoid *N*$^{2'}$-deacetyl-*N*$^{2'}$(4-methyl-4-mercapto-1- oxopentyl)-maytansine (e.g., DM4) as the cytotoxic agent. DM4 is represented by the following structural formula (II):

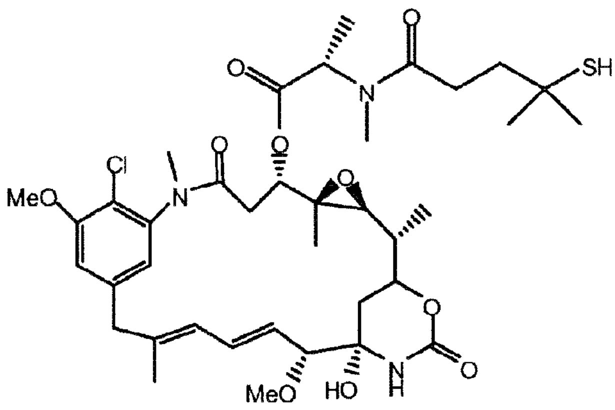

(II)

Another maytansinoid comprising a side chain that contains a stcrically hindered thiol bond is *N*2'-deacetyl-*N*-2'(4-mercapto-1-oxopentyl)-maytansine (termed DM3), represented by the following structural formula (III):

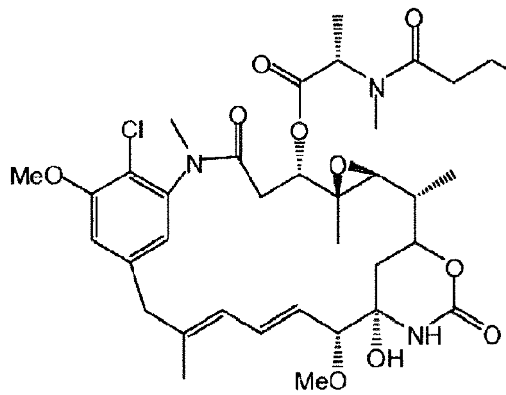

(III)

Each of the maytansinoids taught in US Patent No. 5,208,020 and 7,276,497, can also be used in the conjugate of the present invention. In this regard, the entire disclosure of 5,208,020 and 7,276,697 is incorporated herein by reference.

Many positions on maytansinoids can serve as the position to chemically link the linking moiety. For example, the C-3 position having a hydroxyl group, the C-14 position modified with hydroxymethyl, the C-15 position modified with hydroxy and the C-20 position having a hydroxy group are all expected to be useful. In some embodiments, the C-3 position serves as the position to chemically link the linking moiety, and in some particular embodiments, the C-3 position of maytansinol serves as the position to chemically link the linking moiety.

Structural representations of some conjugates are shown below:

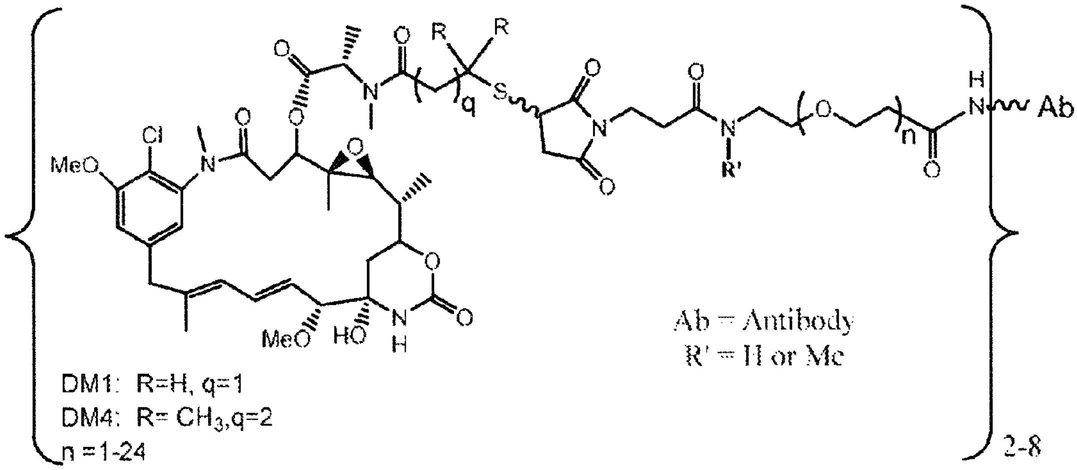

DM1: R=H, q=1
DM4: R= $CH_3$,q=2
n =1-24

Ab = Antibody
R' = H or Me

Ab-PEG-Mal-DM1/DM4

(IV)

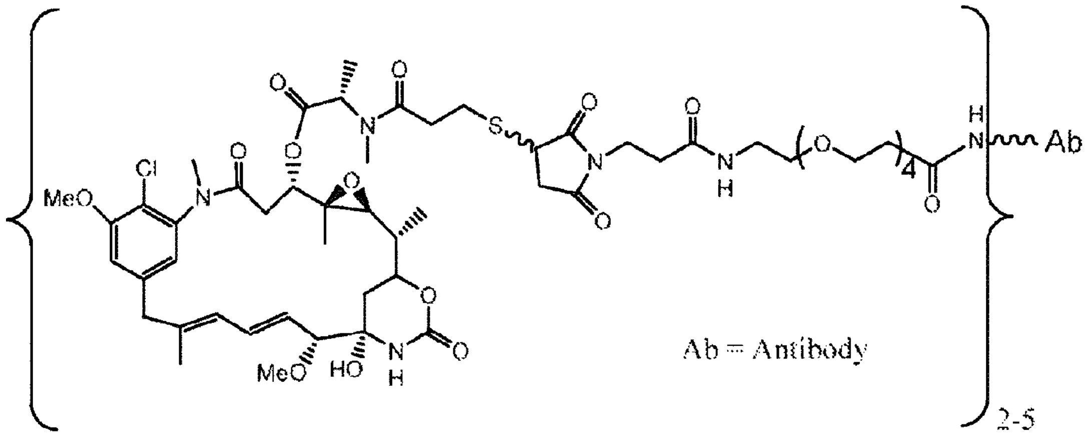

Ab = Antibody

Ab-PEG4-Mal-DM1

(V)

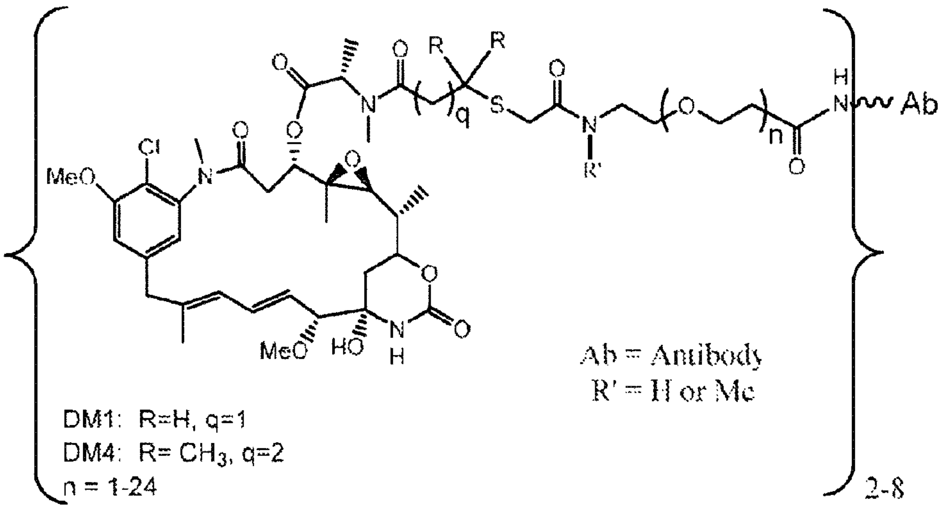

DM1: R=H, q=1
DM4: R= $CH_3$, q=2
n = 1-24

Ab = Antibody
R' = H or Me

Ab-PEG-SIA-DM1/DM4

(VI)

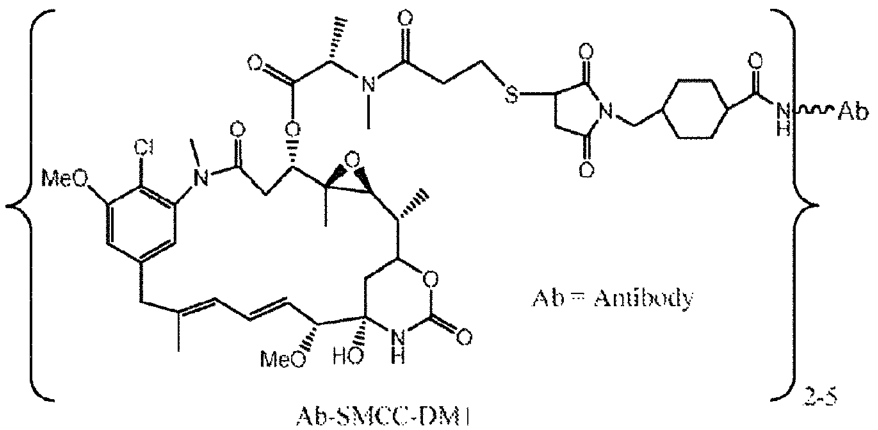


Ab-SMCC-DM1 (VII)

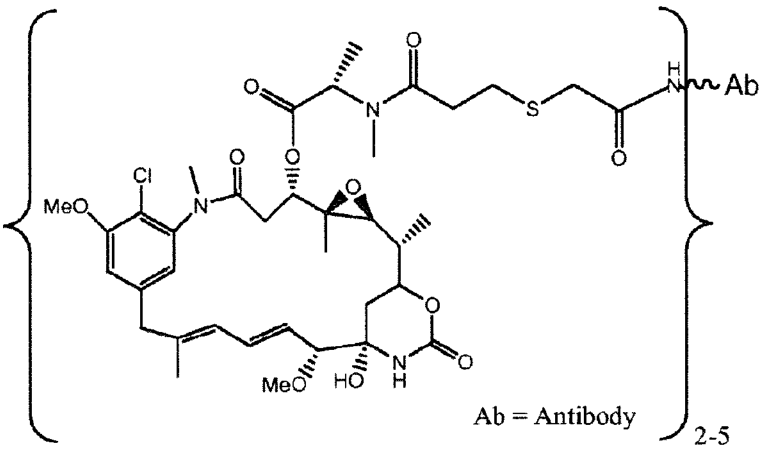


Ab-SIA-DM1 (VIII)

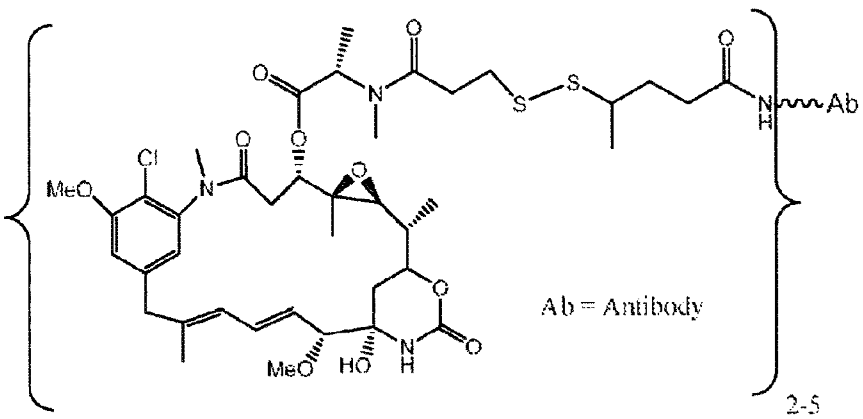


Ab-SPP-DM1 (IX)

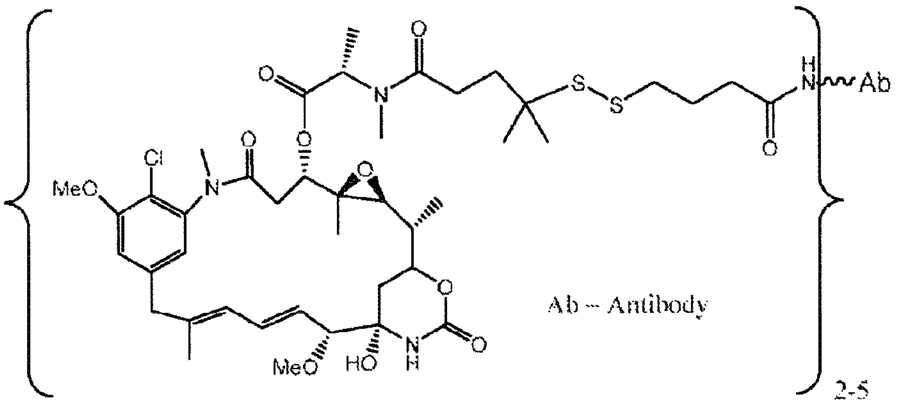


Ab-SPDB-DM4 (X)

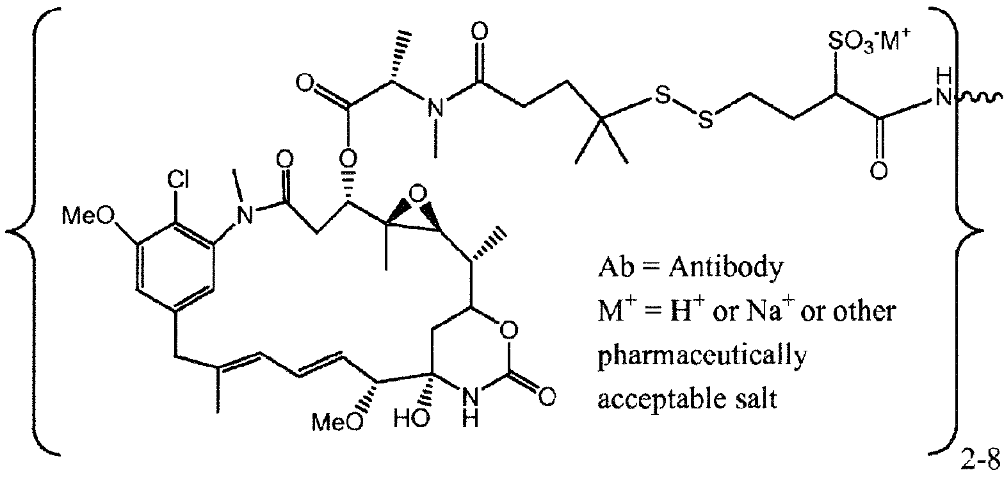


Ab-sulfo-SPDB-DM4 (XI)

Also included in the present invention are any stereoisomers and mixtures thereof for any compounds or conjugates depicted by any structures above.

Several descriptions for producing such antibody-maytansinoid conjugates are provided in U.S. Patent Nos. 6,333,410, 6,441,163, 6,716,821, and 7,368,565, each of which is incorporated herein in its entirety.

In general, a solution of an antibody in aqueous buffer can be incubated with a molar excess of maytansinoids having a disulfide moiety that bears a reactive group. The reaction mixture can be quenched by addition of excess amine (such as ethanolamine, taurine, etc.). The maytansinoid-antibody conjugate can then be purified by gel filtration.

The number of maytansinoid molecules bound per antibody molecule can be determined by measuring spectrophotometrically the ratio of the absorbance at 252 nm and 280 nm. The average number of maytansinoid molecules/antibody can be, for example, 1-10 or 2-5. The average number of maytansinoid molecules/antibody can be, for example about 3 to about 4. The average number of maytansinoid molecules/antibody can be about 3.5.

Conjugates of antibodies with maytansinoid or other drugs can be evaluated for their ability to suppress proliferation of various unwanted cell lines *in vitro*. For example, cell lines such as the human lymphoma cell line Daudi and the human lymphoma cell line Ramos, can easily be used for the assessment of cytotoxicity of these compounds. Cells to be evaluated can be exposed to the compounds for 4 to 5 days and the surviving fractions of cells measured in direct assays by known methods. $IC_{50}$ values can then be calculated from the results of the assays.

The immunoconjugates can, according to some embodiments described herein, be internalized into cells. The immunoconjugate, therefore, can exert a therapeutic effect when it is taken up by, or internalized, by a FOLR1-expressing cell In some particular embodiments, the immunoconjugate comprises an antibody, antibody fragment, or polypeptide, linked to a cytotoxic agent by a cleavable linker, and the cytotoxic agent is cleaved from the antibody, antibody fragment, or polypeptide, wherein it is internalized by a FOLR1-expressing cell.

In some embodiments, the immunoconjugates are capable of reducing tumor volume. For example, in some embodiments, treatment with an immunoconjugate results in a %T/C value that is less than about 50%, less than about

45%, less than about 40%, less than about 35%, less than about 30%, less than about 25%, less than about 20%, less than about 15%, less than about 10%, or less than about 5%. In some particular embodiments, the immunoconjugates can reduce tumor size in a KB, OVCAR-3, IGROV-1, and/or OV-90 xenograft model. In some embodiments, the immunoconjugates are capable of inhibiting metastases.

### III. Methods of administering FOLR1-binding agents

The FOLR1-binding agents (including antibodies, immunoconjugates, and polypeptides) of the invention are useful in a variety of applications including, but not limited to, therapeutic treatment methods, such as the treatment of cancer. In certain embodiments, the agents are useful for inhibiting tumor growth, inducing differentiation, inhibiting metastases, reducing tumor volume, and/or reducing the tumorigenicity of a tumor. The methods of use can be *in vivo* methods.

According to the methods described herein, the FOLR1-binding agents can be administered at particular dosages. For example, the FOLR1-binding agents (e.g., IMGN853) can be administered at a dose of about 0.15 mg/kg to about 7 mg/kg, wherein the kilograms of body weight are adjusted to ideal body weight (IBW), lean body weight (LBW), or adjusted ideal body weight (AIBW or ADJ). In some embodiments, the FOLR1-binding agents (e.g., IMGN853) are administered at a dose of about 3.0 mg/kg to about 6.0 mg/kg, wherein the kilograms of body weight are adjusted to IBW, LBW, or AIBW (ADJ). In some embodiments, the FOLR1-binding agents (e.g., IMGN853) are administered at a dose of about 3.3 mg/kg to about 6.0 mg/kg, wherein the kilograms of body weight are adjusted to IBW, LBW, or AIBW (ADJ). In some embodiments, the FOLR1-binding agents (e.g., IMGN853) are administered at about 0.15 mg/kg, wherein the kilograms of body weight are adjusted to IBW, LBW, or AIBW (ADJ). In some embodiments, the FOLR1-binding agents (e.g., IMGN853) are administered at about 0.5 mg/kg, wherein the kilograms of body weight are adjusted to IBW, LBW, or AIBW (ADJ). In some embodiments, the FOLR1-binding agents (e.g., IMGN853) are administered at about 1.0 mg/kg, wherein the kilograms of body weight are adjusted to IBW, LBW, or AIBW (ADJ). In some embodiments, the FOLR1-binding agents (e.g., IMGN853) are administered at about 1.1 mg/kg, wherein the kilograms of body weight are adjusted to IBW, LBW, or AIBW (ADJ). In some embodiments, the FOLR1-binding agents (e.g., IMGN853) are administered at about 1.5 mg/kg, wherein the kilograms of body weight are adjusted to IBW, LBW, or AIBW (ADJ). In some embodiments, the FOLR1-binding agents (e.g., IMGN853) are administered at about 1.8 mg/kg, wherein the kilograms of body weight are adjusted to IBW, LBW, or AIBW (ADJ). In some embodiments, the FOLR1-binding agents (e.g., 1MGN853) are administered at about 2.0 mg/kg, wherein the kilograms of body weight are adjusted to IBW, LBW, or AIBW (ADJ). In some embodiments, the FOLR1-binding agents (e.g., 1MGN853) are administered at about 2.5 mg/kg, wherein the kilograms of body weight arc adjusted to IBW, LBW, or AIBW (ADJ). In some embodiments, the FOLR1-binding agents (e.g., 1MGN853) are administered at about 2.8 mg/kg, wherein the kilograms of body weight are adjusted to IBW, LBW, or AIBW (ADJ). In some embodiments, the FOLR1-binding agents (e.g., IMGN853) are administered at about 3.0 mg/kg, wherein the kilograms of body weight are adjusted IBW, LBW, or AIBW (ADJ). In some embodiments, the FOLR1-binding agents (e.g., IMGN853) are administered at about 3.3 mg/kg, wherein the kilograms of body weight are adjusted to IBW, LBW, or AIBW (ADJ). In some embodiments, the FOLR1-binding agents (e.g., IMGN853) are administered at about 3.75 mg/kg, wherein the kilograms of body weight are adjusted to IBW, LBW, or AIBW (ADJ). In some embodiments, the FOLR1-binding agents (e.g., IMGN853) are administered at about 4.2 mg/kg, wherein the kilograms of body weight are adjusted to IBW, LBW, or AIBW (ADJ). In some embodiments, the FOLR1-binding agents (e.g., IMGN853) are administered at about 4.5 mg/kg, wherein the kilograms of body weight are adjusted to IBW, LBW, or AIBW (ADJ). In some embodiments, the FOLR1-binding agents (e.g., IMGN853) are administered at about 4.8 mg/kg, wherein the kilograms of body weight are adjusted to IBW, LBW, or AIBW (ADJ). In some embodiments, the FOLR1-binding agents (e.g., IMGN853) are administered at about 5.0 mg/kg, wherein the kilograms of body weight are adjusted to IBW, LBW, or AIBW (ADJ). In some embodiments, the FOLR1-binding agents (e.g., IMGN853) are administered at about 5.5 mg/kg, wherein the kilograms of body weight are adjusted to IBW, LBW, or AIBW (ADJ). In some embodiments, the FOLR1-binding agents (e.g., IMGN853) are administered at about 5.6 mg/kg, wherein the kilograms of body weight are adjusted to IBW, LBW, or AIBW (ADJ). In some embodiments, the FOLR1-binding agents (e.g., IMGN853) are administered at about 6.0 mg/kg, wherein the kilograms of body weight are adjusted to IBW, LBW, or AIBW (ADJ). In some embodiments, the FOLR1-binding agents (e.g., IMGN853) are administered at about 6.1 mg/kg, wherein the kilograms of body weight are adjusted to IBW, LBW, or AIBW (ADJ). In some embodiments, the FOLR1-binding agents (e.g., 1MGN853) are administered at about 6.2 mg/kg, wherein the kilograms of body weight are adjusted to IBW, LBW, or AIBW (ADJ). In some embodiments, the FOLR1-binding agents (e.g., IMGN853) are administered at about 6.3 mg/kg, wherein the kilograms of body weight are adjusted to IBW, LBW, or AIBW (ADJ). In some embodiments, the FOLR1-binding agents (e.g., IMGN853) are administered at about 6.4 mg/kg, wherein the kilograms of body weight are adjusted to IBW, LBW, or AIBW (ADJ). In some embodiments, the FOLR1-binding agents (e.g., IMGN853) are administered at about 6.5 mg/kg, wherein the kilograms of body weight are adjusted to IBW, LBW, or AIBW (ADJ). In some embodiments, the FOLR1-binding agents (e.g., IMGN853) are administered at about 6.6 mg/kg, wherein the kilograms of body weight

are adjusted to IBW, LBW, or AIBW (ADJ). In some embodiments, the FOLR1-binding agents (e.g., IMGN853) are administered at about 6.7 mg/kg, wherein the kilograms of body weight are adjusted to IBW, LBW, or AIBW (ADJ). In some embodiments, the FOLR1-binding agents (e.g., IMGN853) arc administered at about 6.8 mg/kg, wherein the kilograms of body weight are adjusted to IBW, LBW, or AIBW (ADJ). In some embodiments, the FOLR1-binding agents (e.g., 1MGN853) are administered at about 6.9 mg/kg, wherein the kilograms of body weight are adjusted to IBW, LBW, or AIBW (ADJ). In some embodiments, the FOLR1-binding agents (e.g., IMGN853) are administered at about 7.0 mg/kg, wherein the kilograms of body weight are adjusted to IBW, LBW, or AIBW (ADJ). In some embodiments, the kilograms of body weight are adjusted to AIBW (ADJ).

Furthermore, the FOLR1-binding agents can be administered at particular dose interval. For example, the FOLR1-binding agents can be administered from about four times a week to about once every four weeks. Thus, in some embodiments, the FOLR1-binding agents are administered about once every three weeks. In some embodiments, the FOLR1-binding agents are administered about once every two and a half weeks. In some embodiments, the FOLR1-binding agents are administered about once every two weeks. In some embodiments, the FOLR1-binding agents are administered about once every ten days. In some embodiments, the FOLR1-binding agents are administered about once every week.

The FOLR1-binding agents can also be administered in an about 3-wcek (i.e. about 21-day) cycle. For example, the FOLR1-binding agents can be administered twice in about 3 weeks. Thus, in some embodiments, the FOLR1-binding agents can be administered at about days 1 and 8 of a 21-day cycle. In other embodiments, the FOLR1-binding agents can be administered three times in about 3 weeks. Thus, in some embodiments, the FOLR1-binding agents can be administered at about days 1, 8, and 15 of a 21-day cycle.

The FOLR1-binding agents can also be administered in an about 4-week (i.e. about 28-day) cycle. For example, the FOLR1-binding agents can be administered three times in about 4 weeks. Thus, in some embodiments, the FOLR1-binding agents can be administered at about days 1, 8, and 15 of a 28-day cycle.

According to the methods described herein, the FOLR1-binding agents can be administered at particular dosages. For example, the FOLR1-binding agents (e.g., IMGN853) can be administered at a dose of about 0.15 mg/kg to about 7 mg/kg once a week for three weeks on a four-week schedule. In some embodiments, the FOLR1-binding agents (e.g., IMGN853) are administered at a dose of about 3.0 mg/kg to about 6.0 mg/kg once a week for three weeks on a four-week schedule. In some embodiments, the FOLR1-binding agents (e.g., IMGN853) are administered at a dose of about 3.3 mg/kg to about 6.0 mg/kg once a week for three weeks on a four-week schedule. In some embodiments, the FOLR1-binding agents (e.g., IMGN853) are administered at about 0.15 mg/kg once a week for three weeks on a four-week schedule. In some embodiments, the FOLR1-binding agents (e.g., IMGN853) are administered at about 0.5 mg/kg once a week for three weeks on a four-week schedule. In some embodiments, the FOLR1-binding agents (e.g., IMGN853) are administered at about 1.0 mg/kg once a week for three weeks on a four-week schedule. In some embodiments, the FOLR1-binding agents (e.g., 1MGN853) are administered at about 1.1 mg/kg once a week for three weeks on a four-week schedule. In some embodiments, the FOLR1-binding agents (e.g., IMGN853) are administered at about 1.5 mg/kg once a week for three weeks on a four-week schedule. In some embodiments, the FOLR1-binding agents (e.g., IMGN853) are administered at about 1.8 mg/kg once a week for three weeks on a four-week schedule. In some embodiments, the FOLR1-binding agents (e.g., IMGN853) are administered at about 2.0 mg/kg once a week for three weeks on a four-week schedule. In some embodiments, the FOLR1-binding agents (e.g., IMGN853) are administered at about 2.5 mg/kg once a week for three weeks on a four-week schedule. In some embodiments, the FOLR1-binding agents (e.g., IMGN853) are administered at about 2.8 mg/kg once a week for three weeks on a four-week schedule. In some embodiments, the FOLR1-binding agents (e.g., IMGN853) are administered at about 3.0 mg/kg once a week for three weeks on a four-week schedule. In some embodiments, the FOLR1-binding agents (e.g., IMGN853) are administered at about 3.3 mg/kg once a week for three weeks on a four-week schedule. In some embodiments, the FOLR1-binding agents (e.g., IMGN853) are administered at about 3.75 mg/kg once a week for three weeks on a four-week schedule.. In some embodiments, the FOLR1-binding agents (e.g., 1MGN853) are administered at about 4.2 mg/kg once a week for three weeks on a four-week schedule. In some embodiments, the FOLR1 -binding agents (e.g., IMGN853) are administered at about 4.5 mg/kg once a week for three weeks on a four-week schedule. In some embodiments, the FOLR1-binding agents (e.g., IMGN853) are administered at about 4.8 mg/kg once a week for three weeks on a four-week schedule. In some embodiments, the FOLR1 -binding agents (e.g., IMGN853) are administered at about 5.0 mg/kg once a week for three weeks on a four-week schedule. In some embodiments, the FOLR1-binding agents (e.g., 1MGN853) are administered at about 5.5 mg/kg once a week for three weeks on a four-week schedule. In some embodiments, the FOLR1-binding agents (e.g., IMGN853) are administered at about 5.6 mg/kg once a week for three weeks on a four-week schedule. In some embodiments, the FOLR1-binding agents (e.g., IMGN853) are administered at about 6.0 mg/kg once a week for three weeks on a four-week schedule. In some embodiments, the FOLR1-binding agents (e.g., IMGN853) are administered at about 6.1 mg/kg once a week for three weeks on a four-week schedule. In some embodiments, the FULR1-binding agents (e.g., 1MGN853) are administered at about 6.2 mg/kg once a week for three weeks on a four-week schedule. In some embodiments, the FOLR1-binding agents (e.g., IMGN853) are administered at about 6.3 mg/kg once a week for

three weeks on a four-week schedule. In some embodiments, the FOLR1-binding agents (e.g., IMGN853) are administered at about 6.4 mg/kg once a week for three weeks on a four-week schedule. In some embodiments, the FOLR1-binding agents (e.g., IMGN853) are administered at about 6.5 mg/kg once a week for three weeks on a four-week schedule. In some embodiments, the FOLR1-binding agents (e.g., IMGN853) are administered at about 6.6 mg/kg once a week for three weeks on a four-week schedule. In some embodiments, the FOLR1-binding agents (e.g., IMGN853) are administered at about 6.7 mg/kg once a week for three weeks on a four-week schedule. In some embodiments, the FOLR1-binding agents (e.g., IMGN853) are administered at about 6.8 mg/kg once a week for three weeks on a four-week schedule. In some embodiments, the FOLR1-binding agents (e.g., 1MGN853) are administered at about 6.9 mg/kg once a week for three weeks on a four-week schedule. In some embodiments, the FOLR1-binding agents (e.g., IMGN853) are administered at about 7.0 mg/kg once a week for three weeks on a four-week schedule.

According to the methods described herein, the FOLR1-binding agents (e.g., IMGN853) can be administered at a dose of about 0.15 mg/kg to about 7 mg/kg once a week for three weeks on a four-week schedule, wherein the kilograms of body weight are adjusted to ideal body weight (IBW), lean body weight (LBW), or adjusted ideal body weight (AIBW or ADJ). In some embodiments, the FOLR1-binding agents (e.g., IMGN853) are administered at a dose of about 3.0 mg/kg to about 6.0 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at a dose of about 3.3 mg/kg to about 6.0 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 0.15 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 0.5 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 1.0 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 1.1 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 1.5 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 1.8 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 2.0 mg/kg once a week for three weeks on a four-week schedule, wherein the kilograms of body weight arc adjusted to IBW, LBW, or AIBW (ADJ). In some embodiments, the FOLR1-binding agents (e.g., IMGN853) are administered at about 2.5 mg/kg once a week for three weeks on a four-week schedule, wherein the kilograms of body weight are adjusted to TBW, LBW, or ATBW (ADJ). In some embodiments, the FOLR1-binding agents (e.g., IMGN853) are administered at about 2.8 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 3.0 mg/kg once a week for three weeks on a four-week schedule, wherein the kilograms of body weight are adjusted IBW, LBW, or AIBW (ADJ). In some embodiments, the FOLR1-binding agents (e.g., IMGN853) are administered at about 3.3 mg/kg once a week for three weeks on a four-week schedule, wherein the kilograms of body weight are adjusted to IBW, LBW, or AIBW (ADJ). In some embodiments, the FOLR1-binding agents (e.g., 1MGN853) are administered at about 3.75 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 4.2 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 4.5 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 4.8 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 5.0 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 5.5 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 5.6 mg/kg once a week for three weeks on a four-week schedule, wherein the kilograms of body weight are adjusted to IBW, LBW, or AIBW (ADJ). In some embodiments, the FOLR1 -binding agents (e.g., IMGN853) are administered at about 6.0 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 6.1 mg/kg once a week for three weeks on a four-week schedule, wherein the kilograms of body weight are adjusted to IBW, LBW,

or AIBW (ADJ). In some embodiments, the FOLR1-binding agents (e.g., IMGN853) are administered at about 6.2 mg/kg once a week for three weeks on a four-week schedule, wherein the kilograms of body weight are adjusted to IBW, LBW, or AIBW (ADJ). In some embodiments, the FOLR1-binding agents (e.g., IMGN853) arc administered at about 6.3 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 6.4 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 6.5 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 6.6 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 6.7 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 6.8 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 6.9 mg/kg once a week for three weeks on a four-week schedule, wherein the kilograms of body weight are adjusted to IBW, LBW, or AIBW (ADJ). In some embodiments, the FOLR1-binding agents (e.g., IMGN853) are administered at about 7.0 mg/kg once a week for three weeks on a four-week schedule, wherein the kilograms of body weight are adjusted to IBW, LBW, or AIBW (ADJ). In some embodiments, the kilograms of body weight are adjusted to AIBW (ADJ).

In some embodiments, the FOLR1-binding agents can be administered at a dose that results in a particular Cmax. For example, in some embodiments, the FOLR1-binding agents are administered at a dose that results in a Cmax of about 110 to about 160 μg/mL. In some embodiments, the FOLR1-binding agents are administered at a dose that results in a Cmax of about 110 to about 150 μg/mL. In some embodiments, the FOLR1-binding agents are administered at a dose that results in a Cmax of about 110 to about 140 μg/mL. In some embodiments, the FOLR1-binding agents are administered at a dose that results in a Cmax of about 120 to about 160 μg/mL. In some embodiments, the FOLR1-binding agents are administered at a dose that results in a Cmax of about 120 to about 150 μg/mL. In some embodiments, the FOLR1-binding agents are administered at a dose that results in a Cmax of about 120 to about 140 μg/mL. In some embodiments, the FOLR1-binding agents are administered at a dose that results in a Cmax of about 90 to about 160 μg/mL. In some embodiments, the FOLR1-binding agents arc administered at a dose that results in a Cmax of about 90 to about 150 μg/mL. In some embodiments, the FOLR1-binding agents arc administered at a dose that results in a Cmax of about 90 to about 140 μg/mL. In some embodiments, the FOLR1-binding agents are administered at a dose that results in a Cmax of about 100 to about 160 μg/mL. In some embodiments, the FOLR1-binding agents are administered at a dose that results in a Cmax of about 100 to about 150 μg/mL. In some embodiments, the FOLR1-binding agents are administered at a dose that results in a Cmax of about 100 to about 140 μg/mL.

In certain embodiments, the FOLR1 -binding agents can be administered at a dose that results in a particular AUC. For example, in certain embodiments, the FOLR1-binding agents are administered at a dose that results in a $AUC_{0-24}$ of no more than 2785 hr•μg/mL. In certain embodiments, the FOLR1-binding agents arc administered at a dose that results in a $AUC_{0-24}$ of no more than 2741 hr•μg/mL. In certain embodiments, the FOLR1-binding agents are administered at a dose that results in a $AUC_{0-24}$ of no more than 2700 hr•μg/mL. In some embodiments, the administration produces an $AUC_{0-24}$ of about 1000-3500 hr•μg/mL. In some embodiments, the administration produces an $AUC_{0-24}$ of about 1000-3000 hr•μg/mL. In some embodiments, the administration produces an $AUC_{0-24}$ of about 1000-2785 hr•μg/mL. In some embodiments, the administration produces an $AUC_{0-24}$ of about 1000-2741 hr•μg/mL. In some embodiments, the administration produces an $AUC_{0-24}$ of about 1000-2700 hr•μg/mL. In some embodiments, the administration produces an $AUC_{0-24}$ of about 1000-2500 hr•μg/mL. In some embodiments, the administration produces an $AUC_{0-24}$ of no more than 1500-3500 hr•μg/mL. In some embodiments, the administration produces an $AUC_{0-24}$ of no more than 1500-3000 hr•μg/mL. In some embodiments, the administration produces an $AUC_{0-24}$ of no more than 1500-2785 hr•μg/mL. In some embodiments, the administration produces an $AUC_{0-24}$ of no more than 1500-2741 hr•μg/mL. In some embodiments, the administration produces an $AUC_{0-24}$ of no more than 1500-2700 hr•μg/mL. In some embodiments, the administration produces an $AUC_{0-24}$ of about 1500-2500 hr•μg/mL.

In certain embodiments, the disease treated with the FOLR1-binding agent or antagonist (e.g., an anti-FOLR1 antibody) is a cancer. In certain embodiments, the cancer is characterized by FOLR1 expressing cells to which the FOLR1-binding agent (e.g., antibody) binds. In certain embodiments, a tumor overexpresses the human FOLR1.

The present invention provides for methods of treating cancer comprising administering a therapeutically effective amount of a FOLR1-binding agent to a subject (e.g., a subject in need of treatment). Cancers that can be treated by the methods encompassed by the invention include, but are not limited to, neoplasms, tumors, metastases, or any disease or disorder characterized by uncontrolled cell growth. The cancer can be a primary or metastatic cancer. Specific examples of cancers that can be treated by the methods encompassed by the invention include, but are not limited to ovarian cancer, lung cancer, colorectal cancer, pancreatic cancer, liver cancer, breast cancer, brain cancer, kidney

cancer, prostate cancer, gastrointestinal cancer, melanoma, cervical cancer, bladder cancer, glioblastoma, endometrial cancer, and head and neck cancer. In certain embodiments, the cancer is ovarian cancer. In certain embodiments, the cancer is endometrial cancer. In certain embodiments, the cancer is lung cancer. In certain embodiments, the lung cancer is non small cell lung cancer. In certain embodiments, the non-small cell lung cancer is adenocarcinoma of the lung.

In some embodiments, the cancer is a cancer that expresses FOLR1 (polypeptide or nucleic acid). In some embodiments, the FOLR1-binding agent is administered to a patient with an increased expression level of FOLR1, for example, as described in U.S. Published Application No. 2012/0282175 or International Published Application No. WO 2012/135675, both of which are incorporated by reference herein in their entireties. Thus, in some embodiments, the FOLR1 expression is measured by immunohistochemistry (IHC) and given a staining intensity score and/or a staining uniformity score by comparison to controls (e.g., calibrated controls) exhibiting defined scores (e.g. an intensity score of 3 is given to the test sample if the intensity is comparable to the level 3 calibrated control or an intensity of 2 is given to the test sample if the intensity is comparable to the level 2 calibrated control). A staining uniformity that is heterogeneous or homogeneous is also indicative of increased FOLR1 expression. The staining intensity and staining uniformity scores can be used alone or in combination (e.g., 2 homo, 2 hetero, 3 homo, 3 hetero, etc.). In another example, an increase in FOLR1 expression can be determined by detection of an increase of at least 2-fold, at least 3-fold, or at least 5-fold) relative to control values (e.g., expression level in a tissue or cell from a subject without cancer or with a cancer that does not have elevated FOLR1 values).

In some embodiments, the cancer is a cancer that expresses FOLR1 at a level of 1 hetero or higher by IHC. In some embodiments, the cancer is a cancer that expresses FOLR1 at a level of 2 hetero or higher by IHC. In some embodiments, the cancer is a cancer that expresses FOLR1 at a level of 3 hetero or higher by IHC. In some embodiments, the cancer is a lung cancer that expresses FOLR1 at a level of 2 hetero or higher by IHC. In some embodiments, the cancer is a lung cancer that expresses FOLR1 at a level of 3 hetero or higher by IHC. In some embodiments, the cancer is a ovarian cancer that expresses FOLR1 at a level of 2 hetero or higher by IHC. In some embodiments, the cancer is a ovarian cancer that expresses FOLR1 at a level of 3 hetero or higher by IHC. In some embodiments, the cancer is an endometriod cancer that expresses FOLR1 at a level of 1 hetero or higher by IHC. In some embodiments, the cancer is a endometriod cancer that expresses FOLR1 at a level of 2 hetero or higher by IHC.

In certain embodiments, the method of inhibiting tumor growth comprises administering to a subject a therapeutically effective amount of a FOLR1 -binding agent. In certain embodiments, the subject is a human. In certain embodiments, the subject has a tumor or has had a tumor removed.

In addition, the invention provides a method of reducing the tumorigenicity of a tumor in a subject, comprising administering a therapeutically effective amount of a FOLR1-binding agent to the subject. In certain embodiments, the tumor comprises cancer stem cells. In certain embodiments, the frequency of cancer stem cells in the tumor is reduced by administration of the agent.

The present invention further provides pharmaceutical compositions comprising one or more of the FOLR1-binding agents described herein. In certain embodiments, the pharmaceutical compositions further comprise a pharmaceutically acceptable vehicle. These pharmaceutical compositions find use in inhibiting tumor growth and treating cancer in human patients.

In certain embodiments, formulations are prepared for storage and use by combining a purified antibody or agent of the present invention with a pharmaceutically acceptable vehicle (e.g. carrier, excipient) (Remington, The Science and Practice of Pharmacy 20th Edition Mack Publishing, 2000). Suitable pharmaceutically acceptable vehicles include, but are not limited to, nontoxic buffers such as phosphate, citrate, and other organic acids; salts such as sodium chloride; antioxidants including ascorbic acid and methionine; preservatives (e.g. octadecyldimethylbcnzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3-pentanol; and m-crcsol); low molecular weight polypeptides (e.g. less than about 10 amino acid residues); proteins such as serum albumin, gelatin, or immunoglobulins; hydrophilic polymers such as polyvinylpyrrolidone; amino acids such as glycine, glutamine, asparagine, histidine, arginine, or lysine; carbohydrates such as monosaccharides, disaccharides, glucose, mannose, or dextrins; chelating agents such as EDTA; sugars such as sucrose, mannitol, trehalose or sorbitol; salt-forming counter-ions such as sodium; metal complexes (e.g. Zn-protein complexes); and non-ionic surfactants such as TWEEN or polyethylene glycol (PEG).

The pharmaceutical compositions described herein can be administered in any number of ways for either local or systemic treatment. Administration can be topical (such as to mucous membranes including vaginal and rectal delivery) such as transdermal patches, ointments, lotions, creams, gels, drops, suppositories, sprays, liquids and powders; pulmonary (e.g., by inhalation or insufflation of powders or aerosols, including by nebulizer; intratracheal, intranasal, epidermal and transdermal); oral; or parenteral including intravenous, intraarterial, subcutaneous, intraperitoneal or intramuscular injection or infusion; or intracranial (e.g., intrathecal or intraventricular) administration. In some particular embodiments, the administration is intravenous.

An antibody or immunoconjugate can be combined in a pharmaceutical combination formulation, or dosing

regimen as combination therapy, with a second compound. In some embodiments, the second compound is a steroid. In some embodiments, the methods encompass administration of a steroid and an immunoconjugate that results in a reduction of headaches as compared to administration of the immunoconjugate alone.

The steroid can be administered at the same time as the immunoconjugate, prior to the administration of the immunoconjugate, and/or after the administration of the immunoconjugate. In some embodiments, the steroid is administered within about a week, about five days, about three days, about two days, or about one day or 24 hours prior to the administration of the immunoconjugate. In some embodiments, the steroid is administered within one day of the administration of the immunoconjugate. In some embodiments, the steroid is administered multiple times. In some embodiments, the steroid is administered about one day prior to the administration of the immunoconjugate and on the same day as the administration of the immunoconjugate. The steroid can be administered via any number of ways, including for example, topical, pulmonary, oral, parenteral, or intracranial administration. In some embodiments, the administration is oral. In some embodiments, the administration is intravenous. In some embodiments, the administration is both oral and intravenous.

An antibody or immunoconjugate can also be combined in a pharmaceutical combination formulation, or dosing regimen as combination therapy, with an analgesic, or other medications that prevent or treat headaches. For example, acetaminophin and/or dephenhydramine can be administered in addition to the administration of the antibody or immunoconjugate. The analgesic can be administered prior to, at the same time, or after the administration of the immunoconjugate and can be via any appropriate administration route. In some embodiments, the analgesic is administered orally.

In some embodiments, the methods comprise administration of a first compound that is an antibody or immunoconjugate, a second compound that is a steroid, and a third compound that is an analgesic. In some embodiments, the methods comprise administration of a first compound that is 1MGN388, a second compound that is dexamethasone, and a third compound that is acetaminophin and/or diphenydramine.

An antibody or immunoconjugate can be combined in a pharmaceutical combination formulation, or dosing regimen as combination therapy, with a second compound having anti-cancer properties. The second compound of the pharmaceutical combination formulation or dosing regimen can have complementary activities to the ADC of the combination such that they do not adversely affect each other. Pharmaceutical compositions comprising the FOLR1-binding agent and the second anti-cancer agent are also provided.

Embodiments of the present disclosure can be further defined by reference to the following non-limiting examples, which describe in detail preparation of certain antibodies of the present disclosure and methods for using antibodies of the present disclosure. It will be apparent to those skilled in the art that many modifications, both to materials and methods, can be practiced without departing from the scope of the present disclosure.

## Examples

It is understood that the examples and embodiments described herein are for illustrative purposes only and that various modifications or changes in light thereof will be suggested to persons skilled in the art and are to be included within the spirit and purview of this application

### Example 1

### IMGN853 Dosing Trial in Human Cancer Patients

IMGN853 is an antibody-drug conjugate (ADC) comprising a folate receptor 1 (FOLR1)-binding antibody and the potent maytansinoid, DM4. IMGN853 has been previously described in International Published Application Nos. WO 2011/106528, WO 2012/135675, and WO 2012/138749, and U.S. Published Application Nos. 2012/0009181, 2012/0282175, and 2012/0282282, each of which is incorporated by reference herein in its entirety. IMGN853 is huMov19-sSPDB-DM4, and the huMov19 antibody contains a variable heavy chain with the amino acid sequence of SEQ ID NO:3 and a variable light chain with the amino acid sequence of SEQ ID NO: 5. FOLR1 protein is expressed at elevated levels on many solid tumors, particularly epithelial ovarian cancer (EOC), endometrial cancer, non-small cell lung cancer (NSCLC), and clear-cell renal cell cancer.

A study to determine the maximum tolerated dose (MTD) and recommended phase 2 dose (RP2D) as well as to evaluate the safety, pharmacokinetics (PK), pharmacodynamics (PD), and efficacy of IMGN853 was initiated. The study includes two components: an accelerated dose titration component, where the IMGN853 immunoconjugate was administered to patients with any type of FOLR1-expressing refractory solid tumors including epithelial ovarian cancer (EOC) and other FOLR1-positive solid tumors, and a dose expansion component.

For the accelerated titration portion of the study, IMGN853 was given intravenously (IV) on Day 1 of each 21-day (3 week) cycle. Twenty-nine patients have been enrolled across seven dose levels ranging from 0.15 to 7.0 mg/kg IMGN853 in the accelerated portion of the clinical trial and there are safety data currently available for 23 patients. There

were no study drug-related AEs of any grade reported in patients treated in the first 4 dose cohorts. At doses up to 5.0 mg/kg, IMGN853 related AEs have been mild to moderate. At the 5.0 and 7.0 mg/kg dose levels, 4 of 10 and 5 of 5 patients, respectively, have reported ocular toxicity.

Table 1: Enrollment by Tumor Type

| **Enrollment and FOLR1 Expression by Tumor Type N=29** | | | | | | |
|---|---|---|---|---|---|---|
| **Diagnosis** | **FOLR1 Expression Level** | | | | | |
| | 2 Hetero | 2 Homo | 3 Hetero | 3 Homo | Other | Totals |
| Ovarian Cancer | **2** | **2** | **7** | **4** | **1** | **16** |
| Serous | 1[1] | | 6[1] | 3 | | 10 |
| Transitional Cell | | | 1[2] | | | 1 |
| Clear Cell | 1 | 1 | | 1 | | 3 |
| Carcinosarcoma | | 1 | | | 1[4] | 1 |
| Endometrial | **2** | **0** | **5** | **1** | **0** | **8** |
| Serous | | | 3[3] | 1 | | 4 |
| Endometrioid | 2 | | | | | 2 |
| Adenosquamous | | | 1 | | | 1 |
| Mixed | | | 1 | | | **1** |
| NSCLC Adenocarcinoma | **1** | | | | | **1** |
| Renal Cell | **0** | **2** | | | **2[5]** | **4** |
| Clear Cell | | 2 | | | **2[5]** | 4 |
| [1]CA125 response in 1 patient each at 3.3 mg/kg, 5.0 mg/kg, and 7.0 mg/kg<br>[2]Confirmed partial response<br>[3]Unconfirmed partial response and confirmed CA125 response in 1 patient at 5.0 mg/kg<br>[4]Focal<br>[5]Negative | | | | | | |

Drug exposure was measured in 23 patients and found to generally increase linearly, with a half-life at doses ≥ 2.0 mg/kg of approximately 5 days. One patient with serous endometrial cancer also had a CA125 response and an unconfirmed partial response at 5 mg/kg. Three patients with ovarian cancer have reported confirmed CA125 response (one each at 7 mg/kg, 5 mg/kg and one at 3.3 mg/kg). Patients receiving IMGN853 at doses greater than or equal to 5.0 mg/kg received dexamethasone, 10 mg IV (or similar steroid equivalent), 30 to 60 minutes prior to anti-FOLR1 immunoconjugate (e.g., IMGN853) administration.

The pharmacokinetic (PK) parameters are reported for Cycle 1 (first cycle of dosing for each patient only) of the IMGN853 Phase 1 trial. (Figures 1A and B.) The clearance of IMGN853 is shown to be rapid at low doses (CL= 1.1 mL/hr/kg) with a half life of approximately 35.4 hours or 1.5 days. The clearance decreases (CL= 0.4 mL/hcr/kg) at the higher doses, and the half-life increases to about 4 days or about 5 days at doses ≥ 2.0 mg/kg. The exposure (AUC) and the Cmax are shown to generally increase at the higher doses as well.

At the 7.0 mg/kg dose, all 5 patients experienced ocular toxicity. One patient was reported with Grade 3, dose-limiting punctate keratitis and Grade 2 blurred vision that were deemed definitely related to study treatment. Additionally, there was one patient each with Grade 3, Grade 2, and Grade 1 blurred vision; all events were deemed possibly or definitely related to IMGN853 treatment. As a result, the maximum tolerated dose on this schedule of administration (i.e., once every three weeks) was deemed to have been exceeded at the 7.0 mg/kg dose level, and all patients remaining at the 7.0 mg/kg dose level were dose reduced to the previous dose level (5.0 mg/kg) and 7 additional patients were evaluated at the 5 mg/kg dose. Together with the 3 patients treated previously, 10 patients were treated at a dose of 5 mg/kg. Of the total of 10 patients at the 5 mg/kg level, 3 had blurred vision, including 1 patient with grade 3 blurred vision, and 2 patients had corneal changes. Other related grade 3 adverse events included elevated alkaline phosphatase and grade 3 hypophosphatemia. Additional patients were enrolled to the 3.3 mg/kg dose level to further explore and confirm the safety profile seen with the 3 patients originally assigned to this dose. Safety review of the additional 6 patients currently being treated at the 3.3 mg/kg is ongoing and IMGN853 is well tolerated. To date, three of the nine patients

treated at the 3.3 mg/kg dose level have reported IMGN853-related AEs, including grade 2 peripheral neuropathy (1 patient), grade 2 nausea, fatigue and AST elevation (1 patient), and 1 patient with grade 2 vomiting.

Once the MTD is defined, the study will proceed to the dose expansion phase. Three expansion cohorts will evaluate patients with FOLR1 protein positive (1) platinum resistant epithelial ovarian cancer; (2) relapsed or refractory epithelial ovarian cancer, and (3) relapsed or refractory non small cell lung cancer (NSCLC). Cohorts 2 and 3 will have IMGN853 PD assessment by pre-and post-dose tumor biopsy and/or by FLT-PET imaging, respectively. IMGN853 will be administered at a dose of at least 3.3 mg/kg and may include doses of 5.0 mg/kg or as high as 6.0 mg/kg or even 7.0 mg/kg. Initially IMGN853 should be administered at a rate of 1 mg/min; after 30 minutes, the rate can be increased to 3 mg/min if well tolerated. If well tolerated after 30 minutes at 3 mg/min, the rate may be increased to 5 mg/min. Subsequent infusions can be delivered at the tolerated rate.

For all IMGN853 dosing at 3.3 mg/kg or higher, prophylactic steroid treatment will be included using the protocols described in Example 2 (e.g., steroid treatment is included at 10 mg dexamethasone IV (or similar steroid equivalent) 30 to 60 minutes prior to IMGN853 administration is required and prophylactic diphenhydramine HCl and acetaminophen is recommended prior to IMGN853 administration). Cycles are repeated until (i) the patient's disease worsens, (ii) the patient experiences unacceptable toxicity, (iii) the patient withdraws consent, (iv) the patient develops a comorbid condition that would preclude further study treatment or (v) the patient is discontinues due to non-compliance or administrative reasons.

Responses arc assessed using RECIST and Gynecologic Cancer Intergroup (GCIG) criteria (as appropriate).

Example 2

IMGN853 Steroid-Based Prophylaxis for Infusion Reaction

In order to decrease the likelihood of infusion reaction, any of the following steroid-based prophylaxis protocols can be used.

(1) Patients receive dexamethasone, 10 mg IV (or similar steroid equivalent), 30 to 60 minutes prior to anti-FOLR1 immunoconjugate (e.g., IMGN853) administration.
(2) Patients receive dexamethasone, 10 mg IV (or similar steroid equivalent) and diphenhydramine HCl (25-50 mg IV or PO), with or without acetaminophen (325-650 mg IV or PO), 30 to 60 minutes prior to anti-FOLR1 immunoconjugate (e.g., IMGN853) administration. This prophylactic protocol is recommended and at the discretion of each investigator.
(3) Patients receive dexamethasone 8 mg (or similar steroid equivalent) by mouth BID on the day prior to administration of anti-FOLR1 immunoconjugate (e.g., IMGN853). On the day of administration of anti-FOLR1 immunoconjugate (e.g., IMGN853), 30-60 mins prior to anti-FOLR1 immunoconjugate (e.g., IMGN853) administration, patients receive dexamethasone, 10 mg IV (or similar steroid equivalent), diphenhydramine HCl (25-50 mg IV or PO), with or without acetaminophen (325-650 mg IV or PO)
(4) Within 24 hours prior to infusion steroids (e.g., dexamethasone) are administered orally.

Example 3

Relationship of IMGN853 Exposure with Ocular Toxicity

For each patient treated with the IMGN853 protocol described in Examples 1 and 2, the plasma concentration of IMGN853 was measured at various time points across each cycle, beginning at end of infusion and continuing to day 21. Pharmacokinetic (PK) parameter analysis identified an apparent association between Cmax and the occurrence of ocular toxicity, which is characterized by corneal deposits and loss of visual acuity. The statistically significant correlation was also observed with early exposure levels as measured by area under the curve in the first 24 hrs ($AUC_{0-24}$ ). (See Figures 2A-2C.)

In the 3.3 to 7.0 mg/kg cohorts, ocular toxicity was observed in 9/10 patients with Cmax values at or above 147.7 μg/ml, indicated by the dotted line in Figure 2A. No patients with Cmax values below 147.7 μg/ml developed ocular toxicity. All (9/9) patients with an $AUC_{0-24}$ at or above 2785 hr*μg/ml, indicated by the dotted line in Figure 2B, developed ocular toxicity, whereas none below had any eye toxicity. Efficacy signals were observed at doses ≥ 3.3 mg/kg and did not correlate with reports of ocular toxicity. The lowest Cmax value in patients that have had signals of activity was 91.25 μg/ml.

After 24 patients were treated with either 3.3 mg/kg, 5.0 mg/kg, or 7.0 mg/kg 1MGN853, a Cmax value above threshold correlated with (reversible) ocular toxicity using Fisher's exact test, p = 0.00004. An $AUC_{0-24}$ value above the threshold level of 2,741 hr*μg/ml also correlated with (reversible) ocular toxicity using Fisher's exact test, p = 0.00001.

Based on these results and calculations using nominal time and concentration values, it was determined that patients having a Cmax greater than about 150 μg/ml or an $AUC_{0-24}$ value greater than 2785 hr*μg/ml were most likely to have an increased rate of ocular toxicity, and patients having signals of activity had a Cmax level of at least about 90 μg/mg. Strategies were developed to modify the dosing in order to reduce the variability seen within each dose level and to achieve a Cmax level for optimal efficacy and minimal toxicity at each patient weight.

Example 4

IMGN853 Alternate Dosing Approaches

As described in Example 3, above, a correlation between Cmax values over 150 μg/ml or $AUC_{0-24}$ values over 2785 hr*μg/ml and incidence of ocular toxicity was observed across all dose levels. In addition, initial PK parameter analysis demonstrated that although Cmax increased proportionally with the dose of IMGN853, there was a significant variation in Cmax, $AUC_{0-24}$ and volume of distribution within dose levels (Figure 3).

The variation in Cmax was particularly striking in the 5 mg/kg cohort, where PK was analyzed in 10 patients. There was no apparent intra-patient Cmax variability across cycles, and infusion times were similar between patients and were not associated with Cmax variation. Covariate analysis demonstrated a correlation between weight and Cmax. (Figure 4).

Volume of distribution ($V_{ss}$) is indicative of plasma volume for biologics and does not increase linearly with weight. When Cmax values were normalized by Vss, the variation was reduced. These data suggest that exploring alternate dosing approaches other than based on total body weight could produce more uniform dosing across cohorts. To this end, Cmax values were estimated using alternative dosing calculations for all patients treated in the 3.3 (n=3), 5.0 (n=10), and 7 mg/kg (n=5) dose groups. Calculated Cmax values were normalized to a 5 mg/kg dose level and compared to Cmax values observed from total body weight (TBW) dosing. Body surface area (BSA) was also considered; however, Cmax values based on BSA dosing decreased the Cmax, but the variability was only minimally impacted and a positive correlation between weight and Cmax was still observed though to a lesser extent. Three additional alternate formulas were evaluated: (1) Ideal Body Weight (IBW), Lean Body Weight (LBW), and Adjusted Body Weight (ADJ). The formula for each of IBW, LBW, ADJ, and BSA is provided below:

*Ideal Body Weight (IBW)*

$$\text{IBW (male)} = 0.9\text{H-}88$$

$$\text{IBW (fem)} = 0.9\text{H-}92$$

(where H=height in cm)

*Lean Body Weight (LBW)*

$$\text{Men} = 1.10 \text{ x weight in kg} - 128([\text{weight in kg}]^2/[100 \text{ x height in meters}]^2)$$

$$\text{Women} = 1.07 \text{ x weight in kg} - 148([\text{weight in kg}]^2/[100 \text{ x height in meters}]^2)$$

*Adjusted Ideal Body Weight (AIBW or ADJ)*

$$\text{IBW} + 0.4(\text{Actual weight in kg} - \text{IBW})$$

*Body Surface Area (BSA) - Mosteller Formula*

$$\text{BSA (m}^2) = (\text{Height(cm) x Weight(kg)} / 3600)^{1/2}$$

*Body Surface Area (BSA) - Boyd Formula*

$$BSA\ (m^2) = (0.0003207 \times Height(cm)^{0.3} \times Weight(grams)^{(0.7285 - (0.0188 \times LOG(grams))})$$

Average Cmax values were 93.06, 82.72, 110.77 and 137.46 μg/ml for IBW, LBW, AIBW (ADJ) and TBW, respectively. Additionally, all three alternate metrics reduced the standard deviation in Cmax (21.7, 20.5, 22.9 vs. 33.7 μg/ml for TBW). (See Figure 5.)

As mentioned above, a positive correlation was observed for TBW versus Cmax. Correlation analysis of the IBW and LBW versus weight plots demonstrated a negative correlation versus weight. Dosing by AIBW (ADJ) body weight had the least body weight dependence (see Figure 6), similar to BSA but with less PK variability.

Dosing by IBW, LBW, or AIBW (ADJ) results in less weight dependence compared to TBW dosing. Based on the current data, dosing by AIBW (ADJ) weight results in the least variance in Cmax.

$AUC_{0-24}$ values were observed in 24 patients who received 3.3, 5, or 7 mg/kg of 1MGN853 based on total body weight (see Figure 7 "TBW Actual"). In addition, $AUC_{0-24}$ values were also observed in 7 patients who received 5 mg/kg of TMGN853 based on adjusted ideal body weight (see Figure 7 "5 ADJ Actual"). These actual values obtained with the 24 patients were compared with the projected values that would have been obtained with those same patients if they had all been treated with 5 mg/kg based on total body weight (Figure 7 "TBW 5 mg/kg") or if they had all been treated with 5, 5.4, or 6 mg/kg based on adjusted ideal body weight (Figure 7 "ADJ 5 mg/kg," "ADJ 5.4 mg/kg," and "ADJ 6 mg/kg.") As shown in the table in Figure 7, administering 5 mg/kg IMGN853 based on AIBW (ADJ) minimizes the number of patients who are projected to exceed the threshold level of $AUC_{0-24}$ 2741 hr*μg/ml associated with ocular toxicity. In addition, only 14% of the 7 patients who received 5 mg/kg IMGN853 based on AIBW (ADJ) reached $AUC_{0-24}$ levels above 2741 hr*μg/ml, whereas 38% of the patients who received 3.3, 5, or 7 mg/kg of IMGN853 based on TBW exceeded this level. The original analysis of $AUC_{0-24}$ was calculated using nominal time and concentration values and resulted in a value of 2785 hr* μg/ml. When recalculated using actual time, the result was a slight modification to the $AUC_{0-24}$ values and a determination of a threshold value of 2741 hr* μg/ml.

Patients were then treated with either 5 mg/kg based on adjusted ideal body weight, 6 mg/kg based on adjusted ideal body weight, 5 mg/kg based on total body weight, or 7 mg/kg based on total body weight. The observed $AUC_{0-24}$ values in these patients are shown in Figure 8 ("Acutal"), and these values arc compared with the projected values ("Proj") that would have been obtained if all of these patients had been treated with 5 mg/kg based on adjusted ideal body weight, 6 mg/kg based on adjusted ideal body weight, 5 mg/kg based on total body weight, or 7 mg/kg based on total body weight. Dosing based on adjusted ideal body weight decreased the variability in early exposure levels and decreased ocular adverse events as shown in Table 2 below. In particular, only one patient receiving 5 mg/kg based on adjusted ideal body weight experienced Grade 1 visual disturbance and three patients receiving 6 mg/kg based on adjusted ideal body weight experienced Grade 1-2 ocular toxicity. In contrast, doses of 5 mg/kg or more based on total body weight were associated with more patients having ocular adverse events and with the occurance of Grade 3 ocular adverse events.

Table 2: Ocular Adverse Events (OAE) Reported with Dosing based on Adjusted Idead Body Weight (All Reversible)

| IMGN853 (mg/kg adusted ideal body weight) | Cohort Size | Patients with Any Grade OAE | OAEs | | DLTs |
|---|---|---|---|---|---|
| | | | Grade 1-2 | Grade 3[1] | |
| 5.0 | 7 | 1 | 1[2] | 0 | 0 |
| 6.0 | 7 | 3 | 5[3] | 0 | 0 |

[1]There were no Grade 4 adverse events.
[2]One patient reported visual disturbance.
[3]Grade 2 blurred vision and punctate keratitis in 1 patient; Grade 2 retinopathy in one patient; and Grade 1 blurred vision and floaters in 1 patient.

Example 5

IMGN853 Alternate Schedules

A population PK model based on rich and sparse (peak and through) concentration-time profiles of IMGN853 following Q3W dosing (escalating dose) was developed. The PK of IMGN853 as stated above appears to be linear over the dose range studied.

This model was used to simulate steady-state exposure to IMGN853 for the following candidate regimens:

1 to 2.5 mg/kg QW (10 dose levels at 0.15 mg/kg increments)
2 to 5 mg/kg Q2W (10 dose levels at 0.3 mg/kg increments)
1 to 2.5 mg/kg QWx4 then Q2Wx4 (10 dose levels at 0.15 mg/kg increments)
1 to 2.5 mg/kg QWx3 in 4W (10 dose levels at 0.15 mg/kg increments)

The QWx4 then Q2Wx4 dosing regimen resulted in the least accumulation (i.e., accumulation index of 1) over time, while the QW dosing regimen resulted in the highest accumulation (i.e., accumulation index of 1.97). QWx3 in 4W allowed overall exposure increases up to -3-fold while limiting Cmax to levels below that observed with ocular toxicity (Table 3).

Table 3

| Schedule | Dose (mg/kg) | Cmax (μg/ml) | Cmin (μg/ml) | Cavg (μg/ml) | $AUC_{12w}$ (h*mg/ml) |
|---|---|---|---|---|---|
| Q3W | 4.2 | 112 | 7.8 | 30 | 32 |
| Q2W | 4.1 | 119 | 18 | 44 | 84 |
| QWx3 in 4W | 3.3 | 119 | 20 | 52 | 105 |

The population PK model was also used to simulate a hypothetical population of 500 patients via Monte Carlo resampling. Descriptive statistics were derived in order to determine a dosing regimen with the percentage of subjects with $C_{max}$ < 150 μg/ml to optimize the safety profile of IMGN853. For the QWx3 in 4W regimen, the 1.5, 2.0 and 2.5 mg/kg dose levels resulted in 99%, 95% and 90% of the population with a $C_{max}$ < 150 μg/ml, respectively.

Example 6

*In Vivo* Antitumor Activity and Predicted Pharmacokinetics of Multi-dose IMGN853

The plasma concentrations of intact IMGN853 conjugate administered intravenously at a dose of 10 mg/kg in female CD-1 mice was determined by ELISA at various time points post injection. Pharmacokinetic (PK) analyses were performed using the standard algorithms of the non-compartmental pharmacokinetic analysis program (201), WinNonlin, Professional version 6.1 (Pharsight, Mountain View, CA). The maximal concentration (Cmax), the total area under the concentration-time curve ($AUC_{0-\infty}$), the half-life ($t_{1/2}$) in terminal elimination phase, the total blood clearance (CL) and the volume of distribution at steady-state (Vss) were estimated. The values of the first order rate constant for determining conjugate $t_{1/2}$ were evaluated by using the concentration data from 1 to 28 days post-administration. The values of the first order rate constant for determining antibody $t_{1/2}$ were evaluated by using the concentration data from 1 to 28 days post-administration. Based on the measured values generated at the 10 mg/kg dose, PK simulations were performed with WinNonlin using various dose levels, with both single and multi-dose schedules. The resulting parameters were evaluated in comparison to the anti-tumor activity of IMGN853 at various dose levels and schedules in NCI-H2110 (non-small cell lung cancer, NSCLC) xenografts in female SCID mice.

When IMGN853 was administered as a single injection, dose-dependent antitumor activity was observed in the NCI-H2110 model. All dose levels (2.8, 5.6, and 8.5 mg/kg) were highly active with T/C values <10%, yet there were an increase in numbers of complete tumor regressions (CR) with increased IMGN853 dose. The predicted plasma PK parameters also showed a dose-dependent increase in Cmax (maximal plasma concentration), Cavg (average plasma concentration), and exposure ($AUC_{0-540}$ hrs). Thus, the activity of single doses of IMGN853 was shown to be dose-dependent and predictable based on plasma PK parameters, as shown in Figure 9.

In contrast to the single-dose activity, multi-dose schedules of IMGN853 show that activity does not depend on Cmax (Figure 10). IMGN853 administered at a dose of 2.8 mg/kg x 3, either daily or every 3 days schedule (total dose of 8.4 mg/kg) had similar activity to single-dose IMGN853 at 8.5 mg/kg. Interestingly, total exposure (AUC) and average plasma concentration (Cavg) of conjugate was comparable among the treatment groups, while the Cmax was highest in the 8.5 mg/kg single-dose group. The activity observed with multi-dose schedules suggests that this method of dosing has greater activity, as there were tumor-free animals remaining at the end of the study, versus no tumor-free animals in the single high dose group.

Additional IMGN853 dose levels and schedules were evaluated for activity against NCI-112110 xenografts, with results consistently demonstrating that multi-dose administration is equally active or has better activity than single-dose IMGN853. The predicted average plasma concentration of IMGN853 administered as a single-dose of 5.6 mg/kg IMGN853 was comparable to that of a 1.4 mg/kg daily for 3 days. Despite having a lower total dose (4.2 mg/kg), Cmax and exposure ($AUC_{0-540}$) the 1.4 mg/kg qd x3 had comparable antitumor activity in vivo (Figure 11). These results

suggest that maintenance of a certain minimal plasma concentration is critical for activity.

A weekly schedule of IMGN853 with matched total dose to a single high dose of IMGN853 were also found to have comparable activity in vivo (Figure 12). Again, maintenance of an average plasma concentration above a minimum threshold was required for activity, with the single-dose IMGN853 (8.5 mg/kg) resulting in a slightly higher Cavg and AUC but with comparable overall activity. The key difference in predicted pharmacokinetic parameters with single versus multi-dose schedule is the dramatic reduction in Cmax. The weekly dosing Cmax is predicted to be almost 60% lower than the Cmax of single-dose IMGN853. As there is no apparent activity benefit of reaching a higher Cmax, avoiding high plasma concentrations can be beneficial in reducing toxicity.

It is to be appreciated that the Detailed Description section, and not the Summary and Abstract sections, is intended to be used to interpret the claims. The Summary and Abstract sections sets forth one or more, but not all, exemplary embodiments of the present invention as contemplated by the inventor(s), and thus, are not intended to limit the present invention and the appended claims in any way.

The present invention has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof arc appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described exemplary embodiments, but should be defined only in accordance with the following claims and their equivalents.

SEQUENCES

SEQ ID NO:1 - human folate receptor 1

MAQRMTTQLLLLLVWVAVVGEAQTRIAWARTELLNVCMNAKHHKEKPGPEDKLHEQ
CRPWRKNACCSTNTSQEAHKDVSYLYRFNWNHCGEMAPACKRHFIQDTCLYECSPNLG
PWIQQVDQSWRKERVLNVPLCKEDCEQWWEDCRTSYTCKSNWHKGWNWTSGFNKCA
VGAACQPFHFYFPTPTVLCNEIWTHSYKVSNYSRGSGRCIQMWFDPAQGNPNEEVARFY
AAAMSGAGPWAAWPFLLSLALMLLWLLS

SEQ ID NO:2 - human folate receptor 1 nucleic acid sequence

atggctcagcggatgacaacacagctgctgctccttctagtgtgggtggctgtagtaggggaggctcagacaaggattgcatgggccagga
ctgagcttctcaatgtctgcatgaacgccaagcaccacaaggaaaagccaggccccgaggacaagttgcatgagcagtgtcgaccctgga
ggaagaatgcctgctgttctaccaacaccagccaggaagcccataaggatgtttcctacctatatagattcaactggaaccactgtggagag
atggcacctgcctgcaaacggcatttcatccaggacacctgcctctacgagtgctcccccaacttggggccctggatccagcaggtggatc
agagctggcgcaaagagcgggtactgaacgtgcccctgtgcaaagaggactgtgagcaatggtgggaagattgtcgcacctcctacacct
gcaagagcaactggcacaagggctggaactggacttcagggtttaacaagtgcgcagtgggagctgcctgccaacctttccatttctacttc
cccacacccactgttctgtgcaatgaaatctggactcactcctacaaggtcagcaactacagccgagggagtggccgctgcatccagatgtg
gttcgacccagcccagggcaaccccaatgaggaggtggcgaggttctatgctgcagccatgagtggggctgggccctgggcagcctggc
ctttcctgcttagcctggccctaatgctgctgtggctgctcagc

SEQ ID NO:3 - huMov19 vHC

QVQLVQSGAEVVKPGASVKISCKASGYTFTGYFMNWVKQSPGQSLEWIGRIHPYDGDT
FYNQKFQGKATLTVDKSSNTAHMELLSLTSEDFAVYYCTRYDGSRAMDYWGQGTTVT
VSS

SEQ ID NO:4 - huMov19 vLCvl.00

DIVLTQSPLSLAVSLGQPAIISCKASQSVSFAGTSLMHWYHQKPGQQPRLLIYRASNLEA
GVPDRFSGSGSKTDFTLNISPVEAEDAATYYCQQSREYPYTFGGGTKLEIKR

SEQ ID NO:5 - huMov19 vLCv1.60

DIVLTQSPLSLAVSLGQPAIISCKASQSVSFAGTSLMHWYHQKPGQQPRLLIYRASNLEA
GVPDRFSGSGSKTDFTLTISPVEAEDAATYYCQQSREYPYTFGGGTKLEIKR

SEQ ID NO:6 - huMovl9 vLC CDR1 KASQSVSFAGTSLMH

SEQ ID NO:7 - huMovl9 vLC CDR2 RASNLEA

SEQ ID NO:8 - huMov19 vLC CDR3 QQSREYPYT

SEQ ID NO:9 - huMovl9 vHC CDR1 GYFMN

SEQ ID NO:10 - huMov19 vHC CDR2 - Kabat Defined RIHPYDGDTFYNQKFQG

SEQ ID NO:11 - huMov19 vHC CDR2 - Abm Defined RIHPYDGDTF

SEQ ID NO:12 - huMov19 vHC CDR3 YDGSRAMDY

SEQ ID NO:13 - huMov19 HC amino acid sequence

QVQLVQSGAEVVKPGASVKISCKASGYTFTGYFMNWVKQSPGQSLEWIGRIHPYDGDT
FYNQKFQGKATLTVDKSSNTAHMELLSLTSEDFAVYYCTRYDGSRAMDYWGQGTTVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK

SEQ ID NO:14 - huMov19 LCv1.00

DIVLTQSPLSLAVSLGQPAIISCKASQSVSFAGTSLMHWYHQKPGQQPRLLIYRASNLEA
GVPDRFSGSGSKTDFTLNISPVEAEDAATYYCQQSREYPYTFGGGTKLEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC

SEQ ID NO:15 - huMov 19 LCv 1.60

DIVLTQSPLSLAVSLGQPAIISCKASQSVSFAGTSLMHWYHQKPGQQPRLLIYRASNLEA
GVPDRFSGSGSKTDFTLTISPVEAEDAATYYCQQSREYPYTFGGGTKLEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC
SEQ ID NO:16 – muMov19 vHC CDR2 – Kabat Defined
RIHPYDGDTFYNQNFKD

The following pages 60 to 68 contain specific embodiements

1. A method for treating a human patient having an FOLR1-expressing cancer comprising administering to the patient an immunoconjugate which binds to FOLR1 polypeptide, wherein the immunoconjugate is administered at a dose of about 3.0 to about 7 milligrams (mg) per kilogram (kg) of body weight of the patient, wherein the kilograms of body weight of the patient are adjusted to adjusted ideal body weight (AIBW).

2. The method of 1, wherein the administration produces a Cmax of about 90-160 μg/mL.

3. The method of 2, wherein the administration produces a Cmax of about 90-150 μg/mL.

4. The method of any one of 1-3, wherein the administration produces an area-under-the-curve $(AUC)_{0-24}$ of no more than 2700 hr•μg/mL.

5. The method of any one of 1-4, wherein the immunoconjugate is administered at a dose of about 5.0 mg/kg.

6. The method of any one of 1-4, wherein the immunoconjugate is administered at a dose of about 6.0 mg/kg.

7. The method of any one of 1-4, wherein the immunoconjugate is administered at a dose of about 6.5 mg/kg.

8. The method of any one of 1-7, wherein the immunoconjugate is administered once every three weeks.

9. The method of any one of 1-4, wherein the immunoconjugate is administered once every week.

10. A method for treating a human patient having an FOLR1-expressing cancer comprising administering to the patient an immunoconjugate which binds to FOLR1 polypeptide, wherein the immunoconjugate is administered at a dose of about 3.0 to about 7 milligrams (mg) per kilogram (kg) of body weight of the patient, and wherein the administration produces a Cmax of about 110-160 μg/mL.

11. The method of 10, wherein the administration produces a Cmax of about 110-150 μg/mL.

12. A method for treating a human patient having an FOLR1-expressing cancer comprising administering to the patient an immunoconjugate which binds to FOLR1 polypeptide, wherein the immunoconjugate is administered at a dose of about 3.0 to about 7 milligrams (mg) per kilogram (/kg) of body weight of the patient, and wherein the administration produces an $AUC_{0-24}$ of no more than 2700 hr•μg/mL.

13. The method of any one of 1-12, wherein immunoconjugate comprises an antibody or antigen-binding fragment thereof that comprises the CDRs of SEQ ID NOs: 6-9, 11, and 12.

14. The method of 13, wherein the antibody is huMov19.

15. The method of any one of 1-14, wherein the immunoconjugate comprises a maytansinoid.

16. The method of 15, wherein the maytansinoid is DM4.

17. The method of any one of 1-16, wherein the immunoconjugate comprises the linker sulfo-SPDB.

18. The method of any one of 1-17, wherein the immunoconjugate is IMGN853.

19. The method of any one of 1-18, wherein the immunoconjugate is administered intravenously.

20. The method of any one of 1-19, wherein said cancer is selected from the group consisting of ovarian, brain, breast, uterine, endometrial, pancreatic, renal, and lung cancer.

21. The method of 20, wherein the lung cancer is non small cell lung cancer.

22. The method of 21, wherein the non small cell lung cancer is adenocarcinoma.

23. The method of 20, wherein the ovarian cancer is epithelial ovarian cancer.

24. The method of 23, wherein the ovarian cancer is platinum resistant, relapsed, or refractory.

25. The method of 20, wherein the cancer is endometrial cancer.

26. The method of any one of 1-25, wherein a sample obtained from the patient exhibits FOLR1 expression as measured by immunohistochemistry (1HC).

27. The method of 26, wherein the sample has a staining intensity of at least 2 hetero.

28. The method of 26, wherein the sample has a staining intensity of at least 2 homo.

29. The method of 26, wherein the sample has a staining intensity of at least 3 hetero.

30. The method of 26, wherein the sample has a staining intensity of at least 3 homo.

31. The method of any one of ; 1-30, further comprising administering a steroid to the patient.

32. The method of 31, wherein the steroid is dexamethasone.

33. The method of any one of 1-32, wherein the administration results in a decrease in tumor size.

34. The method of any one of 1-20, 23, 24, and 26-33, wherein the cancer is ovarian cancer and wherein the administration results in a decrease in CA125.

35. The method of any one of 1-34, wherein the administration results in a decrease in toxicity.

36. The method of 35, wherein the toxicity is ocular toxicity.

37. The method of any one of 1-36, further comprising administering a second compound having anti-cancer properties.

38. The method of any one of 1-3, 5-11, and 13-37 wherein the administration produced an $AUC_{0-24}$ of no more than 2785 hr•μg/mL.

39. The method of any one of 1-3, 5-11, and 13-37, wherein the administration produced an $AUC_{0-24}$ of no more than 2741 hr•μg/mL.

40. A method for treating a human patient having an FOLR1-expressing cancer comprising administering to the patient an effective dose of an immunoconjugate which binds to FOLR1 polypeptide, wherein the administration produces an $AUC_{0-24}$ of no more than 2741 hr•μg/mL.

41. The method of 40, wherein the administration produces a Cmax of no more than 160 μg/mL.

42. The method of 41, wherein the administration produces a Cmax of no more than 150 μg/mL.

43. The method of any one of 40-42, wherein immunoconjugate comprises an antibody or antigen-binding fragment thereof that comprises the CDRs of SEQ ID NOs: 6-9, 1 1, and 12.

44. The method of 43, wherein the antibody is huMov19.

45. The method of any one of 40-44, wherein the immunoconjugate comprises a maytansinoid.

46. The method of 45, wherein the maytansinoid is DM4.

47. The method of any one of 40-46, wherein the immunoconjugate comprises the linker sulfo-SPDB.

48. The method of any one of 40-47, wherein the immunoconjugate is IMGN853.

49. The method of any one of 40-48, wherein the immunoconjugate is administered intravenously.

50. The method of any one of 40-49, wherein said cancer is selected from the group consisting of ovarian, brain, breast, uterine, endometrial, pancreatic, renal, and lung cancer.

51. The method of 50, wherein the lung cancer is non small cell lung cancer.

52. The method of 51, wherein the non small cell lung cancer is adenocarcinoma.

53. The method of 50, wherein the ovarian cancer is epithelial ovarian cancer.

54. The method of 53, wherein the ovarian cancer is platinum resistant, relapsed, or refractory.

55. The method of 50, wherein the cancer is endometrial cancer.

56. The method of any one of 40-55, wherein a sample obtained from the patient exhibits FOLR1 expression as measured by immunohistochemistry (THC).

57. The method of 56, wherein the sample has a staining intensity of at least 2 hetero.

58. The method of 56, wherein the sample has a staining intensity of at least 2 homo.

59. The method of 56, wherein the sample has a staining intensity of at least 3 hetero.

60. The method of 56, wherein the sample has a staining intensity of at least 3 homo.

61. The method of any one of 40-60, further comprising administering a steroid to the patient.

62. The method of 61, wherein the steroid is dexamethasone.

63. The method of any one of 40-62, wherein the administration results in a decrease in tumor size.

64. The method of any one of 40-50, 53, 54, and 56-63, wherein the cancer is ovarian cancer and wherein the administration results in a decrease in CA125.

65. The method of any one of 40-64, wherein the administration results in a decrease in toxicity.

66. The method of 65, wherein the toxicity is ocular toxicity.

67. The method of any one of 40-66, further comprising administering a second compound having anti-cancer properties.

68. A method for treating a human patient having a FOLR1-expressing cancer comprising administering to the patient an immunoconjugate which binds to FOLR1 polypeptide, wherein the immunoconjugate is administered once a week for three weeks on a four-week schedule.

69. The method of 68, wherein the immunoconjugate is administered on days 1, 8, and 15 of a four-week schedule.

70. The method of 68 or 69, wherein the administration produces a Cmax of about 90-160 μg/mL.

71. The method of 70, wherein the administration produces a Cmax of about 90-150 μg/mL.

72. The method of 68 or 69, wherein the administration produces a Cmax of about 110-160 μg/mL.

73. The method of 72, wherein the administration produces a Cmax of about 110-150 μg/mL.

74. The method of any one of 68-73, wherein the administration produces an $AUC_{0-24}$ of no more than 2700 hr•μg/mL.

75. The method of any one of 68-74, wherein the immunoconjugate is administered at a dose of about 1.5 to about 6 mg/kg.

76. The method of 75, wherein the immunoconjugate is administered at a dose of about 1.5, 2.0, 2.5, 3, 3.3, 4.0, 4.1, 4.2, 5.0, 5.5, or 6.0 mg/kg.

77. The method of any one of 68-76, wherein the immunoconjugate comprises an antibody or antigen-binding fragment thereof that comprises the CDRs of SEQ ID NOs: 6-9, 11, and 12.

78. The method of 77, wherein the antibody is huMov19.

79. The method of any one of 68-78, wherein the immunoconjugate comprises a maytansinoid.

80. The method of 79, wherein the maytansinoid is DM4.

81. The method of any one of 68-80, wherein the immunoconjugate comprises the linker sulfo-SPDB.

82. The method of any one of 68-81, wherein the immunoconjugate is IMGN853.

83. The method of any one of 68-82, wherein the immunoconjugate is administered intravenously.

84. The method of any one of 68-83, wherein said cancer is selected from the group consisting of ovarian, brain, breast, uterine, endometrial, pancreatic, renal, and lung cancer.

85. The method of any one of 68-84, further comprising administering a steroid to the patient.

86. The method of 85, wherein the steroid is dexamethasone.

87. The method of any one of 68-86, wherein the administration results in a decrease in tumor size.

88. The method of any one of 68-87, wherein the cancer is ovarian cancer and wherein the administration results in a decrease in CA125.

89. The method of any one of 68-88, wherein the administration results in a decrease in toxicity.

90. The method of 89, wherein the toxicity is ocular toxicity.

91. The method of any one of 68-90, further comprising administering a second compound having anti-cancer properties.

92. A method for treating a human patient having an FOLR1-expressing cancer comprising administering to the patient an immunoconjugate which binds to FOLR1 polypeptide, wherein the immunoconjugate is administered at a dose of about 1 to about 7 milligrams (mg) per kilogram (kg) of body weight of the patient, wherein the kilograms of body weight of the patient are adjusted to adjusted ideal body weight and wherein the immunoconjugate is administered once a week for three weeks on a four-week schedule.

93. The method of 92, wherein the immunoconjugate is administered on days 1, 8, and 15 of the four-week schedule.

94. The method of 92 or 93, wherein the immunoconjugate is administered at a dose of about 1.1 mg/kg.

95. The method of 92 or 93, wherein the immunoconjugate is administered at a dose of about 1.8 mg/kg.

96. The method of 92 or 93, wherein the immunoconjugate is administered at a dose of about 2.5 mg/kg.

97. The method of 92 or 93, wherein the immunoconjugate is administered at a dose of about 3.3 mg/kg.

98. The method of any one of 92-97, wherein the administration produces a Cmax of about 90-160 μg/mL.

99. The method of 98, wherein the administration produces a Cmax of about 90-150 μg/mL.

100. The method of any one of 92-99, wherein the administration produces a Cmax of about 110-160 μg/mL.

101. The method of 100, wherein the administration produces a Cmax of about 110-150 μg/mL.

102. The method of any one of 92-101, wherein the administration produces an $AUC_{0-24}$ of no more than 2700 hr•μg/mL.

103. The method of any one of 92-102, wherein the immunoconjugate comprises an antibody or antigen-binding fragment thereof that comprises the CDRs of SEQ ID NOs: 6-9, 11, and 12.

104. The method of 103, wherein the antibody is huMov19.

105. The method of any one of 92-104, wherein the immunoconjugate comprises a maytansinoid.

106. The method of 105, wherein the maytansinoid is DM4.

107. The method of any one of 92-106, wherein the immunoconjugate comprises the linker sulfo-SPDB.

108. The method of any one of 92-107, wherein the immunoconjugate is TMGN853.

109. The method of any one of 92-108, wherein the immunoconjugate is administered intravenously.

110. The method of any one of 92-109, wherein said cancer is selected from the group consisting of ovarian, brain, breast, uterine, endometrial, pancreatic, renal, and lung cancer.

111. The method of 110, wherein the lung cancer is non small cell lung cancer.

112. The method of 111, wherein the non small cell lung cancer is adenocarcinoma.

113. The method of 110, wherein the ovarian cancer is epithelial ovarian cancer.

114. The method of 113, wherein the epithelial ovarian cancer is platinum resistant, relapsed, or refractory.

115. The method of 110, wherein the cancer is endometrial cancer.

116. The method of any one of 92-115, wherein a sample obtained from the patient exhibits FOLR1 expression as measured by immunohistochemistry (1HC).

117. The method of 116, wherein the sample has a staining intensity of at least 2 hetero.

118. The method of 116, wherein the sample has a staining intensity of at least 2 homo.

119. The method of 116, wherein the sample has a staining intensity of at least 3 hetero.

120. The method of 116, wherein the sample has a staining intensity of at least 3 homo.

121. The method of any one of 92-120, further comprising administering a steroid to the patient.

122. The method of 121, wherein the steroid is dexamethasone.

123. The method of any one of 92-122, wherein the administration results in a decrease in tumor size.

124. The method of any one of 92-110, 113, 114, and 116-123, wherein the cancer is ovarian cancer and wherein the administration results in a decrease in CA125.

125. The method of any one of 92-124, wherein the administration results in a decrease in toxicity.

126. The method of 125, wherein the toxicity is ocular toxicity.

127. The method of any one of 92-126, further comprising administering a second compound having anti-cancer properties.

128. The method of any one of 92, 93, and 98-127, wherein the immunoconjugate is administered at a dose of about 2.8 mg/kg.

129. The method of any one of 92, 93, and 98-127, wherein the immunoconjugate is administered at a dose of about 3.0 mg/kg.

130. The method of any one of 92, 93, and 98-127, wherein the immunoconjugate is administered at a dose of about 3.75 mg/kg.

131. The mtheod of any one of 92-101 and 103-130, wherein the administration produces an $AUC_{0-24}$ of no more than 2785 hr•μg/mL

132. The method of any one of 92-101 and 103-130, wherein the administration produces an $AUC_{0-24}$ of no more than 2741 hr•μg/mL.

**Claims**

1. An immunoconjugate which binds to FOLR1 polypeptide for use in the treatment of a FOLR1-expressing cancer, wherein the immunoconjugate comprises a maytansinoid and a FOLR1 binding molecule, wherein the FOLR1 binding molecule is an antibody or antigen-binding fragment thereof that comprises the sequences of SEQ ID NOs: 6 to 10 and the sequence of SEQ ID NO: 12, and wherein the immunoconjugate is administered at a dose of 5 milligrams (mg) per kilogram (kg) of adjusted ideal body weight (AIBW).

2. The immunoconjugate of claim 1, wherein the immunoconjugate comprises 1-10 maytansinoid molecules, more preferably 2-5 maytansinoid molecules, and most preferably 3-4 maytansinoid molecules.

3. The immunoconjugate of claim 1 or 2, wherein the maytansinoid is DM4.

4. The immunoconjugate of any one of claims 1-3, wherein the immunoconjugate comprises the linker sulfo-SPDB.

5. A composition comprising immunoconjugates which bind to FOLR1 polypeptide for the treatment of a FOLR1-expressing cancer in a human patient, wherein the immunoconjugates comprise an antibody or antigen-binding fragment thereof that comprises the sequences of SEQ ID NOs: 6 to 9, and the sequences of SEQ ID NOs: 11 and 12, and a maytansinoid, and wherein the composition is formulated for administration at a dose of 5 milligrams (mg) per kilogram (kg) of adjusted ideal body weight (AIBW) of the patient, wherein the composition comprises an average of about 1 to about 10 maytansinoids per antibody or antigen-binding fragment.

6. The composition of claim 5, wherein the composition is administered once every three weeks.

7. The composition of claim 5, wherein the composition is administered once every week.

8. The composition of any one of claims 5-7, wherein the antibody or antigen-binding fragment thereof comprises the VH CDR-2 of SEQ ID NO:10.

9. The composition of any one of claims 5-8, wherein the antibody or antigen-binding fragment thereof comprises a variable heavy chain domain comprising SEQ ID NO: 3 and a variable light chain domain comprising SEQ ID NO: 4 or SEQ ID NO: 5 or comprises a variable heavy chain domain comprising SEQ ID NO: 3 and a variable light chain domain comprising SEQ ID NO: 5.

10. The composition of any one of claims 5-9, wherein the antibody comprises (i) a heavy chain comprising the same amino acid sequence as the amino acid sequence of the heavy chain encoded by the plasmid deposited with the American Type Culture Collection (ATCC) as PTA-10772 and (ii) a light chain comprising the same amino acid sequence as the amino acid sequence of the light chain encoded by the plasmid deposited with the ATCC as PTA-10774.

11. The composition of any one of claims 5-10, wherein the composition comprises an average of about 2-5 maytansinoids or an average of about 3-4 maytansinoids.

12. The composition of any one of claims 5-11, wherein the maytansinoid is DM4.

13. The composition of any one of claims 5-12, wherein the immunoconjugates comprise the linker sulfo-SPDB.

14. The composition of any one of claims 5-13, wherein the immunoconjugates comprise DM4 and an antibody comprising (i) a heavy chain comprising the same amino acid sequence as the amino acid sequence of the heavy chain encoded by the plasmid deposited with the American Type Culture Collection (ATCC) as PTA-10772 and (ii) a light chain comprising the same amino acid sequence as the amino acid sequence of the light chain encoded by the plasmid deposited with the ATCC as PTA-10774, and wherein the DM4 is linked to the antibody by sulfo-SPDB.

15. The composition of any one of claims 5-14, wherein the composition is formulated for IV administration.

16. The composition of any one of claims 5-15, wherein said cancer is ovarian cancer, cancer of the peritoneum, endometrial cancer, or uterine cancer, optionally wherein the ovarian cancer is platinum resistant, relapsed, or refractory.

# Figure 1A

## IMGN853 Pharmacokinetic Results

| Dose (mg/kg) | 0.15 (n=2) | 0.5 (n=1) | 1.0 (n=1) | 2.0 (n=1) | 3.3 (n=3) | 5.0 (n=3) | 7.0 (n=4) |
|---|---|---|---|---|---|---|---|
| Cmax (ug/mL) | 2.9 | 10.5 | 22.1 | 65.7 | 96.6 (16.4) | 108 (32.7) | 179 (21.8) |
| Half-life (hr) | 35.4 | 41.2 | 70.1 | 69.9 | 99.5 (15.7) | 105 (4.4) | 87.6 (11.5) |
| Half-life (d) | 1.5 | 1.7 | 2.9 | 2.9 | 4.1 (0.65) | 4.4 (1.0) | 3.6 (0.5) |
| $AUC_{(0-inf)}$ (hr*ug/mL) | 150.9 | 596.6 | 1779 | 6505 | 12188 (2581) | 12708(2112) | 17559(2850) |
| $AUC_{(0-168)}$(hr*ug/mL) | 104.6 | 496.6 | 1678 | 5330 | 8178 (1129) | 8254 (1771) | 12177(1621) |
| CL (mL/hr/kg) | 1.1 | 0.8 | 0.6 | 0.3 | 0.3 (0.06) | 0.4 (0.07) | 0.4 (0.06) |
| Vss (mL/kg) | 51.8 | 46.7 | 54.9 | 28.2 | 38.6 ( 5.2) | 61.2 (16.1) | 52.8 (8.3) |

**Figure 1B**

## IMGN853 Pharmacokinetic Results

| Cohort Dose (mg/kg) | N | $C_{Max}$ (µg/ml) | $T_{1/2}$ (days) | Cl (ml/hr/kg) | $V_{ss}$ (ml/kg) | $AUC_{0_\infty}$ (hr*µg/ml) |
|---|---|---|---|---|---|---|
| **0.15** | 2 | 2.90 (± 0.47) | 1.5 (± 0.2) | 1.08 (± 0.38) | 53.2 (± 11.2) | 149 (± 52) |
| **0.5** | 1 | 10.55 | 3.2 | 0.58 | 59.2 | 865 |
| **1** | 1 | 22.09 | 3.4 | 0.56 | 57.0 | 1,777 |
| **2** | 1 | 65.73 | 5.0 | 0.30 | 32.4 | 6,757 |
| **3.3** | 3 | 96.64 (± 16.39) | 4.7 (± 0.3) | 0.26 (± 0.05) | 36.82 (± 5.7) | 12,915 (± 2,056) |
| **5** | 10 | 141.90 (± 41.63) | 4.9 (± 1.2) | 0.35 (± 0.08) | 51.9 (± 17.0) | 15,079 (± 3,740) |
| **7** | 5 | 172.98 (± 23.58) | 6.2 (± 1.3) | 0.36 (± 0.08) | 65.7 (± 8.9) | 20,146 (± 4,099) |

Figure 2A-C

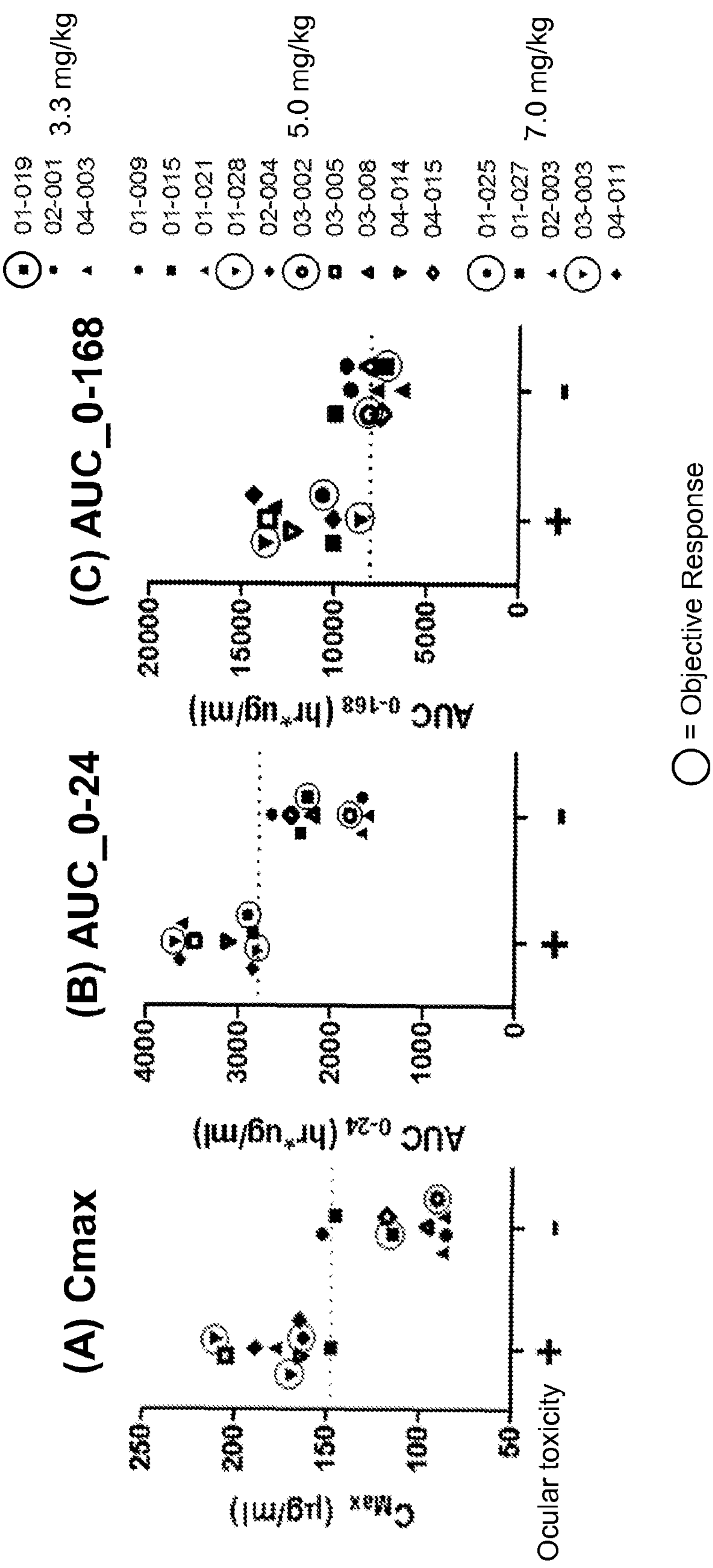
(A) Cmax
$C_{Max}$ (µg/ml)
250
200
150
100
50
Ocular toxicity
+
-
(B) AUC_0-24
AUC 0-24 (hr*ug/ml)
4000
3000
2000
1000
0
+
-
(C) AUC_0-168
AUC 0-168 (hr*ug/ml)
20000
15000
10000
5000
0
+
-
01-019
02-001
04-003
3.3 mg/kg
01-009
01-015
01-021
01-028
02-004
03-002
03-005
03-008
04-014
04-015
5.0 mg/kg
01-025
01-027
02-003
03-003
04-011
7.0 mg/kg
= Objective Response

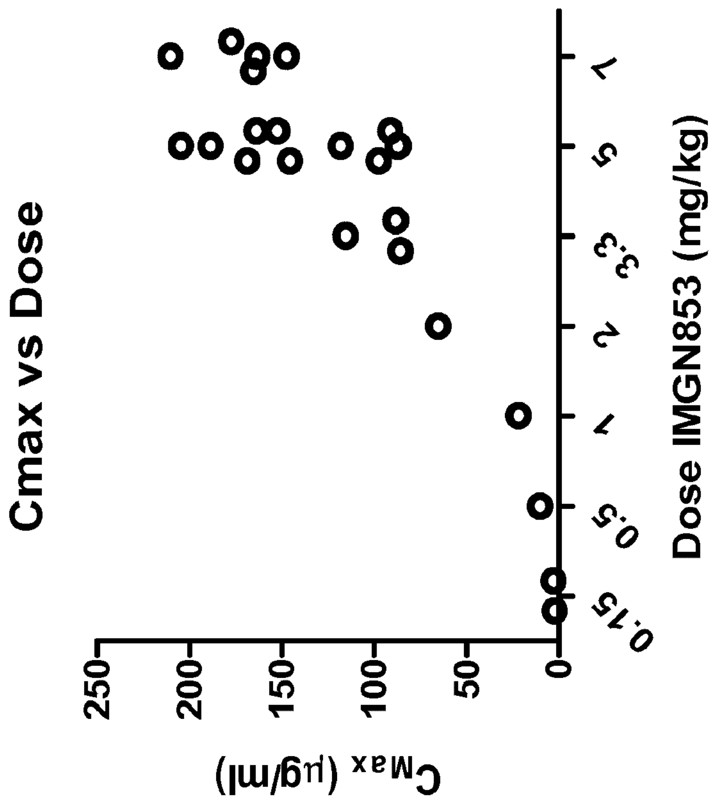
Cmax vs Dose
Cmax (µg/ml)
250
200
150
100
50
0
0.15
0.5
1
2
3.3
5
7
Dose IMGN853 (mg/kg)

Figure 3

Figure 4

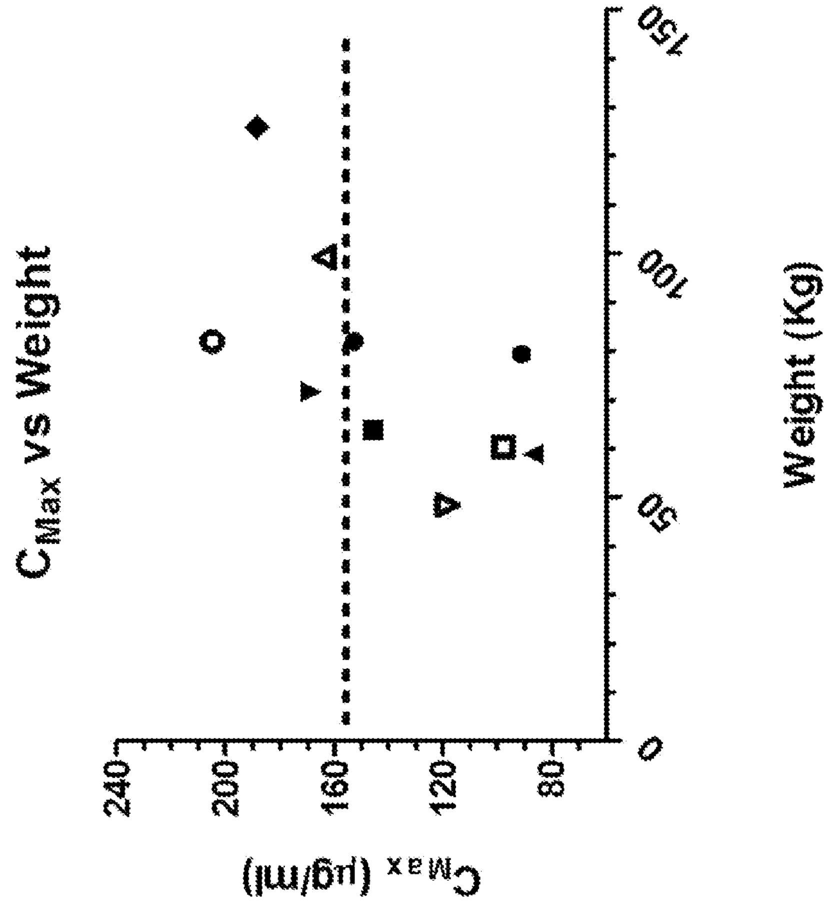
$C_{Max}$ vs Weight
$C_{Max}$ (µg/ml)
240
200
160
120
80
0
50
100
150
Weight (Kg)

Figure 5

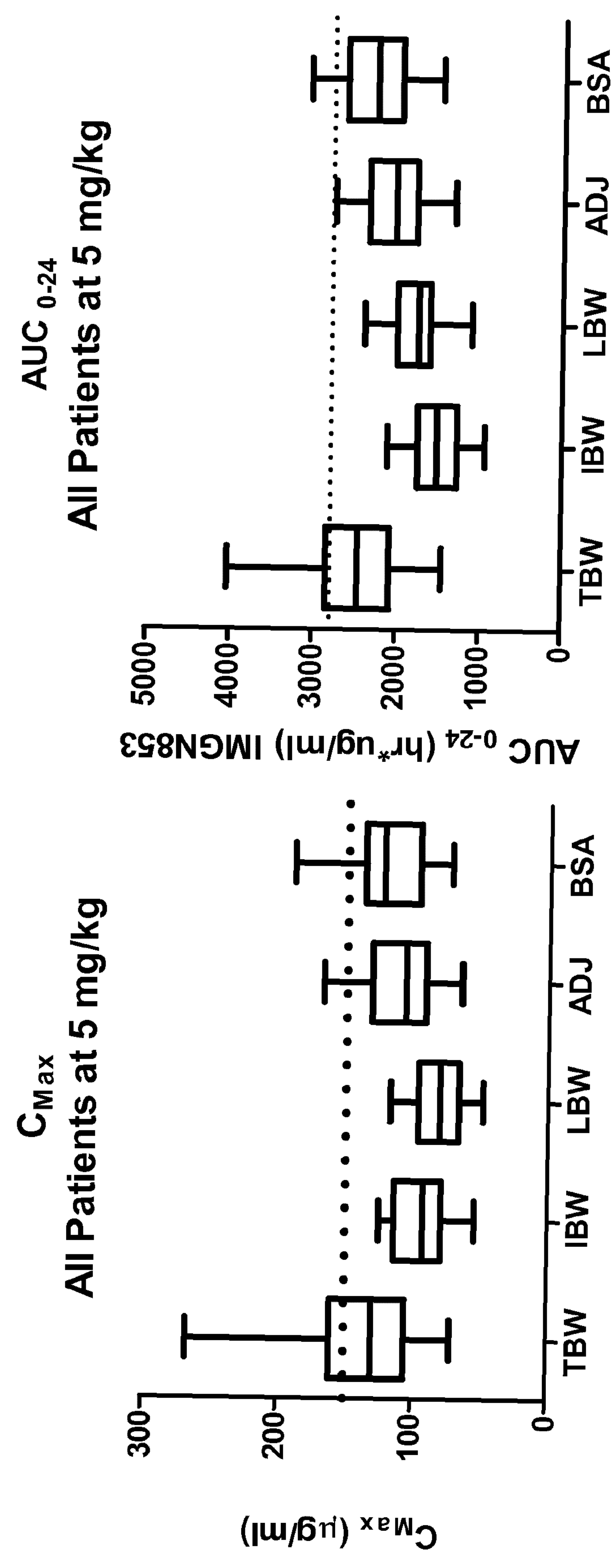
C<sub>Max</sub>
All Patients at 5 mg/kg
CMax (μg/ml)
300
200
100
0
TBW
IBW
LBW
ADJ
BSA
AUC 0-24
All Patients at 5 mg/kg
AUC 0-24 (hr*ug/ml) IMGN853
5000
4000
3000
2000
1000
0
TBW
IBW
LBW
ADJ
BSA

# Figure 6

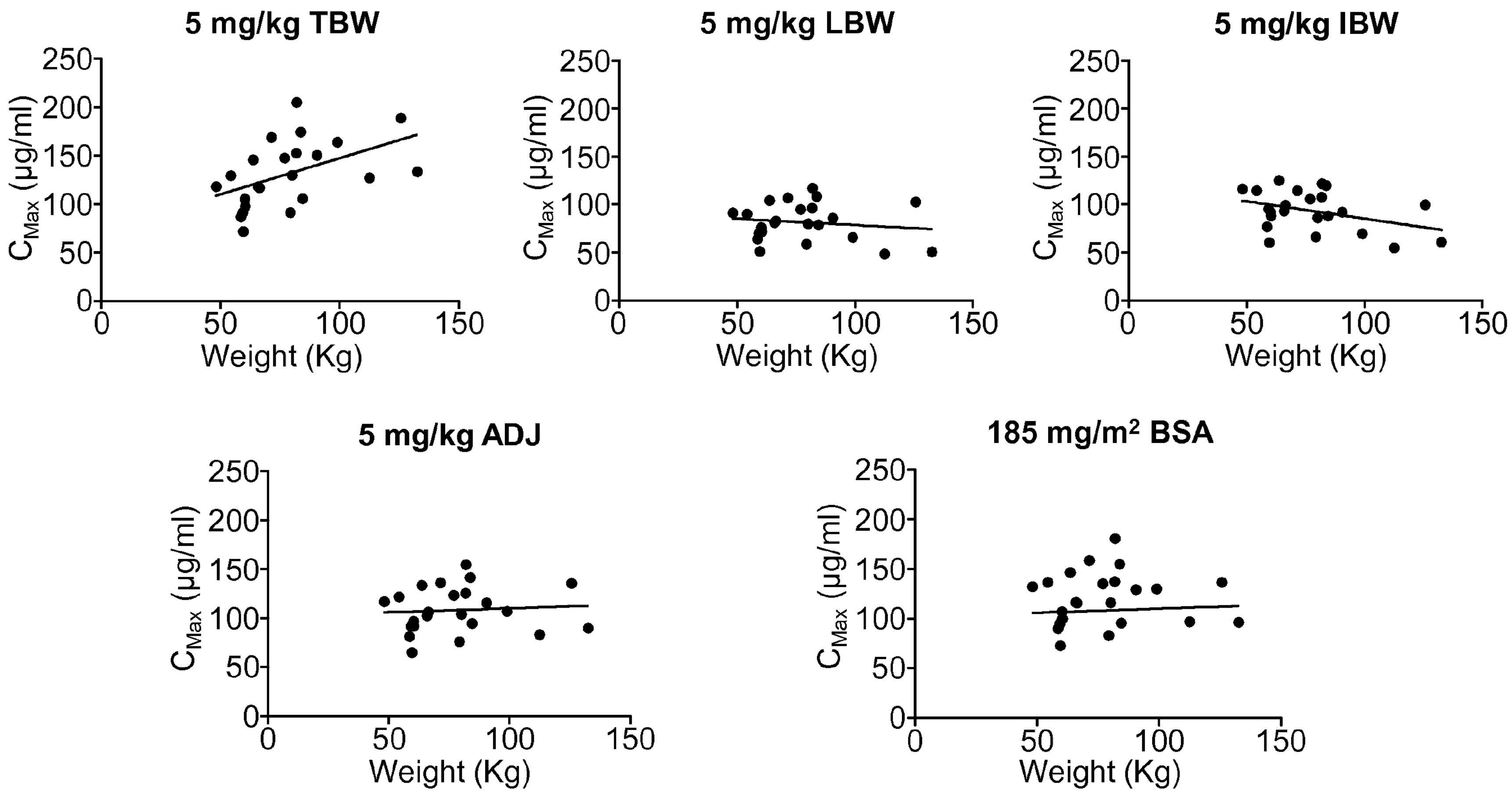
5 mg/kg TBW
5 mg/kg LBW
5 mg/kg IBW
5 mg/kg ADJ
185 mg/m2 BSA
CMax (µg/ml)
0
50
100
150
200
250
Weight (Kg)
0
50
100
150

**Figure 7**

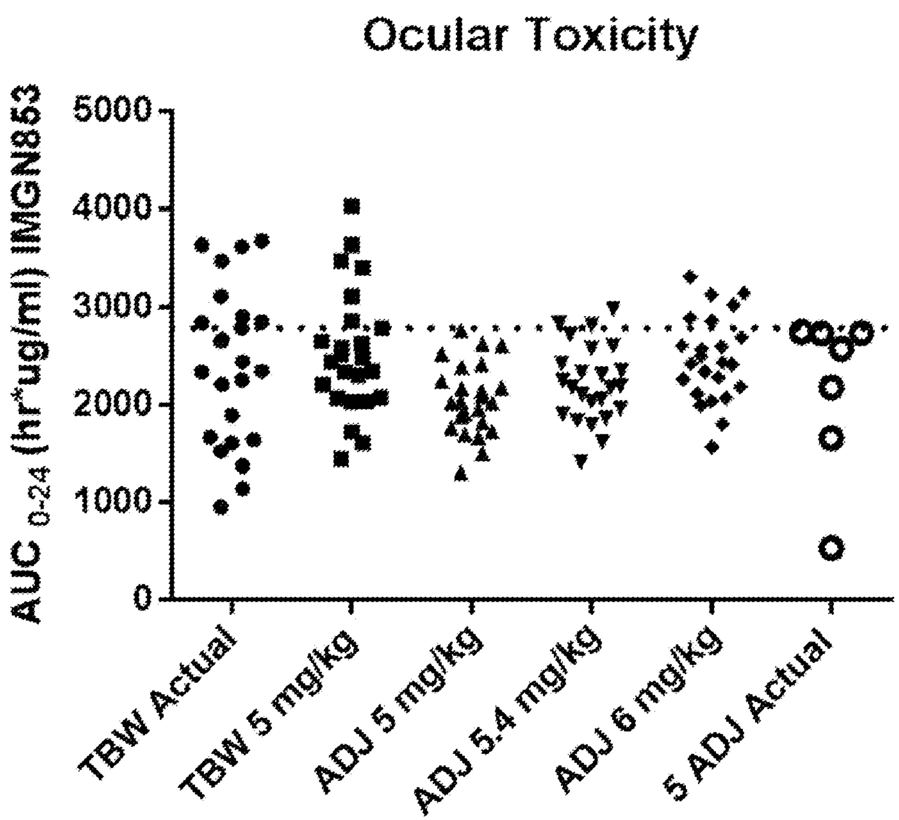


| | | | Percent of Patients Above Threshold | | | | | |
|---|---|---|---|---|---|---|---|---|
| Readout | PK Parameter | Threshold (hr*ug/ml) | TBW Actual | TBW 5 mg/kg | ADJ 5 mg/kg | ADJ 5.4 mg/kg | ADJ 6 mg/kg | 5 ADJ Actual |
| Ocular Toxicity | AUC_0-24 | >2741 | 38% | 29% | 0% | 13% | 25% | 14%* |

* - Patient @ 2749 hr*ug/ml

Figure 8

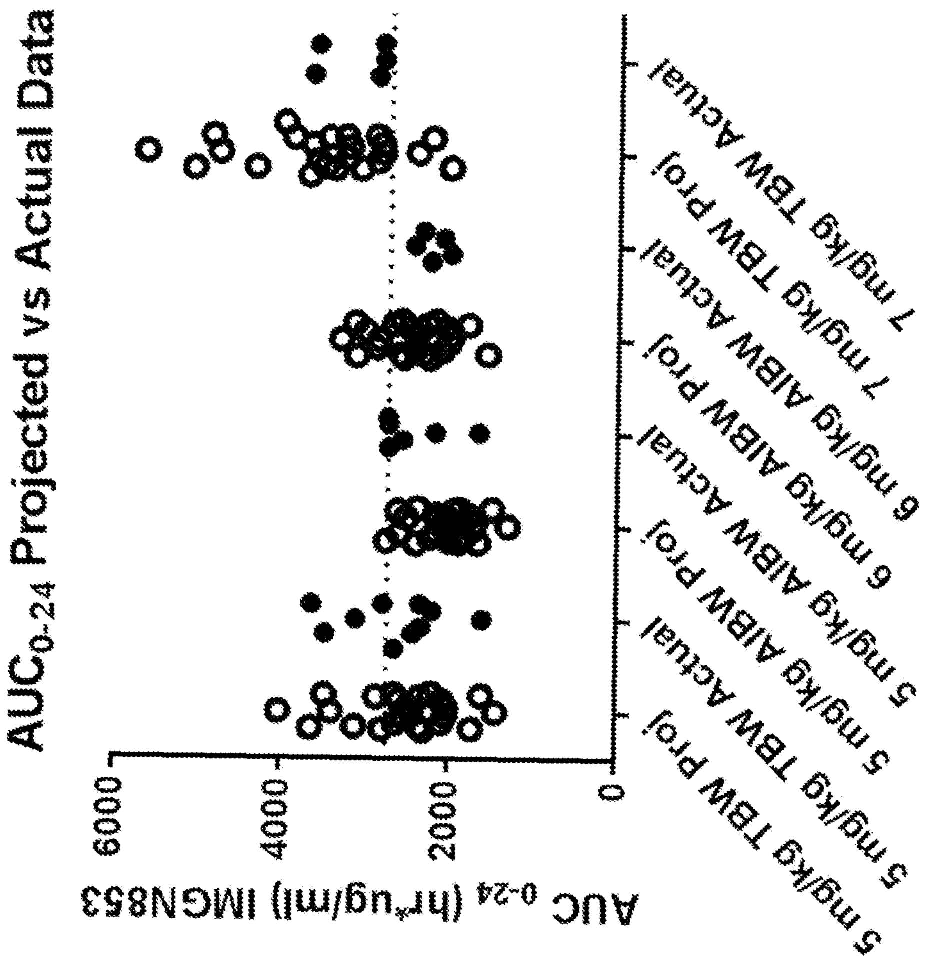
AUC$_{0-24}$ Projected vs Actual Data
AUC $_{0-24}$ (hr*ug/ml) IMGN853
6000
4000
2000
0
5 mg/kg TBW Proj
5 mg/kg TBW Actual
5 mg/kg AIBW Proj
5 mg/kg AIBW Actual
6 mg/kg AIBW Proj
6 mg/kg AIBW Actual
7 mg/kg TBW Proj
7 mg/kg TBW Actual

# Figure 9

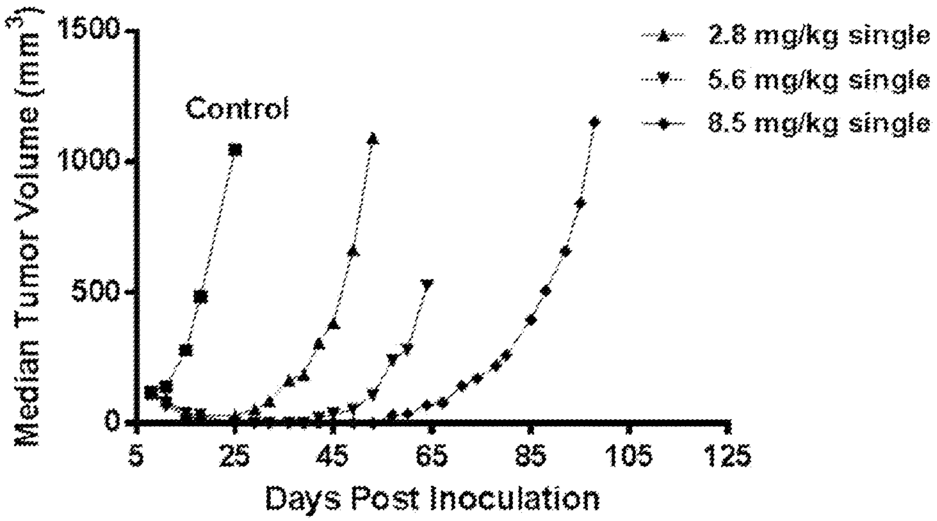


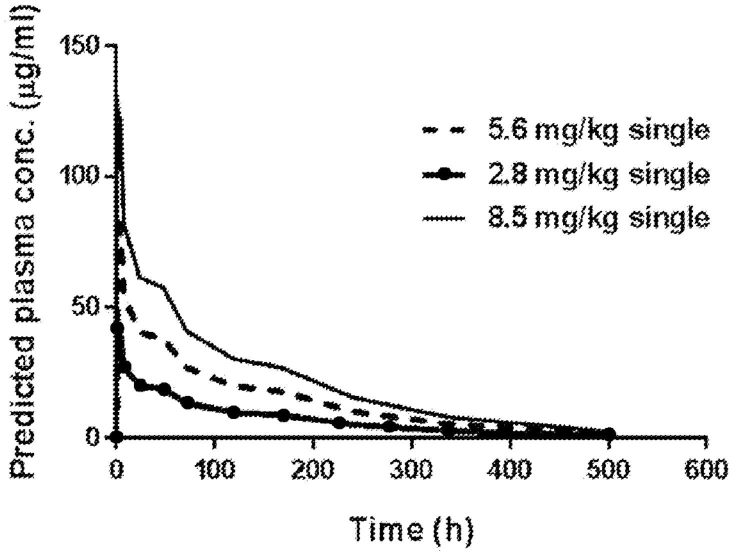


| Dose (mg/kg) | Schedule | T/C (%) | Log cell kill | CR | Cmax (µg/ml) | Cavg (µg/ml) | AUC 0-540 (hr*µg/ml) |
|---|---|---|---|---|---|---|---|
| 2.8 | Single | 3 | 1.5 | 2/6 | 43 | 7 | 3740 |
| 5.6 | Single | 0 | 2.7 | 4/6 | 86 | 15 | 7481 |
| 8.5 | Single | 0 | 3.9 | 6/6 | 131 | 23 | 11354 |

# Figure 10

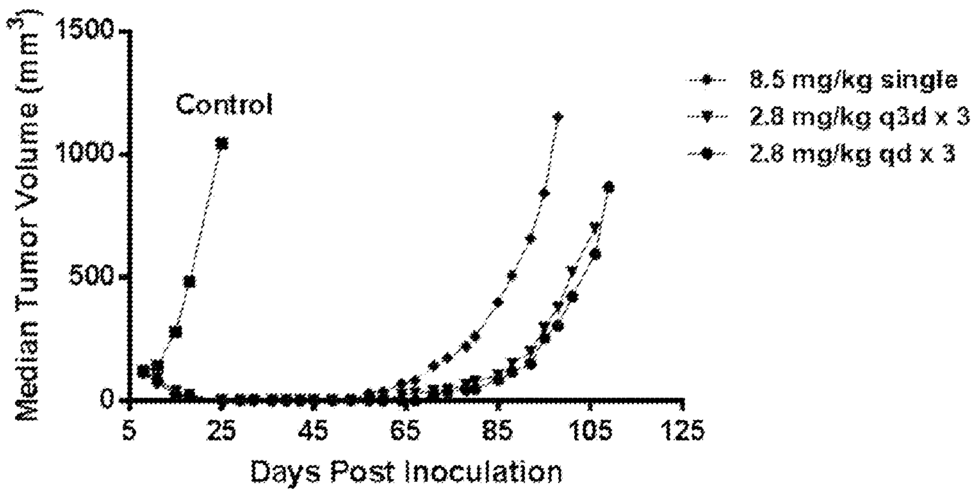


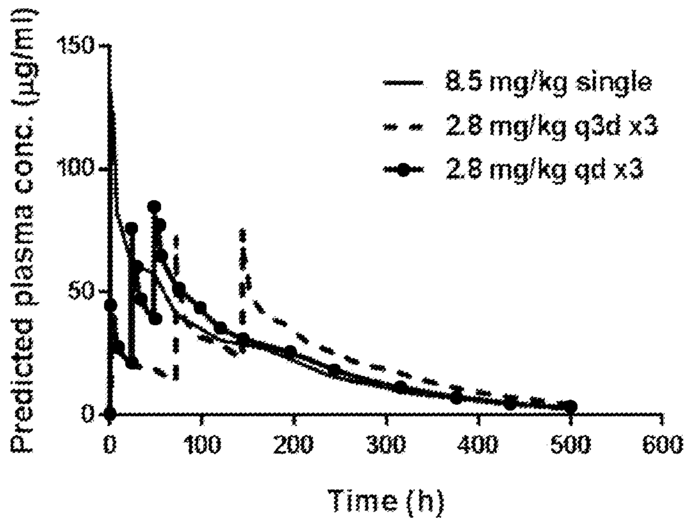


| Dose (mg/kg) | Schedule | Total dose (mg/kg) | T/C (%) | Log cell kill | CR | Tumor-free (Day 120) | Cmax (µg/ml) | Cavg (µg/ml) | AUC 0-540 (hr*µg/ml) |
|---|---|---|---|---|---|---|---|---|---|
| 8.5 | Single | 8.5 | 0 | 3.9 | 6/6 | 0/6 | 131 | 23 | 11354 |
| 2.8 | qd x 3 | 8.4 | 0 | 4.6 | 6/6 | 1/6 | 85 | 28 | 10961 |
| 2.8 | q3d x 3 | 8.4 | 0 | 4.1 | 6/6 | 2/6 | 75 | 21 | 10762 |

# Figure 11

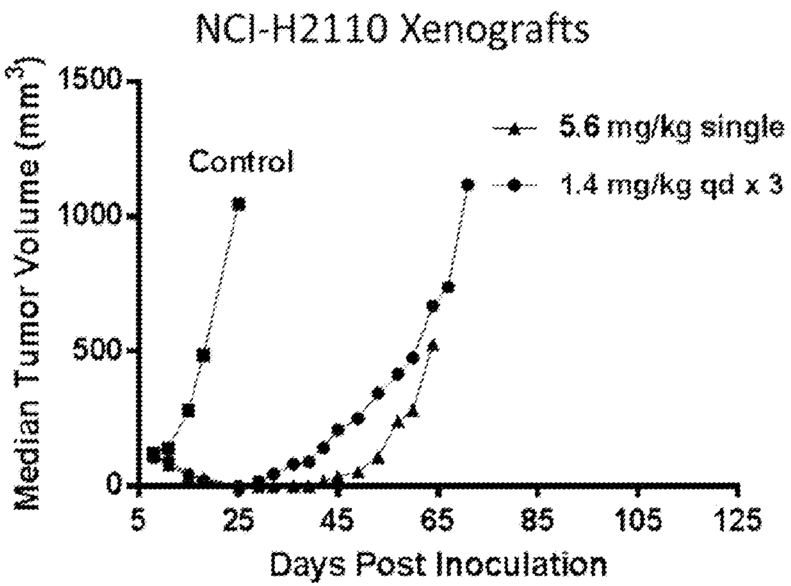


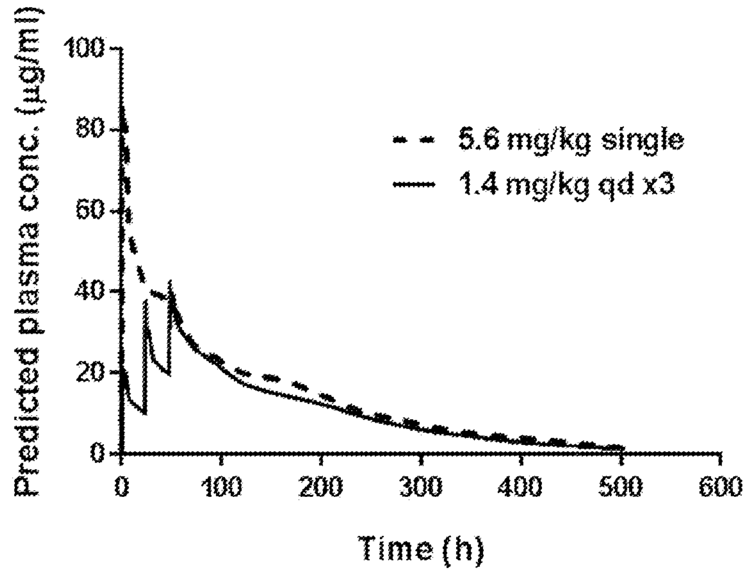


| Dose (mg/kg) | Schedule | Total Dose (mg/kg) | T/C (%) | Log cell kill | CR | Cmax (µg/ml) | Cavg (µg/ml) | AUC 0-540 (hr*µg/ml) |
|---|---|---|---|---|---|---|---|---|
| 5.6 | Single | 5.6 | 0 | 2.7 | 4/6 | 86 | 15 | 7481 |
| 1.4 | qd x 3 | 4.2 | 0 | 2.5 | 4/6 | 43 | 14 | 5480 |

# Figure 12

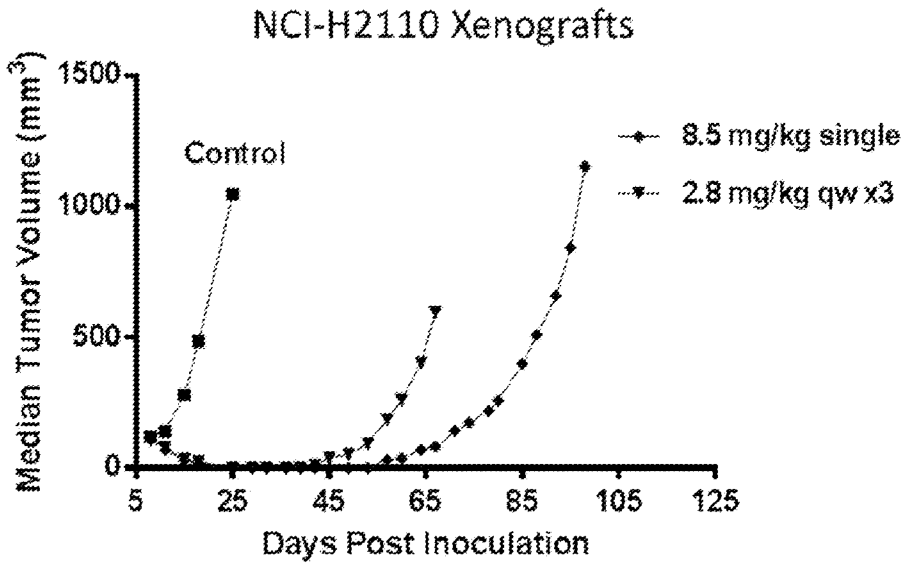


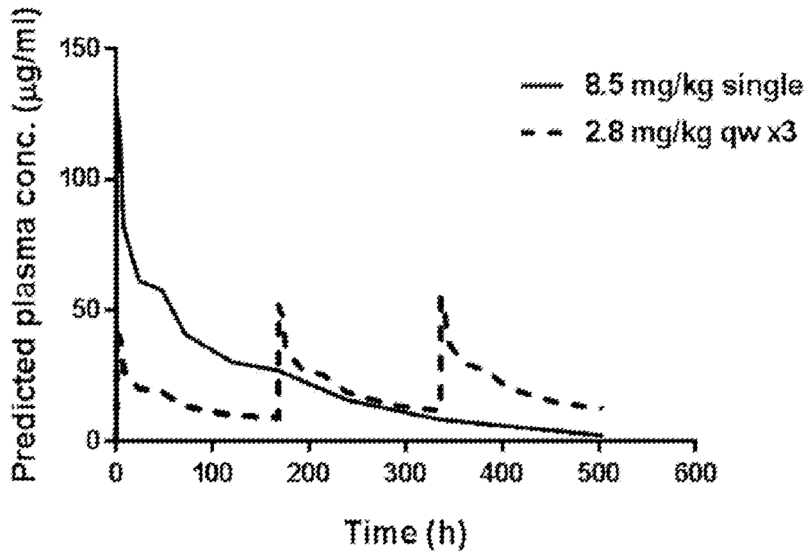


| Dose (mg/kg) | Schedule | Total Dose (mg/kg) | T/C (%) | Log cell kill | CR | Cmax (µg/ml) | Cavg (µg/ml) | AUC 0-540 (hr*µg/ml) |
|---|---|---|---|---|---|---|---|---|
| 8.5 | Single | 8.5 | 0 | 3.9 | 6/6 | 131 | 23 | 11354 |
| 2.8 | qw x 3 | 8.4 | 0 | 3.0 | 6/6 | 55 | 15 | 9614 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3896111 A
- US 5225539 A
- US 20120009181 A
- US 4816567 A
- US 5750373 A
- US 5969108 A
- US 6172197 B
- US 5885793 A
- US 6521404 B
- US 6544731 B
- US 6555313 B
- US 6582915 B
- US 6593081 B
- US 6300064 B
- US 6653068 B
- US 6706484 B
- US 7264963 B
- US 5545807 A
- US 5545806 A
- US 5569825 A
- US 5625126 A
- US 5633425 A
- US 5661016 A
- US 20080312425 A
- US 20080177048 A
- US 20090187005 A
- US 4563304 A
- US 20090274713 A
- WO 20090134976 A
- US 20050169933 A
- US 20120282282 A
- US 4424219 A
- US 4256746 A
- US 4294757 A
- US 4307016 A
- US 4313946 A
- US 4315929 A
- US 4331598 A
- US 4361650 A
- US 4362663 A
- US 4364866 A
- US 4450254 A
- US 4322348 A
- US 4371533 A
- US 5208020 A
- US 5416064 A
- US 5475092 A
- US 5585499 A
- US 5846545 A
- US 6333410 B
- US 7276497 B
- US 7473796 B
- US 6441163 B
- US 6716821 B
- US 7368565 B
- US 20120282175 A
- WO 2012135675 A
- WO 2011106528 A
- WO 2012138749 A

### Non-patent literature cited in the description


- **JEMAL et al.** *Cancer J. Clin.,* 2003, vol. 53, 5-26
- **WEITMAN SD et al.** *Cancer Res,* 1992, vol. 52, 3396-3401
- **ANTONY AC.** *Annu Rev Nutr,* 1996, vol. 16, 501-521
- **KALLI KR et al.** *Gynecol Oncol,* 2008, vol. 108, 619-626
- **VON GRUENIGEN V et al.** *Cancer,* 2008, vol. 112, 2221-2227
- **AYHAN A et al.** *Am J Obstet Gynecol,* 2007, vol. 196 (81), e81-86
- **HARRY VN et al.** *Obstet Gynecol Surv,* 2009, vol. 64, 548-560
- **JONES.** *Nature,* 1986, vol. 321, 522-525
- **RICCHMANN et al.** *Nature,* 1988, vol. 332, 323-327
- **VERHOEYEN et al.** *Science,* 1988, vol. 239, 1534-1536
- **KABAT et al.** Sequences of Proteins of Immunological Interest. National Institutes of Health, 1991
- **AL-LAZIKANI et al.** *J. Molec. Biol.,* 1997, vol. 273, 927-948
- **KABAT et al.** Sequences of Immunological Interest. Public Health Service, National Institutes of Health, 1991
- **KABAT et al.** Sequences of Proteins of Immunological Interest. Public Health Service, National Institutes of Health, 1991

- **CHOTHIA ; LESK.** *J. Mol. Biol.,* 1987, vol. 196, 901-917
- **GREEN ; DUFFULL.** *British Journal of Clinical Pharmacology,* 2004, vol. 58, 119-133
- **W.A. WEBER.** *J. Nucl. Med.,* 2009, vol. 50, 1S-10S
- **JOHNSON et al.** *J. Clin. Oncol.,* 2003, vol. 21 (7), 1404-1411
- **KARLIN et al.** *Proc. Natl. Acad. Sci.,* 1990, vol. 87, 2264-2268
- **KARLIN et al.** *Proc. Natl. Acad. Sci.,* 1993, vol. 90, 5873-5877
- **ALTSCHUL et al.** *Nucleic Acids Res.,* 1991, vol. 25, 3389-3402
- **ALTSCHUL et al.** *Nucleic Acids Res.,* 1997, vol. 25, 3389-3402
- **ALTSCHUL et al.** *Methods in Enzymology,* 1996, vol. 266, 460-480
- **NEEDLEMAN ; WUNSCH.** *J. Mol. Biol.,* 1970, vol. 48, 444-453
- **MYERS ; MILLER.** *CABIOS,* 1989, vol. 4, 11-17
- **SMITH ; WATERMAN.** *Advances in Applied Mathematics,* 1981, vol. 2, 482-489
- **BRUMMELL.** *Biochem.,* 1993, vol. 32, 1180-1187
- **KOBAYASHI et al.** *Protein Eng.,* 1999, vol. 12 (10), 879-884
- **BURKS et al.** *Proc. Natl. Acad. Sci. USA,* 1997, vol. 94, 412-417
- **KOHLER ; MILSTEIN.** *Nature,* 1975, vol. 256, 495
- **GODING.** Monoclonal Antibodies: Principles and Practice. Academic Press, 1986
- **MCCAFFERTY et al.** *Nature,* 1990, vol. 348, 552-554
- **CLACKSON et al.** *Nature,* 1991, vol. 352, 624-628
- **MARKS et al.** *J. Mol. Biol.,* 1991, vol. 222, 581-597
- **COLE et al.** *Monoclonal Antibodies and Cancer Therapy, Alan R. Liss,* 1985, 77
- **BOCMER et al.** *J. Immunol.,* 1991, vol. 147 (1), 86-95
- **VAUGHAN et al.** *Nat. Biotech.,* 1996, vol. 14, 309-314
- **SHEETS et al.** *Proc. Nat'l. Acad. Sci.,* 1998, vol. 95, 6157-6162
- **HOOGENBOOM ; WINTER.** *J. Mol. Biol.,* 1991, vol. 227, 381
- **MARKS et al.** *J. Mol. Biol.,* 1991, vol. 222, 581
- **ROTHE et al.** *J. Mol. Bio.,* 2007
- **MARKS et al.** *BioITcchnology,* 1992, vol. 10, 779-783
- REMINGTON'S PHARMACEUTICAL SCIENCES. Mack Publishing Co, 2000
- **CARLSSON et al.** *Biochem. J.,* 1978, vol. 173, 723-737
- *CHEMICAL ABSTRACTS,* 341498-08-6
- **YOSHITAKC et al.** *Eur. J. Biochem.,* 1979, vol. 101, 395-399
- **HASHIDA et al.** *J. Applied Biochem.,* 1984, 56-63
- **LIU et al.** *Biochem.,* 1979, vol. 18, 690-697
- Remington, The Science and Practice of Pharmacy. Mack Publishing, 2000